(19) Europäisches Patentamt
European Patent Office
Office européen des brevets

(11) Veröffentlichungsnummer: **0 428 849 A2**

(12) **EUROPÄISCHE PATENTANMELDUNG**

(21) Anmeldenummer: **90118377.2**

(22) Anmeldetag: **25.09.90**

(51) Int. Cl.[5]: **C07K 5/06**, C07K 5/08, A61K 37/64, C07C 237/10, C07C 271/22, C07C 271/20, A61K 31/16, A61K 31/27, C07C 237/22, C07C 211/27, A61K 31/13

(30) Priorität: **28.09.89 DE 3932390**
**25.04.90 DE 4013149**

(43) Veröffentlichungstag der Anmeldung:
**29.05.91 Patentblatt 91/22**

(84) Benannte Vertragsstaaten:
**AT BE CH DE DK ES FR GB GR IT LI LU NL SE**

(71) Anmelder: **HOECHST AKTIENGESELLSCHAFT**
**Postfach 80 03 20**
**W-6230 Frankfurt am Main 80(DE)**

(72) Erfinder: **Budt, Karl-Heinz, Dr.**
**Am Flachsland 18**
**W-6233 Kelkheim (Taunus)(DE)**
Erfinder: **Stowasser, Bernd, Dr.**
**Pilgerpfad 24**
**W-6114 Gross-Umstadt(DE)**
Erfinder: **Ruppert, Dieter, Dr.**
**Schreyerstrasse 30**
**W-6242 Kronberg(DE)**
Erfinder: **Meichsner, Christoph, Dr.**
**Bienerstrasse 30**
**W-6238 Hofheim am Taunus(DE)**
Erfinder: **Paessens, Arnold, Dr.**
**Stresemannstrasse 51**
**W-5657 Haan(DE)**
Erfinder: **Hansen, Jutta, Dr.**
**Claudiusweg 9**
**W-5600 Wuppertal(DE)**
Erfinder: **Knolle, Jochen, Dr.**
**Hochster Strasse 21**
**W-6239 Kriftel(DE)**

(54) **Inhibitoren retroviraler Proteasen.**

(57) Die vorliegende Erfindung betrifft Verbindung der Formel I

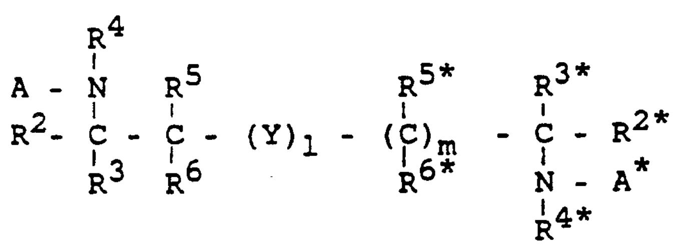

(I)

worin
A, Y, $R^2$, $R^3$, $R^4$, $R^5$, $R^6$, l, m, sowie die entsprechenden mit * versehenen Reste wie in der Beschreibung angegeben definiert sind, ein Verfahren zu deren Herstellung, sowie ihre Verwendung zur Hemmung retroviraler Proteasen.

EP 0 428 849 A2

# INHIBITOREN RETROVIRALER PROTEASEN

Die vorliegende Erfindung betrifft Substanzen, die die Wirkung retroviraler Proteasen hemmen, Verfahren zu ihrer Herstellung, ihre Verwendung sowie diese enthaltende Arzneimittel.

Die etiologische Ursache des "erworbenen Immunschwäche-Syndroms" (engl.: aquired immune deficiency syndrome (AIDS)) ist der sogenannte human immunodeficiency virus (HIV) (F. Barre-Sinoussi et al., Soience 220, (1983), 868-870; R.C. Gallo et al., Science 224, (1984), 500-502; R.C. Gallo und L. Montagnier, Scient. Am. 259(4), (1988), 40-48). HIV ist ein Retrovirus und gehört in die Gruppe der Lentiviren (M.A. Gonda, F. Wong-Staal und R.C. Gallo, Science, 227, (1985), 173; P. Sonigo et al., Cell, 42, (1985), 369).

Die Aids Epidemie hat sich mittlerweile über nahezu alle Staaten mehr oder weniger ausgebreitet. Aus 149 Ländern wurden der Welt-Gesundheits Organisation (WHO) bisher etwa 160.000 Krankheitsfälle gemeldet. Die WHO schätzt die wirkliche Zahl auf etwa 500.000 Fälle, die Zahl der infizierten Personen auf 5-10 Millionen (J. M. Mann auf der 5th International Conference on Aids, Montreal, 4.-9. Juni 1989; siehe z.B. C&EN, June 26, (1989), 7-16).

Die einzige bisher für die Indikation AIDS zugelassene Substanz Zidovudine (AZT) vermag das Leben der Patienten in vielen Fällen zu verlängern, besitzt jedoch ernste, toxische Nebeneffekte, die in vielen Fällen den Absatz der Therapie verlangen. Auch wurden bereits erste Stämme von HIV entdeckt, die eine deutlich geringere Empfindlichkeit gegen AZT zeigten und somit die Gefahr einer Resistenz andeuten (C&EN s.o.). Weitere Ansatzpunkte in der HIV-Therapie sind somit dringend erforderlich.

HIV-Proteine werden analog zu Proteinen anderer Retroviren zuerst als lange Vorläufer Polyproteine gag, pol und env translatiert (C. Dickson et al. in RNA Turmor Viruses (Herausgeber: R. Weiss, N. Teich, H. Varmus und J. Coffin) 2nd Ed., revised, Seite 513-648, Cold Spring Harbor Laboratory, Cold Spring Harbor, NY) und erst anschließend proteolytisch zu den Strukturproteinen (p17 (MA), p24 (CA), p7 (NC) und p6), den Enzymen (Protease (PR), Reverse Transkriptase (RT) und Integrase (IN)), und den Hüllproteinen (gp120 (SU) und gp41 (TM)) prozessiert (Nomenklatur: J. Leis et al., J. Virol, 62, (1988), (1808-1809). Man nimmt an, daß die Spaltung der gag und pol Polyproteine durch eine viral codierte Protease bewirkt wird. Mutationen innerhalb der die Protease codierenden Region führen zu nicht infektiösen Viruspartikeln (N.E. Kohl et al. Proc. Natl. Acad. Sci. USA 85, (1988), 4686-4690).

Die HIV Protease besteht aus 99 Aminosäuren und spaltet sich offensichtlich selbst durch Hydrolyse der beiden Phe-Pro-Bindungen in den Positionen 68-69 bzw. 167-168 aus dem pol Polyprotein heraus (M.C. Graves, J.J. Lim, E.P. Heimer und R.A. Kramer Proc. Natl. Acad. Sci. USA 85 (1988), 2449-2453; J. Hansen, S. Billich, T. Schulze, S. Sukrow und K. Mölling, EMBO J. 7 (1988), 1785-1791; E.P. Lillehoj et al., J. Virology 62 (1988) 3053-3058; J. Schneider und S.B.H. Kent, Cell 54 (1988) 363-368).

In der Literatur sind bisher erst wenige Inhibitoren der HIV-Protease bekannt. Erster Vertreter war das Pepstatin A mit einem $IC_{50}$-Wert von ca. 0,5 mmol (I. Katoh, T. Yasunaga, Y. Ikawa und Y. Yoshinaka, Nature, 329, (1987), 654-656). Inzwischen sind einige weitere mäßig bis gut wirksame Inhibitoren beschrieben (S. Billich et al., J. Biol. Chem. 34, (1988), 17905-17098; M. Moore et al., Biochem. Biophys. Res. Comm., 159, (1989), 420-425; A.D. Richards, R. Roberts, B.M. Dunn, M.C. Graves und J. Kay, FEBS Lett., 247, (1989), 113-117).

Hohe Dosen von Pepstatin A waren in der Lage in der Biosynthese die Bildung des Kernproteins p24 zu verringern (v.d.Helm, L. Gürtler, J. Eberle und F. Deinhardt, FEBS Lett., 247, (1989), 349-352).

Es wurde nun eine neue Strukturklasse gefunden, die im Enzymtest hochwirksam die HIV-Protease hemmt.

Die vorliegende Erfindung betrifft Verbindungen der Formel I

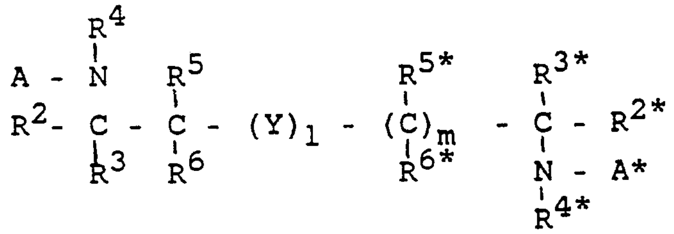

(I)

worin

Y für Sauerstoff, Schwefel, einen Rest der Formel II oder einen Rest der Formel III

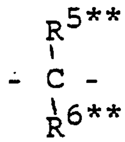 (II) 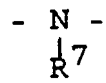 (III)

steht;
l und m unabhängig voneinander 0 oder 1 sind; A einen Rest der Formel IV und A* einen Rest der Formel IV* bedeuten

$D - (E)_n - (F)_o - (G)_p -$ (IV)

$D^* - (E^*)_{n^*} - (F^*)_{o^*} - (G^*)_{p^*} -$ (IV*)

wobei
E, E*, F, F*, G und G* unabhängig voneinander für eine natürliche oder unnatürliche Aminosäure, Azaaminosäure oder Iminosäure stehen;
n, n*, o, o*, p und p* unabhängig voneinander 0 oder 1 bedeuten;
D für $R^1$ oder einen Rest der Formeln V, VI oder VII und
D* für $R^{1*}$ oder einen Rest der Formeln V*, VI* oder VII* steht

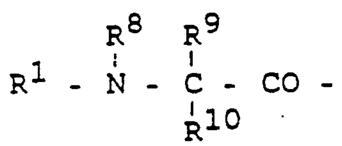 (V) 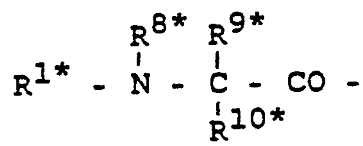 (V*)

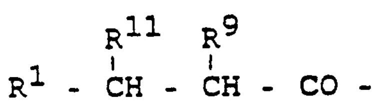 (VI) 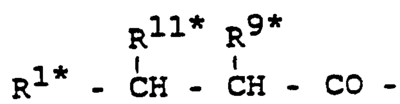 (VI*)

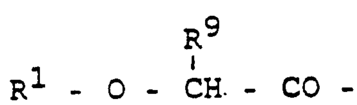 (VII) 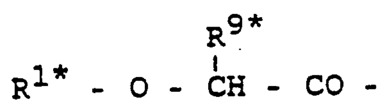 (VII*)

und worin $R^1$ und $R^{1*}$ unabhängig voneinander stehen für

$a_1$)
- Wasserstoff,
- Carboxyl,
- $(C_1\text{-}C_{18})$-Alkyl, das gegebenenfalls einfach oder zweifach ungesättigt ist und das gegebenenfalls durch bis zu 3 gleiche oder verschiedene Reste aus der Reihe
- Mercapto,
- Hydroxy,
- $(C_1\text{-}C_7)$-Alkoxy,
- Carbamoyl,
- $(C_1\text{-}C_8)$-Alkanoyloxy,
- Carboxy,
- $(C_1\text{-}C_7)$-Alkoxycarbonyl,
- F, Cl, Br, I,
- Amino,
- Amidino, das gegebenenfalls durch einen, zwei oder drei $(C_1\text{-}C_8)$-Alkylreste substituiert sein kann,
- Guanidino, das gegebenenfalls durch einen oder zwei Benzyloxycarbonylreste oder durch einen, zwei, drei oder vier $(C_1\text{-}C_8)$-Alkylreste substituiert sein kann,
- $(C_1\text{-}C_7)$-Alkylamino,
- Di-$(C_1\text{-}C_7)$-Alkylamino,
- $(C_1\text{-}C_6)$-Alkoxycarbonylamino,
- $(C_7\text{-}C_{15})$-Aralkoxycarbonyl,
- $(C_7\text{-}C_{15})$-Aralkoxycarbonylamino,

- Phenyl-($C_1$-$C_4$)-alkoxy,
- 9-Fluorenylmethoxycarbonylamino,
- ($C_1$-$C_6$)-Alkylsulfonyl,
- ($C_1$-$C_6$)-Alkylsulfinyl,
- ($C_1$-$C_6$)-Alkylthio,
- Hydroxamino,
- Hydroximino,
- Sulfamoyl,
- Sulfo,
- Carboxamido,
- Formyl,
- Hydrazono,
- Imino,
- einen Rest $CONR^{12}R^{13}$ bzw. $CONR^{12*}R^{13*}$,
- durch bis zu sechs Hydroxy oder
- durch bis zu fünf ($C_1$-$C_8$)-Alkanoyloxy substituiert ist;
- mono-, bi- oder tricyclisches ($C_3$-$C_{18}$)-Cycloalkyl,
- ($C_3$-$C_{18}$)-Cycloalkyl-($C_1$-$C_6$)-alkyl wobei der Cycloalkylteil jeweils gegebenenfalls durch einen oder zwei gleiche oder verschiedene Reste aus der Reihe
- F, Cl, Br, I,
- Carboxy,
- Carbamoyl,
- Carboxymethoxy,
- Hydroxy,
- ($C_1$-$C_7$)-Alkoxy,
- ($C_1$-$C_7$)-Alkyl,
- ($C_1$-$C_7$)-Alkyloxycarbonyl,
- Amino,
- ($C_1$-$C_6$)-Alkylamino-($C_1$-$C_6$)-alkyl,
- Di-($C_1$-$C_6$)-Alkylamino-($C_1$-$C_6$)-alkyl,
- Amidino,
- Hydroxamino,
- Hydroximino,
- Hydrazono,
- Imino,
- Guanidino,
- ($C_1$-$C_6$)-Alkoxysulfonyl,
- ($C_1$-$C_6$)-Alkoxysulfinyl,
- ($C_1$-$C_6$)-Alkoxycarbonylamino,
- ($C_6$-$C_{12}$)-Aryl-($C_1$-$C_4$)-Alkoxycarbonylamino,
- ($C_1$-$C_7$)-Alkylamino,
- Di-($C_1$-$C_7$)-alkylamino und
- Trifluormethyl

substituiert ist;
- ($C_6$-$C_{14}$)-Aryl,
- ($C_6$-$C_{14}$)-Aryl-($C_1$-$C_6$)-alkyl,
- ($C_6$-$C_{14}$)-Aryloxy-($C_1$-$C_6$)-alkyl oder
- ($C_6$-$C_{14}$)-Aryl-($C_3$-$C_8$)-cycloalkyl, worin der Arylteil jeweils gegebenenfalls durch einen, zwei oder drei gleiche oder verschiedene Reste aus der Reihe
- F, Cl, Br, I,
- Hydroxy,
- Mono-, Di- oder Trihydroxy-($C_1$-$C_4$)-alkyl,
- Trifluormethyl,
- Formyl,
- Carboxamido,
- Mono- oder Di-($C_1$-$C_4$)-alkylaminocarbonyl,
- Nitro,
- ($C_1$-$C_7$)-Alkoxy,

- $(C_1-C_7)$-Alkyl,
- $(C_1-C_7)$-Alkoxycarbonyl,
- Amino,
- $(C_1-C_7)$-Alkylamino,
- Di-$(C_1-C_7)$-alkylamino,
- Carboxy,
- Carboxymethoxy,
- Amino-$(C_1-C_7)$-alkyl,
- $(C_1-C_7)$-Alkylamino-$(C_1-C_7)$-alkyl,
- Di-$(C_1-C_7)$-alkylamino-$(C_1-C_7)$-alkyl,
- $(C_1-C_7)$-Alkoxycarbonylmethoxy,
- Carbamoyl,
- Sulfamoyl,
- $(C_1-C_7)$-Alkoxysulfonyl,
- $(C_1-C_8)$-Alkylsulfonyl,
- Sulfo-$(C_1-C_8)$-alkyl,
- Guanidino-$(C_1-C_8)$-alkyl und
- $(C_1-C_6)$-Alkoxycarbonylamino substituiert ist;
- Het,
- Het-$(C_1-C_6)$-alkyl,
- Het-$(C_3-C_8)$-cycloalkyl,
- Het-$(C_3-C_8)$-cycloalkyl-$(C_1-C_4)$-alkyl,
- Het-$(C_3-C_8)$-cycloalkoxy-$(C_1-C_4)$-alkyl,
- Het-thio-$(C_1-C_6)$-alkyl,
- Het-thio-$(C_3-C_8)$-cycloalkyl,
- Het-thio-$(C_3-C_8)$-cycloalkyl-$(C_1-C_4)$-alkyl, wobei Het jeweils für den Rest eines 5- bis 7-gliedrigen monocyclischen oder 8- bis 10-gliedrigen bicyclischen Ringsystems steht, das benzanelliert, aromatisch, teilhydriert oder vollständig hydriert sein kann, das als Heteroelemente einen, zwei, drei oder vier verschiedene Reste aus der Gruppe N, O, S, NO, SO, $SO_2$ enthalten kann, das mit 1 bis 6 Hydroxy substituiert sein kann und das gegebenenfalls wie bei $(C_6-C_{14})$-Aryl unter $a_1$) definiert und/oder mit Oxo, mono-, di- oder trisubstituiert ist,

oder einen Rest $NR^{12}R^{13}$ bzw. $NR^{12*}R^{13*}$ bedeuten oder,

$a_2$)

- einen Rest der Formel VIII beziehungsweise VIII*

$R^{1a}$ - W (VIII)

$R^{1a*}$ - W * (VIII*)

worin $R^{1a}$ und $R^{1a*}$ wie $R^1$ bzw. $R^{1*}$ unter $a_1$) definiert sind und W bzw. W* für -CO-, -CS-, O-CO-, $-SO_2-$, -SO-, -S-, $-NHSO_2-$, -NHCO-, -CH(OH)-, -N(OH)- oder -CO-V- wobei V ein Peptid mit 1 bis 10 Aminosäuren bedeutet, stehen;

oder worin $R^1$ und $R^{1*}$ unabhängig voneinander zusammen mit $R^{11}$ bzw. $R^{11*}$ und den diese tragenden Atomen mono- oder bicyclische, gesättigte oder teilweise ungesättigte Ringsysteme mit 5-12 Ringgliedern bilden, die außer Kohlenstoff noch 1 Schwefelatom enthalten können, welches gegebenenfalls zum Sulfoxid oder Sulfon oxidiert sein kann;

$a_3$)

- einen Glycosylrest, bevorzugt einen Glucofuranosyl- oder Glucopyranosyl-Rest, der sich von natürlich vorkommenden Aldotetrosen, Aldopentosen, Aldohexosen, Ketopentosen, Ketohexosen, Desoxyaldosen, Aminoaldosen und Oligosacchariden sowie deren Stereoisomeren ableitet;

$R^2$ und $R^{2*}$

unabhängig voneinander definiert sind wie $R^1$ bzw. $R^{1*}$ unter $a_1$) oder $a_2$) oder zusammen mit $R^4$ bzw. $R^{4*}$ und den diese tragenden Atomen mono- oder bicyclische, gesättigte oder teilweise ungesättigte Ringsysteme mit 5 bis 12 Ringgliedern bilden, oder zusammen mit $R^3$ bzw. $R^{3*}$ und den diese tragenden Atomen cyclische, gesättigte oder teilweise ungesättigte Ringsysteme mit 3 bis 12 Ringgliedern bilden;

$R^3$ und $R^{3*}$

unabhängig voneinander

- Wasserstoff oder
- $(C_1-C_3)$-Alkyl bedeuten;

$R^4$ und $R^{4*}$

unabhängig voneinander
- Wasserstoff oder
- $(C_1-C_8)$-Alkyl bedeuten;
$R^5$, $R^{5*}$ und $R^{5**}$
unabhängig voneinander
- Wasserstoff,
- Hydroxy,
- Amino oder
- Carboxy bedeuten, oder
mit $R^6$, $R^{6*}$ bzw. $R^{6**}$ zusammen mit den sie tragenden Kohlenstoffatomen jeweils unabhängig voneinander eine Ketogruppe bilden;
$R^6$, $R^{6*}$ und $R^{6**}$
unabhängig voneinander
- Wasserstoff oder
- $(C_1-C_6)$-Alkyl bedeuten oder
im Falle $l = 0$, $R^6$ und $R^{6*}$ gegebenenfalls eine gemeinsame Bindung ausbilden können;
$R^7$
- Wasserstoff,
- Hydroxy oder
- $(C_1-C_6)$-Alkyl bedeutet;
$R^8$ und $R^{8*}$
unabhänging voneinander
- Wasserstoff oder
- $(C_1-C_8)$-Alkyl bedeuten, oder zusammen mit $R^9$ bzw. $R^{9*}$ und den diese tragenden Atomen mono- oder bicyclische, gesättigte oder teilweise ungesättigte Ringsysteme mit 5-12 Ringgliedern bilden;
$R^9$ und $R^{9*}$
unabhängig voneinander definiert sind wie $R^1$ bzw. $R^{1*}$ unter $a_1$), für Hydroxy oder $(C_1-C_4)$-Alkanoyloxy stehen oder zusammen mit $R^{10}$ bzw. $R^{10*}$ und den diese tragenden Atomen cyclische, gesättigte oder teilweise ungesättigte Ringsysteme mit 3 bis 12 Ringgliedern bilden;
oder
zusammen mit $R^{11}$ bzw. $R^{11*}$ und den diese tragenden Atomen ein mono- oder bicyclisches, gesättigtes oder teilweise ungesättigtes Ringsystem mit 5-12 Ringgliedern bilden, das außer Kohlenstoff noch 1 Schwefelatom enthalten kann, welches gegebenenfalls zum Sulfoxid oder Sulfon oxidiert sein kann; oder 1 Stickstoffatom enthalten kann, wobei das Ringsystem gegebenenfalls durch Amino substituiert sein kann;
$R^{10}$ und $R^{10*}$
unabhängig voneinander
- Wasserstoff oder
- $(C_1-C_6)$-Alkyl bedeuten;
$R^{11}$ und $R^{11*}$
unabhängig voneinander
- Wasserstoff,
- Hydroxy,
- $(C_1-C_4)$-Alkanoyloxy oder
- $(C_1-C_8)$-Alkyl bedeuten;
$R^{12}$, $R^{12*}$, $R^{13}$ und $R^{13*}$
unabhängig voneinander
- Wasserstoff,
- $(C_1-C_8)$-Alkyl, das durch
- Amino,
- $(C_1-C_4)$-Alkylamino,
- Di-$(C_1-C_4)$-alkylamino,
- Mercapto,
- Carboxy,
- Hydroxy oder
- $(C_1-C_4)$-Alkoxy substituiert sein kann,
- $(C_3-C_7)$-Cycloalkyl,
- $(C_1-C_4)$-Alkoxycarbonyl,
- $(C_6-C_{14})$-Aryl, $(C_6-C_{14})$-Aryl-$(C_1-C_4)$-Alkoxycarbonyl, die im Arylteil wie bei $R^1$ bzw. $R^{1*}$ beschrieben,

substituiert sein können,

- Het oder
- Het-($C_1$-$C_4$)-alkyl, wobei Het wie bei $R^1$ bzw. $R^{1*}$ beschrieben definiert ist, bedeuten

oder wobei $R^{12}$ und $R^{13}$ bzw. $R^{12*}$ und $R^{13*}$ zusammen mit dem sie tragenden Stickstoffatomen monocyclische oder bicyclische, gesättigte, teilweise ungesättigte oder aromatische Ringsysteme bilden, die als weitere Ringglieder neben Kohlenstoff noch 1 oder 2 Stickstoffatome, 1 Schwefelatom oder 1 Sauerstoffatom enthalten und durch ($C_1$-$C_4$)-Alkyl substituiert sein können,

wobei

in den vorstehenden Verbindungen der Formel I eine oder mehrere Amidgruppen (-CONH-) der Hauptkette ersetzt sein können durch $-CH_2NR^{14}-$, $-CH_2S-$, $-CH_2O-$, $-OCH_2-$, $-CH_2CH_2-$, -CH=CH-(cis und trans), $-COCH_2-$, $-CH(OH)CH_2-$, $-CH_2SO-$, $-CH_2SO_2-$, -COO-, $-P(O)(OR^{15})CH_2-$ und $-P(O)(OR^{15})NH-$, oder auch durch eine Amidgruppe mit umgekehrter Polarität (-NHCO-);

worin $R^{14}$ und $R^{15}$

unabhängig voneinander stehen für

- Wasserstoff oder
- ($C_1$-$C_4$)-Alkyl;

sowie deren physiologisch verträgliche Salze.

Die in dieser Beschreibung benutzte Nomenklatur folgt der allgemeinen Praxis bei Aminosäuren, das heißt, die Aminogruppe steht links, die Carboxygruppe rechts von jeder Aminosäure. Entsprechendes gilt für Azaaminosäuren und Iminosäuren.

Natürliche oder unnatürliche Aminosäuren können, falls chiral, in der D- oder L-Form vorliegen. Bevorzugt sind $\alpha$-Aminosäuren. Beispielsweise seien genannt:

Aad, Abu, $\gamma$Abu, ABz, 2ABz, $\epsilon$Aca, Ach, Acp, Adpd, Ahb, Aib, $\beta$Aib, Ala, $\beta$Ala, Ala, Alg, All, Ama, Amt, Ape, Apm, Apr, Arg, Asn, Asp, Asu, Aze, Azi, Bai, Bph, Can, Cit, Cys, $(Cys)_2$, Cyta, Daad, Dab, Dadd, Dap, Dapm, Dasu, Djen, Dpa, Dtc, Fel, Gln, Glu, Gly, Guv, hAla, hArg, hCys, hGln, hGlu, His, hIle, hLeu, hLys, hMet, hPhe, hPro, hSer, hThr, hTrp, hTyr, Hyl, Hyp, 3Hyp, Ile, Ise, Iva, Kyn, Lant, Lcn, Leu, Lsg, Lys, $\beta$Lys, $\Delta$Lys, Met, Mim, Min, nArg, Nle, Nva, Oly, Orn, Pan, Pec, Pen, Phe, Phg, Pic, Pro, $\Delta$Pro, Pse, Pya, Pyr, Pza, Qin, Ros, Sar, Sec, Sem, Ser, Thi, $\beta$Thi, Thr, Thy, Thx, Tia, Tle, Tly, Trp, Trta, Tyr, Val, Nal, Tbg, Npg, Chg, Thia, (vgl. z.B. Houben-Weyl, Methoden der organischen Chemie, Band XV/1 und 2, Stuttgart, 1974).

Azaaminosäuren sind von natürlichen oder unnatürlichen Aminosäuren abgeleitet, wobei der Zentralbaustein -CHR-bzw. $-CH_2-$ durch -NR- bzw. -NH- ersetzt ist.

Unter einer Iminosäure werden allgemein natürliche oder unnatürliche Aminosäuren, deren Aminogruppe monosubstituiert ist, verstanden. Besonders seien in diesem Zusammenhang Verbindungen genannt, die durch ($C_1$-$C_8$)-Alkyl, das wiederum gegebenenfalls wie auf den Seiten 4/5 beschrieben, substituiert sind. Ferner kommen Heterocyclen aus der folgenden Gruppe in Betracht:

Pyrrolidin-2-carbonsäure; Piperidin-2-carbonsäure;
1,2,3,4-Tetrahydroisochinolin-3-carbonsäure;
Decahydroisochinolin-3-carbonsäure;
Octahydroindol-2-carbonsäure;
Decahydrochinolin-2-carbonsäure;
Octahydrocyclopenta[b]pyrrol-2-carbonsäure;
2-Aza-bicyclo[2.2.2]octan-3-carbonsäure;
2-Azabicyclo[2.2.1]heptan-3-carbonsäure;
2-Azabicyclo[3.1.0]hexan-3-carbonsäure;
2-Azaspiro[4.4]nonan-3-carbonsäure;
2-Azaspiro[4.5]-decan-3-carbonsäure;
Spiro[(bicyclo[2.2.1]-heptan)-2,3-pyrrolidin-5-carbonsäure];
Spiro[(bicyclo[2.2.2]octan)-2,3-pyrrolidin-5-carbonsäure];
2-Azatricyclo[$4.3.0.1^{6,9}$]decan-3-carbonsäure;
Decahydrocyclohepta[b]pyrrol-2-carbonsäure;
Decahydrocycloocta[b]pyrrol-2-carbonsäure;
Octahydrocyclopenta[c]pyrrol-2-carbonsäure;
Octahydroisoindol-1-carbonsäure;
2,3,3a,4,6a-Hexahydrocyclopenta[b]pyrrol-2-carbonsäure;
2,3,3a,4,5,7a-Hexahydroindol-2-carbonsäure;
Tetrahydrothiazol-4-carbonsäure;
Isoxazolidin-3-carbonsäure; Pyrazolidin-3-carbonsäure;
Hydroxyprolin-2-carbonsäure, die alle gegebenenfalls substituiert sein können:

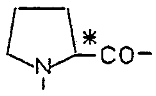 ; 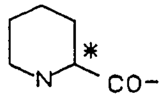

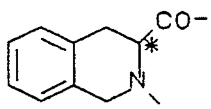 ; 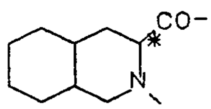 ; 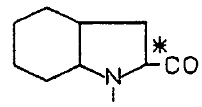 ; 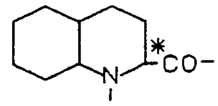 ;

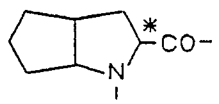 ; 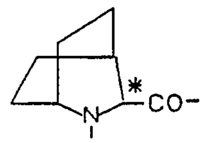 ; 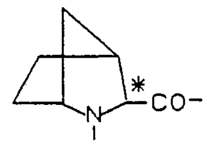 ; 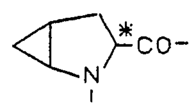 ;

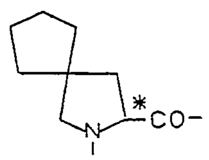 ; 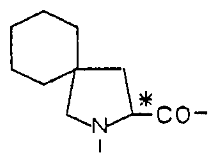 ; 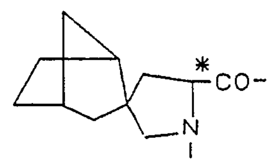 ;

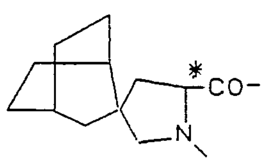 ; 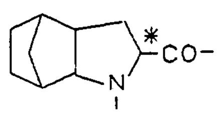 ; 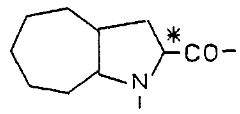 ;

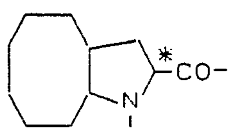 ; 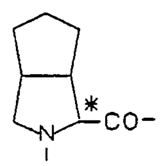 ; 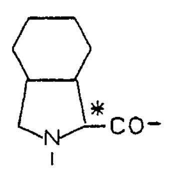 ;

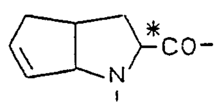 ; 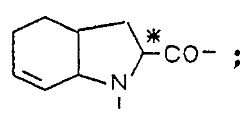 ; 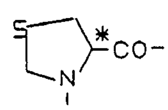 ;

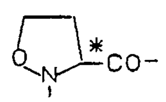 ; 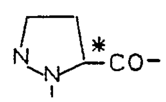 ; 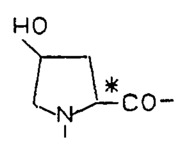 .

Glycosylreste wie vorstehend beschrieben leiten sich insbesondere von natürlichen, im Mikroorganismen, Pflanzen, Tieren oder Menschen vorkommenden D- oder L-Monosacchariden wie Ribose (Rib), Arabinose (Ara), Xylose (Xyl), Lyxose (Lyx), Allose (All), Altrose (Alt), Glucose (Glc), Mannose (Man), Gulose (Gul), Idose (Ido), Galactose (Gal), Talose (Tal), Erythrose (Ery), Threose (Thr), Psicose (Psi), Fructose (Fru), Sorbose (Sor), Tagatose (Tag), Xylulose (Xyu), Fucose (Fuc), Rhamnose (Rha), Olivose (Oli), Oliose (Olo), Mycarose (Myc), Rhodosamin (RN), N-Acetyl-glucosamin (GlcNAc), N-Acetyl-galactosamin (GalNAc), N-Acetyl-mannosamin (ManNAc) oder Disacchariden, wie Maltose (Mal), Lactose (Lac); Cellobiose (Cel), Gentibiose (Gen), N-Acetyl-lactosamin (LacNAc), Chitobiose (Chit), β-Galactopyranosyl-(1-3)-N-acetylgalactosamin und β-Galactopyranosyl-(1-3)- oder -(1-4)-N-acetyl-glucosamin, sowie deren synthetischen Derivaten, wie 2-Desoxy-, 2-Amino-, 2-Acetamido- oder 2-Halogeno-, bevorzugt Bromo-und Jodo-Zucker ab.

Die Chiralitätszentren in den Verbindungen der Formel (I) können die R-, S- oder R,S-Konfiguration aufweisen.

Alkyl kann geradkettig oder verzweigt sein. Entsprechendes gilt für davon abgeleitete Reste, wie z.B. Alkoxy, Alkylthio, Alkylamino, Dialkylamino, Alkanoyl und Aralkyl.

Unter Cycloalkyl werden auch alkylsubstituierte Reste, wie z.B. 4-Methylcyclohexyl oder 2,3-Dimethylcyclopentyl verstanden.

Unter Bicycloalkyl bzw. Tricycloalkyl versteht man einen isocyclischen aliphatischen, nicht aromatischen Rest, der gegebenenfalls unsymmetrisch verteilte Doppelbindungen enthalten kann, gegebenenfalls auch mit offenkettigen aliphatischen Seitenketten substituiert sein kann. Die zwei oder drei Ringe als Komponenten eines derartigen Restes sind kondensiert oder spiroverknüpft und über ein Ring-C-Atom oder ein Seitenketten-C-Atom verknüpft. Beispiele für diese Reste sind Bornyl-, Norbornyl-, Pinanyl-, Norpinanyl-, Caranyl-, Norcaranyl-, Thujanyl-, Adamantyl-, Bicyclo(3.3.0)octyl-, Bicyclo(4:4.0)decyl-, Bicyclo(1.1.0)butyl-, Spiro(3.3)heptyl-Substituenten.

Falls die genannten Cyclen mehr als einen Substituenten tragen, so können diese sowohl cis als auch trans zueinander stehen.

$(C_6-C_{14})$-Aryl ist beispielsweise Phenyl, Naphthyl, Biphenylyl oder Fluorenyl; bevorzugt sind Phenyl und Naphthyl. Entsprechendes gilt für davon abgeleitete Reste, wie z.B. Aryloxy, Aroyl, Aralkyl und Aralkoxy. Unter Aralkyl versteht man einen mit $(C_1-C_6)$-Alkyl verknüpften unsubstituierten oder substituierten $(C_6-C_{14})$-Aryl-Rest, wie z.B. Benzyl, 1- und 2-Naphthylmethyl, wobei Aralkyl jedoch nicht auf die genannten Reste beschränkt wäre.

Reste Het im Sinne vorstehender Definition sind Pyrrolyl, Furyl, Thienyl, Imidazolyl, Pyrazolyl, Oxazolyl, Isoxazolyl, Thiazolyl, Isothiazolyl, Tetrazolyl, Pyridyl, Pyrazinyl, Pyrimidinyl, Indolyl, Isoindolyl, Indazolyl, Phthalazinyl, Chinolyl, Isochinolyl, Chinoxalinyl, Chinazolinyl, Cinnolinyl, β-Carbolinyl, oder ein benzannelliertes, cyclopenta-, cyclohexa- oder cycloheptaannelliertes Derivat dieser Reste.

Diese Heterocyclen können an einem Stickstoffatom durch Oxide; $(C_1-C_7)$-Alkyl z.B. Methyl oder Ethyl; Phenyl; Phenyl-$(C_1-C_4)$-alkyl z.B. Benzyl; und/oder an einem oder mehreren Kohlenstoffatomen durch $(C_1-C_4)$-Alkyl z.B. Methyl; Phenyl; Phenyl-$(C_1-C_4)$-alkyl z.B. Benzyl; Halogen; Hydroxy; $(C_1-C_4)$-Alkoxy, z.B. Methoxy; Phenyl-$(C_1-C_4)$-alkoxy, z.B. Benzyloxy; oder Oxo substituiert und teilweise oder vollständig gesättigt sein.

Derartige Reste sind beispielsweise 2- oder 3-Pyrrolyl; Phenyl-pyrrolyl, z.B. 4- oder 5-Phenyl-2-pyrrolyl; 2-Furyl; 2-Thienyl; 4-Imidazolyl; Methyl-imidazolyl, z.B. 1-Methyl-2-, -4- oder -5-imidazolyl; 1,3-Thiazol-2-yl; 2-, 3-oder 4-Pyridyl; 1-Oxido-2-, -3- oder -4-pyridino; 2-Pyrazinyl; 2-, 4- oder 5-Pyrimidinyl; 2-, 3- oder 5-Indolyl; substituiertes 2-Indolyl, z.B. 1-Methyl-, 5-Methyl-, 5-Methyl-, 5-Benzyloxy-, 5-Chlor-oder 4,5-Dimethyl-2-indolyl; 1-Benzyl-2- oder -3-indolyl; 4,5,6,7-Tetrahydro-2-indolyl; Cyclohepta[b]-5-pyrrolyl; 2-, 3- oder 4-Chinolyl; 1-, 3- oder 4-Isochinolyl; 1-Oxo-1,2-dihydro-3-isochinolyl; 2-Chinoxalinyl; 2-Benzofuranyl; 2-Benzoxazolyl; Benzothiazolyl; Benz[e]indol-2-yl oder β-Carbolin-3-yl.

Teilhydrierte oder vollständig hydrierte heterocyclische Ringe sind beispielsweise Dihydropyridinyl; Pyrrolidinyl, z.B. 2-, 3- oder 4-N-methylpyrrolidinyl; Piperazinyl; Morpholino; Thiomorpholino; Tetrahydrothiophenyl; Benzodioxolanyl.

Halogen steht für Fluor, Chlor, Brom oder Jod, insbesondere für Fluor oder Chlor.

Unter Salzen von Verbindungen der Formel (I) sind insbesondere pharmazeutisch verwendbare oder nicht-toxische Salze zu verstehen.

Solche Salze werden beispielsweise von Verbindungen der Formel (I), welche saure Gruppen, z.B. Carboxy, enthalten, mit Alkali- oder Erdalkalimetallen gebildet, wie z.B. Na, K, Mg und Ca, sowie mit physiologisch verträglichen organischen Aminen, wie z.B. Triethylamin und Tris-(2-hydroxy-ethyl)-amin.

Verbindungen der Formel (I), welche basische Gruppen, z.B. eine Aminogruppe oder eine Guanidinogruppe enthalten, bilden mit anorganischen Säuren, wie z.B. Salzsäure, Schwefelsäure oder Phosphorsäure und mit organischen Carbon- oder Sulfonsäuren, wie z.B. Essigsäure, Citronensäure, Benzoesäure, Maleinsäure, Fumarsäure, Weinsäure und p-Toluolsulfonsäure, Salze.

Bevorzugt sind Verbindungen der Formel I, worin die Reste und Symbole mit und ohne Stern jeweils identisch sind.

Ebenfalls bevorzugt sind Verbindungen der Formel I, die $C_2$-symmetrisch sind.

Besonders sind ferner Verbindungen der Formel I bevorzugt, in welcher

Y für einen Rest der Formel II oder einen Rest der Formel III steht;

l, m, A, A*, D, D*, n, n*, o, o*, p und p* wie oben definiert sind;

E, E*, F, F*, G und G* unabhängig voneinander für eine natürliche oder unnatürliche α-Aminosäure oder α-Iminosäure stehen;

$R^1$ und $R^{1*}$

unabhängig voneinander stehen für

$a_1$) - Wasserstoff;

- Carboxyl,

- $(C_1-C_{16})$-Alkyl, das gegebenenfalls einfach ungesättigt ist und das gegebenenfalls durch bis zu 2 gleiche oder verschiedene Reste aus der Reihe

- Hydroxy,
- ($C_1$-$C_4$)-Alkoxy,
- Carbamoyl,
- ($C_1$-$C_8$)-Alkanoyloxy,
- Carboxy,
- ($C_1$-$C_4$)-Alkoxycarbonyl,
- F,
- Amino,
- ($C_1$-$C_7$)-Alkylamino,
- Di-($C_1$-$C_7$)-Alkylamino,
- ($C_1$-$C_6$)-Alkoxycarbonylamino,
- Benzyloxycarbonyl
- Benzyloxycarbonylamino,
- 9-Fluorenylmethoxycarbonylamino,
- ($C_1$-$C_4$)-Alkylsulfonyl,
- einen Rest $CONR^{12}R^{13}$ bzw. $CONR^{12*}R^{13*}$,
- durch bis zu sechs Hydroxy oder
- durch bis zu vier ($C_1$-$C_8$)-Alkanoyloxy substituiert ist;
- mono- oder bicyclisches ($C_3$-$C_{12}$)-Cycloalkyl,
- ($C_3$-$C_{12}$)-Cycloalkyl-($C_1$-$C_6$)-alkyl wobei der Cycloalkylteil jeweils gegebenenfalls durch einen oder zwei gleiche oder verschiedene Reste aus der Reihe
- F,
- Carboxy,
- Hydroxy,
- ($C_1$-$C_7$)-Alkoxy,
- ($C_1$-$C_4$)-Alkyl,
- ($C_1$-$C_4$)-Alkyloxycarbonyl,
- Amino,
- ($C_1$-$C_6$)-Alkoxycarbonylamino,
- Benzyloxycarbonylamino
- ($C_1$-$C_4$)-Alkylamino und
- Di-($C_1$-$C_4$)-alkylamino

substituiert ist;
- ($C_6$-$C_{10}$)-Aryl,
- ($C_6$-$C_{10}$)-Aryloxy-($C_1$-$C_6$)-alkyl oder
- ($C_6$-$C_{10}$)-Aryl-($C_1$-$C_6$)-alkyl, worin der Arylteil jeweils gegebenenfalls durch einen, zwei oder drei gleiche oder verschiedene Reste aus der Reihe
- F, Cl, Br,
- Hydroxy,
- Hydroxy-($C_1$-$C_4$)-alkyl,
- Carboxamido,
- Mono- oder Di-($C_1$-$C_4$)-alkylaminocarbonyl,
- ($C_1$-$C_4$)-Alkoxy,
- ($C_1$-$C_4$)-Alkyl,
- ($C_1$-$C_4$)-Alkoxycarbonyl,
- Amino,
- ($C_1$-$C_4$)-Alkylamino,
- Di-($C_1$-$C_4$)-alkylamino,
- Carboxy,
- Carbamoyl,
- ($C_1$-$C_4$)-Alkoxycarbonylamino

substituiert ist;
- Het,
- Het-($C_1$-$C_6$)-alkyl,
- Het-($C_5$-$C_6$)-cycloalkyl,
- Het-thio-($C_1$-$C_4$)-alkyl,
- Het-thio-($C_5$-$C_6$)-cycloalkyl,

wobei Het jeweils für den Rest eines 5- bis 6-gliedrigen monocyclischen oder 8- bis 10-gliedrigen

bicyclischen Ringsystems steht, das aromatisch, teilhydriert oder vollständig hydriert sein kann, das als Heteroelemente einen, zwei, drei oder vier verschiedene Reste aus der Gruppe N, O, S, NO, SO, $SO_2$ enthalten kann, das mit 1 bis 4 Hydroxy substituiert sein kann und das gegebenenfalls wie bei ($C_6$-$C_{10}$)-Aryl unter $a_1$) definiert und/oder mit Oxo mono-, oder di-substituiert ist,
oder einen Rest $NR^{12}R^{13}$ bzw. $NR^{12*}R^{13*}$ bedeuten oder,
$a_2$) - einen Rest der Formel VIII beziehungsweise VIII*
$R^{1a}$ - W (VIII)
$R^{1a*}$ - W* (VIII*)
worin $R^{1a}$ und $R^{1a*}$ wie $R^1$ bzw. $R^{1*}$ unter $a_1$) definiert sind und W bzw. W* für -CO-, -O-CO-, -$SO_2$-, -SO-, -S-, -NHCO- oder -CH(OH)-, stehen;
oder worin $R^1$ und $R^{1*}$ unabhängig voneinander zusammen mit $R^{11}$ bzw. $R^{11*}$ und den diese tragenden Atomen monocyclische, gesättigte oder teilweise ungesättigte Ringsysteme mit 5-8 Ringgliedern bilden, die außer Kohlenstoff noch 1 Schwefelatom enthalten können, welches gegebenenfalls zum Sulfoxid oder Sulfon oxidiert sein kann;
$a_3$) - einen Glycosylrest, der wie oben definiert ist;
$R^2$ und $R^{2*}$
unabhängig voneinander
$b_1$) - Wasserstoff,
- Carboxy,
- ($C_1$-$C_{10}$)-Alkyl, das gegebenenfalls ein- oder zweifach ungesättigt ist und das gegebenenfalls durch bis zu 3 gleiche oder verschiedene Reste aus der Reihe
- Hydroxy,
- ($C_1$-$C_7$)-Alkoxy,
- ($C_1$-$C_7$)-Alkylthio,
- ($C_1$-$C_7$)-Alkylsulfinyl,
- ($C_1$-$C_7$)-Alkylsulfonyl,
- ($C_1$-$C_7$)-Alkanoyloxy,
- Carboxy,
- ($C_1$-$C_7$)-Alkoxycarbonyl,
- Cl, Br,
- Amino,
- Amidino,
- Guanidino,
- N,N'-Di-(benzyloxycarbonyl)-guanidino,
- Carbamoyl,
- ($C_7$-$C_{15}$)-Aralkoxycarbonyl,
- ($C_1$-$C_5$)-Alkoxycarbonylamino,
- ($C_7$-$C_{15}$)-Aralkoxycarbonylamino oder
- 9-Fluorenylmethoxycarbonylamino substituiert ist;
- ($C_3$-$C_{12}$)-Cycloalkyl,
- ($C_3$-$C_{12}$)-Cycloalkyl-($C_1$-$C_3$)-alkyl,
- ($C_6$-$C_{14}$)-Aryl,
- ($C_6$-$C_{14}$)-Aryl-($C_1$-$C_3$)-alkyl, wobei der Aryl-Teil jeweils gegebenenfalls durch einen, zwei oder drei gleiche oder verschiedene Reste aus der Reihe
- F, Cl, Br, I,
- Hydroxy,
- ($C_1$-$C_7$)-Alkoxy,
- ($C_1$-$C_7$)-Alkyl,
- ($C_1$-$C_7$)-Alkoxycarbonyl,
- Amino und
- Trifluormethyl substituiert ist; oder
- Het-($C_1$-$C_6$)-alkyl, wobei Het für den Rest eines 5-oder 6-gliedrigen monocyclischen oder 9- bis 10-gliedrigen bicyclischen, gegebenenfalls teilweise oder vollständig hydrierten Heteroaromaten, mit mindestens 1 C-Atom, 1-4 N-Atomen und/oder 1-2 S-Atomen und/oder 1-2 O-Atomen als Ringglieder steht, der gegebenenfalls wie auf den Seiten 6/7 für den Arylteil beschrieben mono- oder disubstituiert ist, bedeuten; oder
$b_2$) zusammen mit $R^4$ bzw. $R^{4*}$ und den diese tragenden Atomen Pyrrolidin oder Piperidin, die jeweils noch mit Cyclopentyl, Cyclohexyl oder Phenyl annelliert sein können, bilden,

oder zusammen mit $R^3$ bzw. $R^{3*}$ und den diese tragenden Atomen cyclische, gesättigte oder teilweise ungesättigte Ringsysteme mit 3-8 Ringgliedern bilden;

$R^3$ und $R^{3*}$
unabhängig voneinander
- Wasserstoff,
- Methyl oder
- Ethyl bedeuten;

$R^4$ und $R^{4*}$
unabhängig voneinander
- Wasserstoff,
- $(C_1-C_4)$-Alkyl bedeuten;

$R^5$, $R^{5*}$ und $R^{5**}$ unabhängig voneinander wie auf Seite 9 definiert sind;

$R^6$, $R^{6*}$ und $R^{6**}$
unabhängig voneinander
- Wasserstoff,
- $(C_1-C_4)$-Alkyl bedeuten;

$R^7$
- Wasserstoff,
- Hydroxy oder
- $(C_1-C_4)$-Alkyl bedeutet;

$R^8$ und $R^{8*}$
unabhängig voneinander
- Wasserstoff,
- $(C_1-C_8)$-Alkyl bedeuten oder zusammen mit $R^9$ bzw. $R^{9*}$ und den diese tragenden Atomen Pyrrolidin oder Piperidin, die jeweils zusätzlich mit Cyclopentyl, Cyclohexyl oder Phenyl annelliert sein können, bilden;

$R^9$ und $R^{9*}$
unabhängig voneinander definiert sind wie $R^2$ bzw. $R^{2*}$ unter $b_1$), oder
$(C_1-C_8)$-Alkanoyloxy bedeutet oder
zusammen mit $R^{10}$ bzw. $R^{10*}$ und den diese tragenden Atomen cyclische, gesättigte oder teilweise ungesättigten Ringsysteme mit 5 bis 12 Ringgliedern bilden;
oder
zusammen mit $R^{11}$ bzw. $R^{11*}$ und den diese tragenden Atomen ein mono- oder bicyclisches, gesättigtes oder teilweise ungesättigtes Ringsystem mit 5-12 Ringgliedern bilden, das außer Kohlenstoff noch 1 Schwefelatom enthalten kann, welches gegebenenfalls zum Sulfoxid oder Sulfon oxidiert sein kann;

$R^{10}$ und $R^{10*}$
unabhängig voneinander
- Wasserstoff oder
- $(C_1-C_4)$-Alkyl bedeuten;

$R^{11}$ und $R^{11*}$
unabhängig voneinander
- Wasserstoff,
- Hydroxy,
- $(C_1-C_4)$-Alkanoyloxy oder
- $(C_1-C_4)$-Alkyl bedeuten;

$R^{12}$, $R^{12*}$, $R^{13}$ und $R^{13*}$
unabhängig voneinander
- Wasserstoff,
- $(C_1-C_8)$-Alkyl, das durch
- Amino,
- $(C_1-C_4)$-Alkylamino,
- Di-$(C_1-C_4)$-alkylamino,
- Carboxy,
- Hydroxy oder
- $(C_1-C_4)$-Alkoxy substituiert sein kann,
- $(C_1-C_4)$-Alkoxycarbonyl,
- $(C_6-C_{10})$-Aryl, das wie bei $R^1$ bzw. $R^{1*}$ beschrieben substituiert sein kann,
- $(C_6-C_{10})$-Aryl-$(C_1-C_4)$-alkoxycarbonyl,
- Het oder

- Het-$(C_1-C_4)$-alkyl bedeuten, wobei Het wie bei $R^1$ bzw. $R^{1*}$ beschrieben definiert ist,

wobei

in den vorstehenden Verbindungen der Formel I eine oder mehrere Amidgruppen (-CONH-) der Hauptkette ersetzt sein können durch eine Gruppe bestehend aus $-CH_2NR^{14}-$, $-CH_2O-$, $-OCH_2-$, $-CH_2CH_2-$, $-COCH_2-$, $-CH(OH)CH_2-$, -COO- oder auch durch eine Amidgruppe mit umgekehrter Polarität (-NHCO-);

$R^{14}$ für

- Wasserstoff oder
- $(C_1-C_4)$-Alkyl steht;

sowie deren physiologisch verträgliche Salze.

Besonders bevorzugt sind Verbindungen der Formel I in welcher

Y für einen Rest der Formel II oder einen Rest der Formel III steht;

l, m, A, A*, D, D*, n, n*, o, o* wie oben definiert sind, p und p* für 1 stehen;

$R^1$ und $R^{1*}$

unabhängig voneinander stehen für

- Wasserstoff,
- Carboxyl,
- $(C_1-C_{10})$-Alkyl,
- $(C_3-C_8)$-Cycloalkyl,
- $(C_3-C_8)$-Cycloalkyl-$(C_1-C_{10})$-alkyl,
- Phenyl-$(C_1-C_8)$-alkyl, das wie auf den Seiten 19/20 beschrieben im Phenylteil substituiert sein kann,
- gegebenenfalls geschütztes Mono- oder Di-Amino-$(C_1-C_{12})$-alkyl oder Amino-$(C_6-C_{10})$-aryl-$(C_1-C_4)$-alkyl oder Amino-$(C_3-C_{10})$-cycloalkyl-$(C_1-C_4)$-alkyl, wie - 2-Amino-3-phenyl-propyl,
- Mono-, Di-, Tris, Tetra-, Penta- oder Hexahydroxy-$(C_1-C_{10})$-alkyl oder -alkanoyl,
- $(C_1-C_4)$-Alkoxy-$(C_1-C_{10})$-alkyl,
- $(C_1-C_4)$-Alkoxycarbonyl-$(C_1-C_{10})$-alkyl,
- $(C_1-C_{16})$-Alkylsulfonyl,
- $(C_1-C_8)$-Alkylsulfinyl,
- Mono-, Di-, Trihydroxy-$(C_{\cdot}-C_8)$-alkylsulfonyl,
- Mono-, Di-, Trihydroxy-$(C_{\cdot}-C_8)$-alkylsulfinyl,
- Mono-, Di-, Tri- oder Tetra-$(C_{\cdot}-C_8)$-alkanoyloxy-$(C_1-C_{10})$-alkyl,
- $(C_1-C_{14})$-Alkanoyl,
- gegebenenfalls geschütztes Amino-$(C_1-C_{11})$-alkanoyl,
- Di-$(C_1-C_7)$-alkylamino-$(C_2-C_{\cdot\cdot})$-alkanoyl,
- $(C_1-C_9)$-Cycloalkylcarbonyl,
- Aminosubstituiertes $(C_3-C_9)$-Cycloalkylcarbonyl,
- Aminosubstituiertes $(C_3-C_9)$-Cycloalkylsulfonyl,
- $(C_6-C_{10})$-Aryl-$(C_2-C_{\cdot\cdot})$-alkanoyl,
- $(C_6-C_{10})$-Aryloxy-$(C_2-C_{\cdot\cdot})$-alkanoyl,
- gegebenenfalls durch Amino, Halogen, $(C_1-C_7)$-Alkyl, $(C_1-C_7)$-Alkoxy oder $(C_1-C_7)$-Alkoxycarbonyl substituiertes Benzoyl, Benzolsulfonyl oder $(C_6-C_{10})$-Aryl-$(C_1-C_4)$-alkylcarbonyl bzw. -sufonyl,
- $(C_1-C_{10})$-Alkoxycarbonyl,
- substituiertes $(C_{\cdot}-C_{\cdot 0})$-Alkoxycarbonyl, wie
- 2-(Trimethylsilyl)ethoxycarbonyl,
- 2,2,2-Trichlorethoxycarbonyl oder
- 1,1-Dimethyl-2,2,2-trichlorethoxycarbonyl,
- $(C_6-C_{14})$-Aryl-$(C_{\cdot}-C_6)$-alkoxycarbonyl,
- durch gegebenenfalls geschütztes Amino und Hydroxy substituiertes $(C_6-C_{10})$-Aryl-$(C_1-C_8)$-alkyl, $(C_3-C_{10})$-Cycloalkyl-$(C_1-C_8)$-alkyl oder $(C_{\cdot}-C_{10})$-Alkyl, wie
- 2-Amino-1-hydroxy-4-methyl-pentyl,
- 9-Fluorenylmethoxycarbonyl,
- Ketohexosyl,
- Ketopentosyl,
- Desoxyhexoketosyl,
- Desoxypentoketosyl,
- Aldohexosyl,
- Aldopentosyl,
- Desoxyhexoaldosyl,
- Desoxypentoaldosyl,

- 2-Amino-2-desoxyhexosyl,
- 2-Acetamido-2-desoxyhexosyl,
- Lactosyl oder
- Maltosyl wobei die verknüpften Zucker in der Pyranose- oder Furanose-Form vorliegen können,
- Het-($C_1$-$C_6$)-alkyl,
- Het-carbonyl oder -sulfonyl,
- Het-($C_1$-$C_6$)-alkylcarbonyl oder -sulfonyl,
- Het-mercapto-($C_1$-$C_6$)-alkylcarbonyl oder -sulfonyl,

wobei Het jeweils für

Furyl, Thienyl, Benzothienyl, Benzdioxolanyl, Pyrrolyl, Imidazolyl, Isoxazolyl, Thiazolyl, Pyrazolyl, Triazolyl, Pyridyl, Pyrimidyl, Pyrazinyl, Pyridazinyl, Triazinyl, Pyrrolidyl, Piperidyl, Piperazinyl, Morpholino, Thiomorpholino, Tetrahydrofuryl, Tetrahydropyryl, Tetrahydrothienyl, Indolyl, Chinolyl oder Isochinolyl,

wobei diese auch durch eine oder zwei gleiche oder verschiedene Reste aus der Gruppe ($C_1$-$C_4$)-Alkyl, ($C_1$-$C_4$)-Alkoxy, ($C_1$-$C_4$)-Alkoxycarbonyl, ($C_1$-$C_4$)-Alkoxycarbonylamino, Hydroxy, Amino, Mono- oder Di-($C_1$-$C_4$)-Alkylamino und Oxido substituiert sein können;

$R^2$ und $R^{2*}$

unabhängig voneinander

- Wasserstoff,
- Carboxyl,
- ($C_1$-$C_8$)-Alkyl, das gegebenenfalls durch bis zu 2 gleiche oder verschiedene Reste aus der Reihe
- Hydroxy,
- ($C_1$-$C_4$)-Alkoxy,
- ($C_1$-$C_4$)-Alkylthio,
- ($C_1$-$C_4$)-Alkylsulfinyl,
- ($C_1$-$C_4$)-Alkylsulfonyl,
- ($C_1$-$C_4$)-Alkanoyloxy,
- Carboxy,
- ($C_1$-$C_4$)-Alkoxycarbonyl,
- Amino,
- Amidino,
- Guanidino,
- N,N'-Di-(benzyloxycarbonyl)-guanidino,
- Carbamoyl,
- ($C_6$-$C_{10}$)-Aryl-($C_1$-$C_3$)-alkoxycarbonyl,
- ($C_1$-$C_5$)-Alkoxycarbonylamino,
- ($C_6$-$C_{10}$)-Aryl-($C_1$-$C_3$)-alkoxycarbonylamino substituiert ist oder
- ($C_3$-$C_{10}$)-Cycloalkyl,
- ($C_3$-$C_{10}$)-Cycloalkyl-($C_1$-$C_3$)-alkyl,
- ($C_1$-$C_4$)-Alkyl-($C_3$-$C_{10}$)-cycloalkyl-($C_1$-$C_3$)-alkyl,
- ($C_6$-$C_{10}$)-Aryl,
- ($C_6$-$C_{10}$)-Aryl-($C_1$-$C_3$)-alkyl, wobei der Arylteil jeweils gegebenenfalls durch einen, zwei oder drei gleiche oder verschiedene Reste aus der Reihe
- F, Cl, Br,
- Hydroxy,
- ($C_1$-$C_4$)-Alkoxy,
- ($C_1$-$C_4$)-Alkyl,
- ($C_1$-$C_4$)-Alkoxycarbonyl und
- Amino substituiert ist, oder
- Het-($C_1$-$C_4$)-alkyl, wobei Het wie bei $R^1$ bzw $R^{1*}$ definiert ist, bedeuten,

$R^3$ und $R^{3*}$

unabhängig voneinander

- Wasserstoff oder
- Methyl bedeuten;

$R^4$ und $R^{4*}$

unabhängig voneinander

- Wasserstoff oder
- Methyl bedeuten;

$R^5$, $R^{5*}$ und $R^{5**}$

unabhängig voneinander
- Wasserstoff,
- Hydroxy,
- Amino oder
- Carboxy bedeuten;
$R^6$, $R^{6*}$ und $R^{6**}$
unabhängig voneinander
- Wasserstoff oder
- Methyl bedeuten;
$R^7$
- Wasserstoff,
- Hydroxy oder
- Methyl bedeutet;
$R^8$ und $R^{8*}$
unabhängig voneinander
- Wasserstoff,
- Methyl, Ethyl oder n-Propyl bedeuten oder zusammen mit $R^9$ bzw $R^{9*}$ und den diese tragenden Atomen ein 1,2,3,4-Tetrahydroisochinolin oder ein 2-Azabicyclooctan-Gerüst bilden;
$R^9$ und $R^{9*}$
unabhängig voneinander definiert sind wie $R^2$ bzw. $R^{2*}$ auf den Seiten 27/28 oder
($C_1$-$C_8$)-Alkanoyloxy bedeuten oder
zusammen mit $R^{10}$ bzw. $R^{10*}$ und den diese tragenden Atomen cyclische Ringsysteme mit 5 bis 7 Ringgliedern bilden;
oder zusammen mit $R^{11}$ bzw $R^{11*}$ ein Thiochromansystem bilden, dessen Schwefelatom gegebenenfalls zum Sulfon oxidiert sein kann;
$R^{10}$ und $R^{10*}$
unabhängig voneinander
- Wasserstoff oder
- Methyl bedeuten;
$R^{11}$ und $R^{11*}$ wie auf Seite 24 definiert sind;
wobei
in den vorstehenden Verbindungen der Formel I eine oder mehrere Amidgruppen (-CONH-) der Hauptkette ersetzt sein können wie auf Seite 24 definiert;
$R^{14}$ für
- Wasserstoff oder
- Methyl steht;
sowie deren physiologisch verträglichen Salze.

Ferner sind besonders bevorzugt Verbindungen der Formel I, in welcher
$R^1$ und $R^{1*}$
unabhängig voneinander stehen für
$a_1$) - Wasserstoff,
- Carboxyl,
- ($C_1$-$C_{16}$)-Alkylsulfonyl, wie
- Methylsulfonyl,
- tert.-Butylsulfonyl,
- Isopropylsulfonyl oder
- Hexadecylsulfonyl,
- ($C_1$-$C_8$)-Alkylsulfinyl,
- ($C_1$-$C_8$)-Mono-, -Di- oder -Tri-Hydroxyalkylsulfonyl, wie
- 2-Hydroxyethylsulfonyl oder
- 2-Hydroxypropylsulfonyl,
- Hydroxy-($C_1$-$C_{10}$)-alkanoyl, wie
- 2-Hydroxypropionyl,
- 3-Hydroxypropionyl,
- 3- Hydroxybutyryl oder
- 2-Hydroxy-3-methylbutyryl,
- Mono-, Di-, Tri- oder Tetra-Hydroxy-($C_1$-$C_4$)-alkyl, wie
- 1,2,3-Trihydroxypropyl,

- 1,2-Dihydroxyethyl oder
- Hydroxymethyl,
- ($C_1$-$C_8$)-Alkanoyloxy-($C_1$-$C_{10}$)-alkyl, wie
- Acetoxymethyl,
- 1,2-Diacetoxyethyl,
- 1,2,3-Triacetoxypropyl,
- ($C_1$-$C_{14}$)-Alkanoyl, wie
- n-Decanoyl,
- Formyl,
- Acetyl,
- Propionyl,
- Pivaloyl,
- Isovaleryl,
- Isobutyryl oder
- Tetradecanoyl,
- Amino-($C_1$-$C_{12}$)-alkanoyl, wie
- 3-Amino-3,3-dimethyl-propionyl,
- 4-Aminobutyryl,
- 5-Aminopentanoyl,
- 6-Aminohexanoyl oder
- 12-Aminododecanoyl,
- N-($C_1$-$C_4$)-Alkoxycarbonylamino-($C_1$-$C_8$)-alkyl, wie
- 4-N-tert.-Butoxycarbonylaminobutyryl,
- 5-N-tert.-Butoxycarbonylaminopentanoyl oder
- 6-N-tert.-Butoxycarbonylaminohexanoyl,
- Di-($C_1$-$C_7$)-alkylamino-($C_2$-$C_{11}$)-alkanoyl, wie
- Dimethylaminoacetyl,
- ($C_3$-$C_9$)-Cycloalkylcarbonyl, wie
- Cyclopropylcarbonyl,
- Cyclobutylcarbonyl,
- Cyclopentylcarbonyl oder
- Cyclohexylcarbonyl,
- Amino-($C_3$-$C_8$)-Cycloalkylcarbonyl, wie
- 2-Aminocyclopropylcarbonyl
- 3-Aminocyclobutylcarbonyl,
- 3-Aminocyclopentylcarbonyl oder
- 4-Aminocyclohexylcarbonyl,
- Amino-($C_3$-$C_8$)-Cycloalkylsulfonyl, wie
- 3-Aminocyclopentylsulfonyl oder
- 4-Aminocyclohexylsulfonyl,
- Phenyl,
- ($C_6$-$C_{10}$)-Aryl-($C_2$-$C_{11}$)-alkanoyl, wie
- 1-Naphthylacetyl,
- Phenylacetyl,
- Phenylpropanoyl oder
- Phenylbutanoyl,
- ($C_6$-$C_{10}$)-Aryloxy-($C_2$-$C_{11}$)-alkanoyl, wie
- 1-Naphthyloxycarbonyl oder
- Phenyloxycarbonyl,
- gegebenenfalls durch Halogen, Amino, ($C_1$-$C_7$)-Alkyl, ($C_1$-$C_7$)-Alkoxy oder ($C_1$-$C_7$)-Alkoxycarbonyl substituiertes Benzoyl oder Benzolsulfonyl wie
- 4-Chlorbenzoyl,
- 4-Methylbenzoyl,
- 2-Methoxycarbonylbenzoyl,
- 4-Methoxybenzoyl,
- Benzolsulfonyl oder
- 4-Methylphenylsulfonyl,
- gegebenenfalls durch Halogen, Amino, ($C_1$-$C_7$)-Alkyl, ($C_1$-$C_7$)-Alkoxy oder ($C_1$-$C_7$)-Alkoxycarbonyl substitu-

iertes Benzylsulfonyl, Benzylsulfinyl oder Benzylthio, wie
- 4-Chlorbenzylsulfonyl,
- Benzylsulfinyl oder
- 4-Chlorbenzylthio,
- Amino,
- $(C_1-C_4)$-Alkoxycarbonylamino,
- $(C_1-C_{12})$-Alkanoyl, das durch Hydroxy, Amino und gegebenenfalls durch Phenyl oder Cyclohexyl substituiert ist, wie
- 2-Amino-1-hydroxy-4-methyl-pentyl,
- gegebenenfalls geschütztes aminosubstituiertes $(C_6-C_{10})$-Aryl- oder $(C_3-C_{10})$Cycloalkyl-$(C_1-C_4)$-alkyl oder $(C_1-C_8)$-Alkyl, wie
- 2-Amino-3-phenyl-propyl oder
- N-tert.-Butoxycarbonyl-2-amino-3-phenyl-propyl,
- $(C_1-C_{10})$-Alkoxycarbonyl, wie
- Methoxycarbonyl,
- Ethoxycarbonyl,
- Isobutoxycarbonyl oder
- tert.-Butoxycarbonyl,
- substituiertes $(C_1-C_{10})$-Alkoxycarbonyl, wie
- 2-(Trimethylsilyl)-ethoxycarbonyl,
- 2,2,2-Trichlorethoxycarbonyl oder
- 1,1-Dimethyl-2,2,2-trichlorethoxycarbonyl,
- $(C_6-C_{14})$-Aryl-$(C_1-C_6)$-alkoxycarbonyl, wie
- Benzyloxycarbonyl,
- 1- oder 2-Naphthylmethoxycarbonyl oder
- 9-Fluorenylmethoxycarbonyl,
- 1-Desoxyhexoketosyl oder 1-Desoxypentoketosyl, wie
- 1-Desoxyfructos-1-yl, 1-Desoxysorbos-1-yl oder 1-Desoxyribulos-1-yl
- Hexosyl oder Pentosyl, wie
- Mannosyl, Glucosyl oder Galactosyl,
- Xylosyl, Ribosyl oder Arabinosyl,
- 6-Desoxyhexosyl, wie
- Rhamnosyl, Fucosyl oder Desoxyglucosyl,
- Aminozuckerreste, wie
- 2-Amino-2-desoxyglucosyl,
- 2-Acetamido-2-desoxyglucosyl,
- 2-Amino-2-desoxygalactosyl oder
- 2-Acetamido-2-desoxygalactosyl,
- Lactosyl,
- Maltosyl,

wobei die verknüpften Zucker in der Pyranose- oder der Furanose-Form vorliegen können,
- Het wie
- 2-Pyridyl,
- 4-Pyridyl,
- 2-(N-Oxidopyridyl) oder
- 4-(N-Oxidopyridyl),
- Het-carbonyl oder Het-sulfonyl, wie
- Piperidino-4-carbonyl,
- Morpholino-4-carbonyl,
- Pyrrolyl-2-carbonyl,
- Pyridyl-3-carbonyl,
- Chinolyl-2-carbonyl,
- 4-tert.-Butoxycarbonylamino-1-piperidylcarbonyl,
- 4-Amino-1-piperidylcarbonyl,
- 4-tert.-Butoxycarbonylamino-1-piperidylsulfonyl oder
- 4-Amino-1-piperidylsulfonyl,
- Het-$(C_1-C_6)$-alkyl, wie
- 2-Pyridyl-$(C_1-C_6)$-alkyl,

- 3-Pyridyl-($C_1$-$C_6$)-alkyl oder
- 4-Pyridyl-($C_1$-$C_6$)-alkyl,
- Het-($C_1$-$C_6$)-alkanoyl oder Het-($C_1$-$C_6$)-alkylsulfonyl, wie
- 2-Pyridyl-($C_1$-$C_6$)-alkanoyl,
- 3-Pyridyl-($C_1$-$C_6$)-alkanoyl,
- 4-Pyridyl-($C_1$-$C_6$)-alkanoyl,
- 2-Pyridyl-($C_1$-$C_6$)-alkylsulfonyl,
- 3-Pyridyl-($C_1$-$C_6$)-alkylsulfonyl oder
- 4-Pyridyl-($C_1$-$C_6$)-alkylsulfonyl,
- Het-mercapto-($C_1$-$C_3$)-alkylcarbonyl, wie
- 2-Pyridylthioacetyl,

wobei Het jeweils steht für
- Pyrrolyl,
- Imidazolyl,
- Pyridyl,
- Pyrimidyl,
- Pyrrolidyl,
- Piperidyl,
- Morpholino,
- Chinolyl oder
- Isochinolyl,

wobei dieser auch durch einen oder zwei gleiche oder verschiedene Reste aus der Gruppe ($C_1$-$C_4$)-Alkyl, ($C_1$-$C_4$)-Alkoxycarbonyl, ($C_1$-$C_4$)-Alkoxycarbonylamino, Hydroxy, Amino, Mono- oder Di-($C_1$-$C_4$)-Alkylamino substituiert sein kann;

$R^2$ und $R^{2*}$ unabhängig voneinander stehen für
- Wasserstoff,
- Carboxyl,
- Methyl, Ethyl, Isopropyl, n-Propyl, n-Butyl, Isobutyl, sec.-Butyl, n-Pentyl, n-Hexyl,
- Cyclohexyl,
- Cyclopentylmethyl, Cyclohexylmethyl, Cycloheptylmethyl,
- 4-Methylcyclohexylmethyl,
- 1-Dekahydronaphthylmethyl, 2-Dekahydronaphthylmethyl,
- Phenyl,
- Benzyl,
- 2-Phenylethyl,
- 1-Naphthylmethyl, 2-Naphthylmethyl,
- 2-Methylbenzyl, 3-Methylbenzyl, 4-Methylbenzyl,
- 2,4,6-Trimethylbenzyl,
- 4-tert.-Butylbenzyl,
- 4-tert.-Butoxybenzyl
- 4-Hydroxybenzyl,
- 4-Methoxybenzyl,
- 2,4-Dimethoxybenzyl,
- 3,4-Dihydroxybenzyl,
- 3,4-Dimethoxybenzyl,
- (Benzdioxolan-5-yl)methyl,
- 4-Chlorbenzyl,
- Hydroxymethyl,
- 1-Hydroxyethyl,
- 4-Pyridyl,
- 4-(N-Oxidopyridyl),
- 2-Pyridylmethyl, 3-Pyridylmethyl, 4-Pyridylmethyl, 2-(4-Pyridyl)ethyl,
- 2-Thienylmethyl, 3-Thienylmethyl,
- 2-(2-Thienyl)ethyl, 2-(3-Thienyl)ethyl,
- Indol-2-yl-methyl, Indol-3-yl-methyl,
- (1-Methyl-imidazol-4-yl)methyl,
- Imidazol-4-yl-methyl, Imidazol-1-yl-methyl,
- 2-Thiazolylmethyl,

- 3-Pyrazolylmethyl,
- 4-Pyrimidylmethyl,
- 2-Benzo[b]thienylmethyl, 3-Benzo[b]thienylmethyl,
- 2-Furylmethyl,
- 2-(Methylthio)ethyl,
- 2-(Methylsulfinyl)ethyl oder
- 2-(Methylsulfonyl)ethyl,

$R^3$, $R^{3*}$, $R^4$, $R^{4*}$, $R^6$, $R^{6*}$, $R^{10}$ und $R^{10*}$
Wasserstoff bedeuten;
$R^5$ und $R^{5*}$
unabhängig voneinander stehen für
- Wasserstoff
- Hydroxy oder
- Amino;

$R^7$ - Wasserstoff,
- Hydroxy oder
- Methyl bedeutet;

$R^8$ und $R^{8*}$
unabhängig voneinander bedeuten
- Wasserstoff oder

zusammen mit $R^9$ bzw. $R^{9*}$ und den diese tragenden Atomen ein 1,2,3,4-Tetrahydroisochinolin oder 2-Azabicyclooctan-Gerüst bilden;
$R^9$ und $R^{9*}$
unabhängig voneinander
wie $R^2$ bzw. $R^{2*}$ definiert sind oder
- Hydroxy,
- Acetoxy,
- tert.-Butoxymethyl,
- 3-Guanidinopropyl,
- Carbamoylmethyl, Carbamoylethyl,
- Carboxymethyl, Carboxyethyl,
- Mercaptomethyl,
- (1-Mercapto-1-methyl)ethyl,
- Aminomethyl, 2-Aminoethyl, 3-Aminopropyl, 4-Aminobutyl,
- N,N-Dimethylamino,
- N,N′-Di-(benzyloxycarbonyl)-guanidino-propyl,
- 2-Benzyloxycarbonylethyl, Benzyloxycarbonylmethyl,
- tert.-Butylsulfonylmethyl

oder
- 4-Benzylcarbonylaminobutyl bedeuten;

$R^{11}$ und $R^{11*}$ unabhängig voneinander
- Wasserstoff,
- Hydroxy oder
- Acetoxy bedeuten;

wobei
in den vorstehenden Verbindungen dieser Erfindung eine oder mehrere Amidgruppen (-CONH-) der Hauptkette ersetzt sein können durch $-CH_2NR_{14}-$ oder $-CH(OH)CH_2-$;
$R^{14}$ für
- Wasserstoff oder
- Methyl steht;

sowie deren physiologisch verträgliche Salze.

Ganz besonders bevorzugt sind Verbindungen der Formel I,
worin $R^1$ und $R^{1*}$
unabhängig voneinander stehen für
$a_1$) - Wasserstoff,
- Carboxyl,
- $(C_1-C_{16})$-Alkylsulfonyl, wie
- Methylsulfonyl,

- tert.-Butylsulfonyl,
- Isopropylsulfonyl oder
- Hexadecylsulfonyl,
- ($C_1$-$C_8$)-Mono- oder -Dihydroxyalkylsulfonyl, wie
- 2-Hydroxyethylsulfonyl oder
- 2-Hydroxypropylsulfonyl,
- Mono-, Di- oder Trihydroxy-($C_1$-$C_3$)-alkyl, wie
- 1,2,3-Trihydroxypropyl,
- 1,2-Dihydroxyethyl oder
- Hydroxymethyl,
- ($C_1$-$C_8$)-Alkoxycarbonyl, wie
- Methoxycarbonyl,
- Ethoxycarbonyl,
- Isobutoxycarbonyl oder
- tert.-Butoxycarbonyl,
- ($C_1$-$C_{14}$)-Alkanoyl, wie
- Tetradecanoyl,
- Amino-($C_1$-$C_{12}$)-alkanoyl, wie
- 12-Aminododecanoyl,
- ($C_1$-$C_{10}$)-Aryloxy-($C_1$-$C_4$)-alkylcarbonyl, wie
- 1- oder 2-Naphthyloxyacetyl,
- ($C_6$-$C_{10}$)-Aryl-($C_1$-$C_4$)-alkoxycarbonyl, wie
- Benzyloxycarbonyl oder
- 1- oder 2-Naphthylmethoxycarbonyl
- ($C_6$-$C_{10}$)-Aryl-($C_1$-$C_4$)-alkylcarbonyl, wie
- 1- oder 2- Naphthylacetyl,
- 9-Fluorenylmethoxycarbonyl,
- ($C_1$-$C_4$)-Alkanoyloxy-($C_1$-$C_6$)-alkyl, wie
- Acetoxymethyl,
- 1,2-Diacetoxyethyl,
- 1,2,3-Triacetoxypropyl,
- Phenyl
- gegebenenfalls durch Halogen, Amino, ($C_1$-$C_4$)-Alkyl, oder Methoxy substituiertes Benzolsulfonyl, wie
- Benzolsulfonyl oder
- 4-Methylphenylsulfonyl,
- gegebenenfalls durch Halogen, Amino, ($C_1$-$C_4$)-Alkyl, oder Methoxy substituiertes Benzylsulfonyl, -sulfinyl oder -thio, wie
- 4-Chlorbenzylsulfonyl,
- Benzylsulfinyl oder
- 4-Chlorbenzylthio,
- Het,wie
- 2- oder 4-Pyridyl oder
- 2- oder 4-(N-Oxidopyridyl),
- Het-sulfonyl,wie
- 4-tert.-Butoxycarbonylamino-1-piperidylsulfonyl oder
- 4-Amino-1-piperidylsulfonyl,
- Het-($C_1$-$C_4$)-alkylsulfonyl, wie
- 2-(4-Pyridyl)-ethylsulfonyl,
- Het-($C_1$-$C_4$)-alkanoyl, wie
- 2-Pyridylacetyl,
- 3-Pyridylacetyl,
- 4-tert.-Butoxycarbonylamino-1-piperidylcarbonyl,
- 4-Amino-1-piperidylcarbonyl oder
- 2-Chinolylcarbonyl,
- Het-mercapto-($C_1$-$C_3$)-alkylcarbonyl, wie
- 2-Pyridylthioacetyl,

wobei Het jeweils steht für
- Pyrrolyl,

- Imidazolyl,
- Pyridyl,
- Pyrimidyl,
- Pyrrolidyl,
- Chinolyl,
- Isochinolyl,
- Piperidyl oder
- Morpholino,

wobei dieser Rest auch durch durch einen oder zwei gleiche oder verschiedene Reste aus der Gruppe Methyl, Amino und ($C_1$-$C_4$)-Alkoxycarbonylamino substituiert sein kann,

- Amino-($C_3$-$C_6$)-cycloalkylcarbonyl, wie
- 2-Aminocyclopropylcarbonyl
- 3-Aminocyclobutylcarbonyl,
- 3-Aminocyclopentylcarbonyl,
- 4-Aminocyclohexylcarbonyl,
- ($C_1$-$C_8$)-Alkanoyl, das durch Hydroxy und Amino und gegebenenfalls durch Phenyl oder Cyclohexyl substituiert ist, wie

2-Amino-1-hydroxy-4-methyl-pentyl,

- gegebenenfalls geschütztes aminosubstituiertes Phenyl-oder Cyclohexyl-($C_1$-$C_6$)-alkyl, wie
- 2-Amino-3-phenyl-propyl oder
- N-tert.-Butoxycarbonyl-2-Amino-3-phenyl-propyl,
- Amino,
- ($C_1$-$C_4$)-Alkoxycarbonylamino,
- Benzyloxycarbonylamino,
- 1-Desoxyhexoketosyl oder 1-Desoxypentoketosyl, wie
- 1-Desoxyfructos-1-yl, 1-Desoxysorbos-1-yl oder
- 1-Desoxyribulos-1-yl,
- Hexosyl oder Pentosyl, wie
- Mannosyl, Glucosyl oder Galactosyl, oder
- Xylosyl, Ribosyl oder Arabinosyl, wobei die verknüpften Zucker in der Pyranose- oder der Furanose-Form vorliegen können,

$R^2$ und $R^{2*}$ unabhängig voneinander stehen für

- Wasserstoff,
- Methyl, Ethyl, Isopropyl, n-Propyl, n-Butyl, Isobutyl, sec.-Butyl, Pentyl, Hexyl,
- Cyclopentylmethyl, Cyclohexylmethyl,
- 4-Methylcyclohexylmethyl,
- Benzyl,
- 2-Phenylethyl,
- 1-Naphthylmethyl, 2-Naphthylmethyl,
- 2-Methylbenzyl, 3-Methylbenzyl, 4-Methylbenzyl,
- 2,4,6-Trimethylbenzyl,
- 4-tert.-Butylbenzyl,
- 4-Methoxybenzyl,
- 3,4-Dihydroxybenzyl,
- 2,4-Dimethoxybenzyl,
- 3,4-Dimethoxybenzyl,
- 3,4-Methylendioxylbenzyl,
- 2-Pyridylmethyl, 3-Pyridylmethyl, 4-Pyridylmethyl oder
- 2-(4-Pyridyl)ethyl,

$R^3$, $R^{3*}$, $R^4$, $R^{4*}$, $R^6$, $R^{6*}$, $R^7$, $R^{10}$ und $R^{10*}$
Wasserstoff bedeuten;
$R^5$ und $R^{5*}$
unabhängig voneinander

- Wasserstoff oder
- Hydroxy bedeuten;

$R^8$ und $R^{8*}$ unabhängig voneinander wie auf Seite 36 definiert sind,
$R^9$ und $R^{9*}$
unabhängig voneinander wie $R^9$ bzw. $R^{9*}$ auf Seite 36 definiert sind;

$R^{11}$ und $R^{11*}$ unabhängig voneinander wie auf Seite 37 definiert sind, sowie deren physiologisch verträgliche Salze.

Ferner sind insbesondere bevorzugt Verbindungen der Formel I in welcher

Y für einen Rest der Formel III steht;
l 0 oder 1 bedeutet;
m 1 bedeutet;
A, A*, D und D* wie oben definiert sind;
n, n*, o, o*, p und p* unabhängig voneinander 0 oder 1 bedeuten;
E, E*, F, F*, G und G* unabhängig voneinander für eine Aminosäure aus der Reihe Val, Lys, Lys(Z), Phe, Chg, Ser, Asn, Gly, Ile, Tbg, Nva oder Npg bedeuten;
$R^1$ und $R^{1*}$ unabhängig voneinander
- Wasserstoff,
- Carboxyl,
- Methylsulfonyl,
- tert.-Butylsulfonyl,
- tert.-Butoxycarbonyl,
- 2-Hydroxyethylsulfonyl,
- 1,2,3-Trihydroxypropyl,
- 1,2,3-Triacetoxypropyl,
- Benzyloxycarbonyl,
- 4-Methylphenylsulfonyl,
- 4-Chlorbenzylthio,
- Benzylsulfinyl,
- 4-Chlorbenzylsulfonyl,
- Hexadecylsulfonyl,
- 4-Amino-1-piperidyl-sulfonyl,
- N-tert.-butoxycarbonyl-4-amino-1-piperidyl-sulfonyl,
- 4-Amino-1-piperidyl-carbonyl,
- N-tert.-butoxycarbonyl-4-amino-1-piperidyl-carbonyl,
- 2-Amino-3-phenyl-propyl,
- N-tert.-Butoxycarbonyl-2-amino-3-phenyl-propyl,
- 2-Amino-1-hydroxy-4-methyl-pentyl,
- Desoxyfructos-1-yl,
- Mannofuranosyl,
- 4-Aminocyclohexylcarbonyl,
- 2-Chinolylcarbonyl,
- 1-Naphthylacetyl,
- 1-Naphthyloxyacetyl,
- 1-(4-Pyridyl)-ethylsulfonyl,
- 12-Aminododecanoyl,
- 4-(N-Oxidopyridyl),
- 4-Pyridyl,
- Tetradecanoyl,
- 2-Pyridylacetyl,
- 4-Pyridylthio-acetyl,
- Phenyl,
- Amino oder
- tert.-Butoxycarbonylamino bedeuten;

$R^2$ und $R^{2*}$ unabhängig voneinander
- Wasserstoff,
- 2-(4-Pyridyl)ethyl,
- Isopropyl,
- Isobutyl,
- n-Pentyl,
- Benzyl,
- 3,4-Methylendioxybenzyl,
- 2,4-Dimethoxybenzyl,
- 4-tert.-Butylbenzyl,

- 2-Phenylethyl oder
- Cyclohexylmethyl bedeuten;

$R^3$, $R^{3*}$, $R^4$, $R^{4*}$, $R^6$, $R^{6*}$, $R^7$, $R^{10}$ und $R^{10*}$
- Wasserstoff

bedeuten;

$R^5$ und $R^{5*}$ unabhängig voneinander
- Wasserstoff oder
- Hydroxy bedeuten;

$R^8$ und $R^{8*}$
- Wasserstoff bedeuten, oder zusammen mit $R^9$ bzw. $R^{9*}$ und den diese tragenden Atomen ein 1,2,3,4-Tetrahydrochinolin-3,4-diyl-System bilden;

$R^9$ und $R^{9*}$ unabhängig voneinander
- Wasserstoff,
- Hydroxy,
- Acetoxy,
- n-Propyl,
- Isopropyl,
- Isobutyl,
- Aminomethyl,
- 4-Aminobutyl,
- Hydroxymethyl,
- tert.-Butoxymethyl,
- Aminocarbonylmethyl,
- 2-Benzyloxycarbonyl-ethyl,
- 4-Benzyloxycarbonylamino-butyl,
- N,N'-Di-(benzyloxycarbonyl)-guanidino-propyl,
- Cyclohexyl,
- Cyclohexylmethyl,
- Benzyl,
- 2-Phenyl-ethyl
- 4-Hydroxy-benzyl,
- 4-Methoxy-benzyl,
- 4-tert.-Butoxy-benzyl,
- 1-Naphthylmethyl,
- 2-Thienylmethyl,
- 1-Imidazolyl-methyl,
- 3-Indolyl-methyl,
- 4-Pyridylmethyl
- 4-(N-Oxidopyridyl)methyl
- 2-Methylthio-ethyl,
- 2-Methylsulfonyl-ethyl
- tert.-Butylsulfonyl-methyl oder
- 2-Carboxyl-ethyl bedeuten;

$R^{11}$ und $R^{11*}$ unabhängig voneinander
- Wasserstoff,
- Hydroxy oder
- Acetoxy bedeuten;

wobei in den vorstehenden Verbindungen eine oder mehrere Amidgruppen (-CONH-) der Hauptkette ersetzt sein können durch $-CH_2NH-$ oder $-CH(OH)CH_2-$;
sowie deren physiologisch verträgliche Salze.

Ebenfalls ganz bevorzugt genannt seien Verbindungen der Formel I worin

$l = 0$;

$m = 1$;

$n + o + p = 1$;

D und D* für einen Rest der Formel VI bzw. VI* stehen;

$R^1$ und $R^{1*}$
- $(C_1-C_{12})$-Alkylsulfonyl, das gegebenenfalls durch bis zu 3 gleiche oder verschiedene Resten aus der Reihe

- Hydroxy,
- Amino oder
- Carboxy substituiert sein kann, bedeuten;

$R^2$ und $R^{2*}$ unabhängig voneinander

- Wasserstoff,
- Carboxyl,
- Methyl, Ethyl, Isopropyl, n-Propyl, n-Butyl, Isobutyl, sec.-Butyl, Pentyl, Hexyl,
- Cyclohexyl,
- Cyclopentylmethyl, Cyclohexylmethyl, Cycloheptylmethyl,
- 4-Methylcyclohexylmethyl,
- 1-Dekahydronaphthylmethyl, 2-Dekahydronaphthylmethyl,
- Phenyl,
- Benzyl,
- 2-Phenylethyl,
- 1-Naphthylmethyl, 2-Naphthylmethyl,
- 2-Methylbenzyl, 3-Methylbenzyl, 4-Methylbenzyl,
- 2,4,6-Trimethylbenzyl,
- 4-tert.-Butylbenzyl,
- 4-tert.-Butoxybenzyl
- 4-Hydroxybenzyl,
- 4-Methoxybenzyl,
- 2,4-Dimethoxybenzyl,
- 3,4-Dihydroxybenzyl,
- 3,4-Dimethoxybenzyl,
- (Benzdioxolan-4-yl)methyl,
- 4-Chlorbenzyl,
- Hydroxymethyl,
- 1-Hydroxyethyl,
- 2-Pyridylmethyl, 3-Pyridylmethyl, 4-Pyridylmethyl, 2-(4-Pyridyl)ethyl,
- 2-Thienylmethyl, 3-Thienylmethyl,
- 2-(2-Thienyl)ethyl, 2-(3-Thienyl)ethyl,
- Indol-2-yl-methyl, Indol-3-yl-methyl,
- (1-Methyl-imidazol-4-yl)methyl,
- Imidazol-4-yl-methyl, Imidazol-1-yl-methyl,
- 2-Thiazolylmethyl,
- 3-Pyrazolylmethyl,
- 4-Pyrimidylmethyl,
- 2-Benzo[b]thienylmethyl, 3-Benzo[b]thienylmethyl,
- 2-Furylmethyl,
- 2-(Methylthio)ethyl,
- 2-(Methylsulfinyl)ethyl oder
- 2-(Methylsulfonyl)ethyl bedeuten;

$R^3$, $R^{3*}$, $R^4$, $R^{4*}$, $R^6$, $R^{6*}$, $R^{11}$ und $R^{11*}$

- Wasserstoff bedeuten;

$R^5$ und $R^{5*}$

- Hydroxy bedeuten;

$R^9$ und $R^{9*}$

wie $R^9$ bzw. $R^{9*}$ auf Seite 44 definiert sind;

sowie Verbindungen der Formel I, worin

$l = 0$;

$m = 1$;

$n + o + p = 1$;

D und D* für einen Rest der Formel VII bzw. VII* stehen;

$R^1$ und $R^{1*}$

einen Hexosyl- oder Pentosylrest bzw. einen 1-Desoxyhexoketosyl oder 1-Desoxypentoketosyl-Rest, der wie oben definiert ist, bedeuten;

$R^2$ und $R^{2*}$

- Wasserstoff,

- $(C_1-C_8)$-Alkyl,
- $(C_3-C_{10})$-Cycloalkyl-$(C_1-C_6)$-alkyl oder
- $(C_6-C_{10})$-Aryl-$(C_1-C_4)$-alkyl, die je mit bis zu 3 gleichen oder verschiedenen Resten aus $(C_1-C_4)$-Alkyl oder $(C_1-C_4)$-Alkoxy substituiert sein können,

bedeuten;

$R^3$, $R^{3*}$, $R^4$, $R^{4*}$, $R^6$, $R^{6*}$, $R^{11}$ und $R^{11*}$
- Wasserstoff bedeuten;

$R^5$ und $R^{5*}$
- Hydroxy bedeuten;

$R^9$ und $R^{9*}$
wie $R^9$ bzw. $R^{9*}$ auf Seite 44 definiert sind.

Die vorliegende Erfindung betrifft weiterhin ein Verfahren zur Herstellung von Verbindungen der Formel (I), das dadurch gekennzeichnet ist, daß man ein Fragment mit endständiger Carboxylgruppe oder dessen reaktives Derivat mit einem entsprechenden Fragment mit freier Aminogruppe kuppelt, gegebenenfalls zum Schutz weiterer funktioneller Gruppen (eine) temporär eingeführte Schutzgruppe(n) abspaltet und die so erhaltene Verbindung gegebenenfalls in ihr physiologisch verträgliches Salz überführt.

Fragmente einer Verbindung der Formel (I) mit einer endständigen Carboxylgruppe besitzen z.B. die nachstehenden Formeln:

D - OH (VIII)
D - E - OH (IX)
D - F - OH (X)
D - G - OH (XI)
D - E - F - OH (XII)
D - E - G - OH (XIII)
D - F - G - OH (XIV)
D - E - F - G - OH (XIVa)

Entsprechendes gilt für die analogen, mit einem Stern versehenen Reste.

Fragmente einer Verbindung der Formel (I) mit einer endständigen Aminogruppe besitzen z.B. die nachstehenden Formeln:

H - Z - H (XV)
H - G - Z - G* - H (XVI)
H - F - Z - F* - H (XVIa)
H - E - Z - E* - H (XVIb)
H - F - G - Z - G* - F* - H (XVII)
H - E - G - Z - G* - E* - H (XVIIa)
H - E - F - Z - F* - E* - H (XVIIb)
H - E - F - G - Z - G* - F* - E* - H (XVIII)

wobei Z für einen Rest der Formel (XIX) steht:

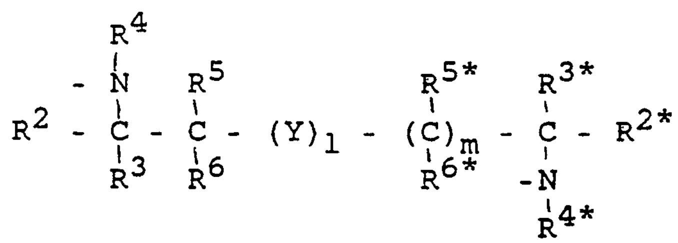
(XIX)

Im Falle nicht symmetrischer Zielmolekühle können auch andere Fragmente außer denen der Formeln XV bis XVIII, die eventuell an einer endständigen Aminogruppe geschützt sind, zum Einsatz kommen.

Methoden, die zur Herstellung einer Amidbindung geeignet sind, werden z.B. in Houben-Weyl, Methoden der organischen Chemie, Band 15/2; Bodanszky et al., Peptide Synthesis. 2nd ed. (Wiley & Sons, New York 1976) oder Gross, Meienhofer, The Peptides: Analysis, synthesis, biology (Academic Press, New York 1979) beschrieben. Vorzugsweise werden die folgenden Methoden herangezogen: Aktivestermethode mit N-Hydroxy-succinimid, 1-Hydroxybenzotriazol oder 3-Hydroxy-4-oxo-3,4-dihydro-1,2,3-benzotriazin als Alkoholkomponente, Kupplung mit einem Carbodiimid wie Dicyclohexylcarbodiimid (DCC) oder mit n-Propanphosphonsäureanhydrid (PPA) und die Gemischt-Anhydrid-Methode mit Pivaloylchlorid oder Chlorameisensäureethylester oder -isobutylester, oder Kupplung mit Phosphonium-Reagenzien, wie Benzotriazol-1-yl-oxy-tris-(dimethylamino)-phosphoniumhexafluorophosphat (BOP) oder Uronium-Reagenzien, wie 2-(1H-Benzotriazol-1-yl)-1,1,3,3-tetramethyluronium tetrafluoroborat (TBTU).

Fragmente der Formel (VIII) bzw. (VIII*), sofern sie unter

a) Formel (V) bzw. (V*) fallen, werden nach den allgemeinen Methoden zur Herstellung von Aminosäuren synthetisiert;

b) Formel (VI) bzw (VI*) fallen, werden z. B. ausgehend von den entsprechenden Aminosäuren synthetisiert, wobei deren Chiralitätszentrum erhalten bleibt. Diazotierung bei -20 °C bis 50 °C in verd. Mineralsäuren führt zu α-Bromcarbonsäuren oder über die Milchsäuren zu α-Trifluormethansulfonyloxy-Carbonsäuren die mit einem $R^1$ und $R^{11}$ bzw. $R^{1*}$ und $R^{11*}$ tragenden Nucleophil umgesetzt werden können, oder werden z. B. ausgehend von Malonestern hergestellt, deren Alkylierung mono- oder disubstituierte Malonester liefert, die nach Verseifung durch Decarboxylierung in die gewünschten Derivat überführt werden.

c) Formel (VII) bzw. (VII*) fallen werden ausgehend von den entsprechenden α-Aminosäuren synthetisiert, wobei deren Chiralitätszentrum erhalten bleibt. Diazotierung bei -20 °C bis 50 °C in verdünnten Mineralsäuren führt zu Milchsäuren, die mit einem $R^1$ bzw. $R^{1*}$ tragenden Elektrophil umgesetzt werden können.

Fragmente der Formeln (IX), (X), (XI), (XII) und (XIII), (XIV) und (XIVa) werden nach den allgemeinen, bekannten Methoden zur Herstellung von Aminosäuren und Peptiden synthetisiert.

Fragmente der Formel (XV) werden ausgehend von optisch aktiven α-Aminosäuren oder Zuckern bzw. deren Abkömmlingen dargestellt. Zum Beispiel werden zur Herstellung von Fragmenten mit m = 1, l = 0, $R^5 = R^{5*} = OH$ und $R^6 = R^{6*} = H$ die Aminosäuren in bekannter Art und Weise in N-geschützte Aminosäurealdehyde überführt (B. Castro et al. Synthesis 1983, 676) und reduktiv mit geeigneten Metallen, Metallsalzen oder auch elektrochemisch zu N-geschützten Diaminodiolen umgesetzt. Die N-geschützten Aldehyde werden dazu z.B. in Tetrahydrofuran gelöst und bei -30 °C bis 60 °C,vorzugsweise -10 °C bis 30 °C, durch Zusatz einer Lösung von Samarium-(II)-jodid in Tetrahydrofuran in die N-geschützten Diaminodiol-Verbindungen überführt.

Abspaltung der Schutzgruppen liefert die Verbindungen der Formel (XV). Man erhält Diastereomerengemische bezüglich der OH-tragenden Zentren, die in an sich bekannter Weise, beispielsweise durch fraktionierte Kristallisation und/oder durch Chromatographie getrennt werden.

Bei Synthese aus Zucker(-derivate)n bleiben die Chiralitätszentren des Ausgangsmaterials erhalten oder werden invertiert. OH-Gruppen, die erhalten bleiben sollen, werden in geeigneter Weise geschützt, die anderen werden durch Umsatz mit z.B. einem Sulfonsäurechlorid oder nach Mitsunobu (Synthesis (1981), 1-28) aktiviert und können durch Nucleophile ausgetauscht werden. Die gewünschten Produkte fallen hier in stereochemisch einheitlicher Form an.

Ausgehend z.B. von D-Mannit werden die Hydroxygruppen des Polyols in Position 3 und 4 durch Behandeln mit Aceton/Schwefelsäure und anschließend mit wäßriger Essigsäure als Acetonid geschützt. Durch Umsatz der beiden endständigen OH-Gruppen mit p-Toluolsulfonsäurechlorid/Pyridin und Behandlung mit Kaliumkarbonat in Methanol erhält man das 1,2R-5R,6-Diepoxy-3,4-O-isopropyliden-3R,4R-diol (Y. Le Merrer et al., Tetrahedron Lett. 26, (1985) 319-322). Behandlung des Diepoxids mit Cupraten in z.B. Tetrahydrofuran führt zur Öffnung der Epoxide und Einführung von Substituenten in Position 1 und 6. Nach Aktivierung der Hydroxygruppen in Position 2 und 5 durch Reaktion mit z.B. einem Sulfonsäurechlorid werden beide durch Umsatz mit einem Azid ausgetauscht. Reduktion der beiden Azidgruppen durch z.B. katalytische Hydrierung und Abspaltung der Acetonid-Schutzgruppe mit HCl/Methanol liefert die Verbindungen des Restes (XV).

Fragmente der Formel (XV) mit m = 1, l = 1 und Y = Rest der Formel III werden derart erhalten, daß N-geschütze Aminosäurealdehyde (s.o.) unter reduktiven Bedingungen (z.B. $NaBH_3CN$) mit einem geeigneten Amin umgesetzt werden.

Dabei werden die Aldehyde in z.B. Methanol gelöst und mit z.B. Ammoniumacetat und z.B. Natriumcyanoborhydrid als Reduktionsmittel umgesetzt. Anschließende Abspaltung der Schutzgruppen liefert den gewünschten Baustein.

Fragmente der Formel XV mit m = 0, l = 0, $R^5 = OH$, $R^6 = H$ werden derart erhalten, daß geeignet Nitroverbindungen mit Basen wie z.B. Tetramethylguanidin deprotoniert und an N-geschützte Aminosäurealdehyde (s.o.) addiert werden. Reduktion der Nitrogruppe mit z.B. Raney-Nickel und Schutzgruppen-Abspaltung liefert die Verbindungen der Formel (XV) als Diastereomere, die, wie oben beschrieben getrennt werden.

Die Fragmente der Formeln XVI, XVIa, XVIb, XVII, XVIIa, XVIIb und XVIII werden nach allgemein bekannten Methoden zur Herstellung von Aminosäuren und Peptiden synthetisiert.

In den Verbindungen der Formel I können eine oder mehrere Amidgruppen durch $-CH_2NR^{14}-$, $-CH_2S-$,

$-CH_2O-$, $OCH_2-$, $-CH_2CH_2-$, $-CH=CH-$ (cis und trans), $-COCH_2-$, $-CH(OH)CH_2-$, $-CH_2SO-$, $-CH_2SO_2-$, $-COO-$, $-P(O)(OR^{15})CH_2-$, $-P(O)(OR^{15})_2NH$-oder -NH-CO- ersetzt sein.

Peptidanaloge dieser Art können nach bekannten Verfahren hergestellt werden, die beispielsweise folgenden Literaturstellen entnommen werden können:

A.F. Spatola in "Chemistry and Biochemistry of Amino Acids Peptides and Proteins" 1983 (B. Weinstein et al. eds.) Marcel Dekker, New York, S. 267 (Reviewartikel);
J.S. Morley, Trends Pharm Sci. (1980) S. 463-468 (Reviewartikel);
D. Hudson et al., Int. J. Pept. Prot. Res. (1979), 14, 177-185 ($-CH_2NH-$, $-CH_2CH_2-$);
A.F. Spatola et al., Life Sci. (1986), 38, 1243-1249 ($-CH_2-S-$);
M.M. Hann, J. Chem. Soc. Perkin Trans.I (1982) 307-314 ($-CH=CH-$, cis und trans);
J.K. Whitesell et al., Chirality 1, (1989) 89-91 ($-CH=CH$-trans)
R.G. Almquist et al., J. Med. Chem. (1980), 23, 1392-1398 ($-COCH_2-$);
C. Jennings-White et al., Tetrahedron Lett. (1982) 23, 2533 ($-COCH_2-$);
M. Szelke et al., EP-A 45665 (1982), CA: 97: 39405 ($-CH(OH)CH_2-$);
M.W. Holladay et al., Tetrahedron Lett. (1983) 24, 4401-4404 ($-CH(OH)CH_2-$);
V.J. Hruby, Life Sci. (1982), 31, 189-199 ($-CH_2-S-$);
N.E. Jacobsen, P.A. Barlett, J. Am. Chem. Soc. (1981) 103, 654-657 (-P(O)(OR)NH-).

Die zur Herstellung von Verbindungen der Formel I erforderlichen Vor- und Nachoperationen wie Einführung und Abspaltung von Schutzgruppen sind literaturbekannt und sind z.B. in T.W. Greene "Protective Groups in Organic Synthesis" (John Wiley & Sons, New York, 1981) beschrieben. Salze von Verbindungen der Formel I mit salzbildenden Gruppen werden in an sich bekannter Weise hergestellt, indem man z.B. eine Verbindung der Formel I mit einer basischen Gruppe mit einer stöchiometrischen Menge einer geeigneten Säure oder Verbindungen der Formel I mit einer sauren Gruppe mit einer stöchiometrischen Menge einer geeigneten Base umsetzt. Stereoisomerengemische, insbesondere Diastereomerengemische, die gegebenenfalls bei der Synthese von Verbindungen der Formel I anfallen, können in an sich bekannter Weise durch fraktionierte Kristallisation oder durch Chromatographie getrennt werden.

Die erfindungsgemäßen Verbindungen der Formel (I) weisen enzymhemmende Eigenschaften auf. Insbesondere hemmen sie die Wirkung retroviraler Aspartylproteasen wie die der HIV-Protease. Ihre enzyminhibitorische Wirkung, die im milli- bis subnanomolaren Bereich liegt, kann wie folgt bestimmt werden.

**Testprinzip:**

Als Substrat der HIV-Protease diente bisher unter anderen das Heptapeptid H-Ser-Phe-Asn-Phe-Pro-Gln-Ile-OH (P.L. Darke et al., Biophys. Res. Commun. 156 (1988) 297-303). Die HIV-Protease spaltet das Substrat dabei zwischen dem zweiten Phe und Pro.

Überraschenderweise wurde nun gefunden, daß Substitution von Prolin durch 5-Oxaprolin in dieser Sequenz zu einem Substrat führt, das wesentlich schneller von der HIV-Protease gespalten werden kann und damit eine schnellere Analyse mit geringerem Enzymbedarf erlaubt.

**Allgemeine Vorschrift für die Austestung von Inhibitoren der HIV-Proteasen:**

a) Herstellung der Substratlösung:

2 mg H-Ser-Phe-Asn-Phe-Opr-Gln-Ile-OH (H-Opr-OH = 5-Oxaprolin) werden in 1 ml MGTE15-Puffer gelöst (evtl. Anwendung von Ultraschall) und anschließend über einen Sterilfilter (0,45 µm) filtriert.

b) Herstellung der Inhibitorlösung:

Vom Inhibitor werden das 2,5-fache der gewünschten Molarität je ml Lösung eingewogen und mit DMSO (10% des Endvolumens) gelöst. Man verdünnt mit MGTE15-Puffer bis zum Endvolumen und filtriert über Sterilfilter (0,45 µm).

c) Herstellung der Proteaselösung:

5 µl der HIV-Proteaselösung werden nach Bedarf mit MGTE25-Puffer verdünnt.

d) Testdurchführung:

Man pipettiert je 10 µl der Substratlösung in Reagenzgläser (16x100) mit Schraubdeckel. Zum Blindversuch werden 10 µl MGTE15-Puffer, der 10 % DMSO enthält, pipettiert. Die übrigen Reagenzgläser werden mit je 10 µl der Inhibitorlösungen versetzt. Man inkubiert 5-10 Minuten bei 37° C und gibt dann zu jeder Probe 5 µl der Proteaselösung. Nach 2 Stunden Reaktion bei 37° C werden von jeder Probe 10 oder 20 µl (je nach Empfindlichkeit des HPLC-Gerätes) abpipettiert, in Mikrovials gefüllt und mit 120 µl des HPLC-Laufmittels verdünnt.

e) Bedingungen für die HPLC-Analyse:

Laufmittelsystem: 80% 0,1 M Phosphorsäure pH 2,5
20% (w/w) Acetonitril
Säule: Merck ®LICHROSORB RP18 (5 µm) 250x4
Fluß: 1ml/min
Temperatur der Säule: 42° C
Detektorparameter: 215 nm, 0,08 AUF, 18,2° C
Analysenzeit: 11 Minuten
Retentionszeit des Substrates: 8,1 Minuten
Retentionszeit des N-terminalen Tetrapeptids: 3,9 Minuten

f) Benötigte Lösungsmittel:

1) MGTE15-Puffer:
20 mM Morpholinoethansulfonsäure (MES)
15 % (w/v) Glycerin
0,1% (v/v) Triton X 100
5 mM EDTA
0,5 M NaCl
1 mM Phenylmethylsulfonylfluorid (PMSF)
2) MGTE25-Puffer:
Zusammensetzung ähnlich wie beim MGTE15-Puffer mit folgender Abweichung:
25 % (w/v) Glycerin,
zusätzlich 1 mM Dithiothreit (DTT)
In einen Erlenmeyerkolben wiegt man MES, EDTA, NaCl, DTT und PMSF ein, löst in wenig Wasser und stellt auf pH 6 ein. In einen Meßkolben wiegt man die entsprechende Menge Glycerin ein und pipettiert ®Triton X 100 dazu. Man überführt die wäßrige Lösung in den Meßkolben und füllt mit Wasser auf.
3) HPLC-Laufmittel:
Man stellt sich aus ortho-Phosphorsäure (FLUKA puriss. p.a.) eine 0,1 M Lösung her. Mit Triethylamin (FLUKA puriss. p.a.) wird diese Lösung genau auf pH 2,5 eingestellt. Das Gewicht der Lösung wird bestimmt und die entsprechende Menge Acetonitril (Abzug!) zugewogen. Gut durchmischen und ca. 5 Minuten mit Helium 5.0 entgasen.

g) Auswertung:

Unter den hier gewählten Bedingungen trennen sich die Heptapeptide von dem bei der enzymatischen Spaltung entstehenden N-terminalen Tetrapeptid. Der %-Gehalt des Tetrapeptid-peaks in Bezug auf Summe Tetrapeptid + Heptapeptid entspricht der Spaltrate. Die nachfolgenden $IC_{50}$-Werte geben an, bei welcher Inhibitorkonzentration die Spaltrate halbiert ist.

| Bsp. Nr. | $IC_{50}$ | Bsp. Nr. | $IC_{50}$ |
|---|---|---|---|
| 1 | 10 nM | 18 | 1,2 nM |
| 5 | 3,6 µM | 19 | 0,7 nM |
| 6 | 8,8 nM | 21 | 220 nM |
| 7 | 18 nM | 25 | 18 µM |
| 8 | 30 µM | 28 | 3 µM |
| 10 | 17 nM | 30 | 30 nM |
| 11 | 0,8 nM | 33 | 20 µM |
| 13 | 1,3 nm | 39 | 1,3 nM |
| 14 | 1,0 nM | 40 | 13 nM |
| 15 | 400 nM | 43 | 1,0 nM |
| 16 | 0,85 nM | 45 | 1,5 µM |
| 17 | 0,85 nM | 48 | 80 µM |

| Bsp. Nr. | $IC_{50}$ | Bsp. Nr. | $IC_{50}$ |
|---|---|---|---|
| 49 | 1,2 nM | 104 | 24 nM |
| 50 | 45 nM | 105 | 19 nM |
| 51 | 0,8 nM | 106 | 85 nM |
| 52 | 3,2 nM | 107 | 8,5 nM |
| 53 | 4,0 nM | 108 | 280 nM |
| 54 | 260 nM | 109 | 5,0 nM |
| 55 | 1,3 nM | 110 | 1,0 nM |
| 58 | 49 nM | 113 | 40 nM |
| 59 | 47 nM | 115 | 2,2 µM |
| 61 | 400 nM | 116 | 1,7 nM |
| 63 | 6,5 nM | 117 | 19 nM |
| 65 | 1,8 nM | 118 | 1,2 nM |
| 72 | 30 nM | 119 | 10 µM |
| 74 | 1,7 nM | 120 | 2,0 nM |
| 75 | 19 nM | 121 | 22 nM |
| 76 | 0,29 nM | 123 | 32 nM |
| 77 | 9,2 nM | 124 | 11 nM |
| 78 | 1,8 nM | 125 | 0,75 nM |
| 80 | 28 nM | 127 | 46 nM |
| 82 | 9 nM | 131 | 40 µM |
| 83 | 10 nM | 132 | 20 µm |
| 84 | 110 nM | 142 | 140 nM |
| 85 | 1,9 nM | 143 | 2,2 nM |
| 86 | 2,2 nM | 145 | 95 nM |
| 87 | 1,6 nM | 146 | 100 nM |
| 88 | 1 µM | 148 | 36 nM |
| 89 | 1,8 nM | 149 | 360 nM |
| 90 | 2,2 nM | 150 | 95 nM |
| 91 | 1,3 nM | 151 | 4 nM |
| 93 | 22 nM | 152 | 1 nM |
| 94 | 6,5 nM | 154 | 1 nM |
| 95 | 380 nM | 155 | 10 nM |
| 97 | 36 nM | 156 | 30 nM |
| 98 | 1 µM | | |
| 99 | 15 nM | | |
| 100 | 400 nM | | |
| 101 | 1,4 nM | | |
| 102 | 38 nM | | |

Das Zielpeptid wurde mit einem Peptid-Synthesizer Modell 430 A der Fa. Applied Biosystems unter Verwendung der Fmoc-Methode an einem mit Fmoc-Ile-OH veresterten p-Benzyloxybenzylalkohol-Harz der Fa. Novabiochem (Beladung ca. 0,5 mmol/g Harz) stufenweise aufgebaut. Es wurde 1 g des Harzes eingesetzt und die Synthese mit Hilfe eines für die Fmoc-Methode modifizierten Synthese-Programmes durchgeführt.

Man verwendet folgende Aminosäure-Derivate: Fmoc-Gln-OH, Fmoc-Opr-OH, Fmoc-Phe-OObt, Fmoc-Asn-OH und Fmoc-Ser(tBu)-OObt. Zur Synthese von Fmoc-Opr-OH wurde H-Opr-OtBu nach der Methode von Vasella et al. (J.C.S. Chem. Comm. 1981, 97-98) synthetisiert und mit Fmoc-OSu in Dioxan/Wasser (1:1) in Gegenwart von $NaHCO_3$ umgesetzt. Die anschließende Spaltung des tert.-Butylesters mit Trifluoressigsäure liefert Fmoc-Opr-OH.

In die Cartridges des Synthesizers wurden jeweils 1 mmol der Aminosäurederivate mit freier Carboxylgruppe zusammen mit 0,95 mmol HOObt eingewogen. Die Voraktivierung dieser Aminosäuren erfolgte direkt in den Cartridges durch Lösen in 4 ml DMF und Zugabe von 2 ml einer 0,55 molaren Lösung von Diisopropylcarbodiimid in DMF. Die HOObt-Ester der anderen Aminosäuren wurden in 6 ml NMP gelöst und dann ebenso wie die in situ voraktivierten Aminosäuren an das vorher mit 20 % Piperidin in DMF entblockierte Harz gekuppelt. Nach beendeter Synthese wurde das Peptid unter gleichzeitiger Entfernung der Seitenkettenschutzgruppen mit Trifluoressigsäure unter Verwendung von Thioanisol und Ethandithiol als Kationenfänger vom Harz abgespalten. Der nach Abziehen der Trifluoressigsäure erhaltene Rückstand wurde mehrfach mit Essigester digeriert und zentrifugiert.

Der verbliebene Rückstand wurde an einem alkylierten Dextrangel mit 10 %iger Essigsäure chromatographiert. Die das reine Peptid enthaltende Fraktion wurde vereinigt und gefriergetrocknet.
Massenspektrum (FAB): 854 ($M+H^+$)
Aminosäureanalyse Asp: 0,98; Ser: 0,80; Glu: 1,00; Ile: 1,05; Phe: 2,10; $NH_3$: 1,76.

Die Erfindung betrifft auch die Verwendung der Verbindungen der Formel I als Heilmittel und pharmazeutische Präparate, die diese Verbindungen enthalten. Bevorzugt ist die Anwendung bei Primaten, insbesondere beim Menschen.

Pharmazeutische Präparate enthalten eine wirksame Menge des Wirkstoffs der Formel I zusammen mit einem anorganischem oder organischen pharmazeutisch verwendbaren Trägerstoff. Die Anwendung kann intranasal, intravenös, subkutan oder peroral erfolgen. Die Dosierung des Wirkstoffs hängt von der Warmblüter-Spezies, dem Körpergewicht, Alter und von der Applikationsart ab.

Die pharmazeutischen Präparate der vorliegenden Erfindung werden in an sich bekannten Lösungs-, Misch-, Granuliert- oder Dragierverfahren hergestellt.

Für eine orale Anwendungsform werden die aktiven Verbindungen mit den dafür üblichen Zusatzstoffen wie Trägerstoffen, Stabilisatoren oder inerten Verdünnungsmitteln vermischt und durch übliche Methoden in geeignete Darreichungsformen gebracht, wie Tabletten, Dragees, Steckkapseln, wäßrige, alkoholische oder ölige Suspensionen oder wäßrige, alkoholische oder ölige Lösungen. Als inerte Träger können z.B. Gummi arabicum, Magnesia, Magnesiumcarbonat, Kaliumphosphat, Milchzucker, Glucose, Magnesiumstearylfumarat oder Stärke, insbesondere Maisstärke verwendet werden. Dabei kann die Zubereitung sowohl als Trocken- oder Feuchtgranulat erfolgen. Als ölige Trägerstoffe oder Lösungsmittel kommen beispielsweise pflanzliche oder tierische Öle in Betracht, wie Sonnenblumenöl und Lebertran.

Zur subkutanen oder intravenösen Applikation werden die aktiven Verbindungen oder deren physiologisch verträgliche Salze, gewünschtenfalls mit den dafür üblichen Substanzen wie Lösungsvermittler, Emulgatoren oder weitere Hilfsstoffe in Lösung, Suspensionen oder Emulsionen gebracht. Als Lösungsmittel kommen z.B. in Frage: Wasser, physiologische Kochsalzlösungen oder Alkohole, z.B. Ethanol, Propandiol oder Glycerin, daneben auch Zuckerlösungen wie Glucose- oder Mannitlösungen, oder auch eine Mischung aus den verschiedenen genannten Lösungsmitteln.

Ebenfalls möglich ist der Einsatz von injizierbaren Retardzubereitungen. Als Arzneiformen können z.B. ölige Kristallsuspensionen, Mikrokapseln, Rods oder Implantate verwendet werden, wobei die letzteren aus gewebeverträglichen Polymeren, insbesondere bioabbaubaren Polymeren, wie z.B. auf der Basis von Polymilchsäure-Polyglykolsäure-Copolymeren oder Humanalbumin sein können.

Verzeichnis der verwendeten Abkürzungen:

Chg Cyclohexylglycyl
Boc tert.-Butyloxycarbonyl
d Dublett
DC Dünnschichtchromatographie
DCC Dicyclohexylcarbodiimid
DCM Dichlormethan

DMF Dimethylformamid
DMAP 4-Dimethylaminopyridin
DMSO Dimethylsulfoxid
EDAC 1-(3-Dimethylamino-propyl)-3-ethyl-carbodiimid Hydrochlorid
EE Essigsäureethylester
FAB Fast atom bombardment
HOBt Hydroxybenzotriazol
i. Vac. im Vakuum
m Multiplett
M Molekularpeak
NEM N-Ethylmorpholin
Npg Neopentylglycyl
MS Massenspetrum
PPA n-Propylphosphonsäureanhydrid
RT Raumtemperatur
s Singulett
Schmp. Schmelzpunkt
t Triplett
Tbg tert.-Butylglycyl
TBTU 2-(1H-Benzotriazol-1-yl)-1,1,3,3-tetramethyluronium tetrafluoroborat
THF Tetrahydrofuran
Thia 2-Thienylalanyl
Z Benzyloxycarbonyl

Die sonstigen für Aminosäuren verwendeten Abkürzungen entsprechen dem in der Peptidchemie üblichen Drei-Buchstaben-Code (wie er z.B. in Eur. J. Biochem. 138, (1984), 9-37 beschrieben ist). Falls nicht ausdrücklich anders angegeben, handelt es sich immer um eine Aminosäure der L-Konfiguration.

Die nachstehenden Beispiele dienen zur Erläuterung der vorliegenden Erfindung, ohne daß diese darauf beschränkt wäre.

**Beispiel 1**

N,N′-Bis-(tert.-butoxycarbonyl-L-phenylalanyl-L-valyl)-2S,5S-diamino-1,6-diphenyl-hexan-3R,4R-diol

100 mg N,N′-Bis-(L-valyl)-2S,5S-diamino-1,6-diphenyl-hexan-3R,4R-diol-dihydrochlorid wurden zusammen mit 111 mg N-tert.-Butoxycarbonyl-L-phenylalanin, 0,57 ml NEM und 60 mg HOBt in 1,5 ml DMF gelöst. Nach Zugabe von 85 mg EDAC bei 0°C rührte man weiter 1 h bei 0°C, dann über Nacht bei RT. Das Lösungsmittel wurde i. Vac. abrotiert, der Rückstand in EE aufgenommen und mit gesättigter $KHCO_3$-, 10 %iger $KHSO_4$-Lösung und Wasser extrahiert. Die organische Phase wurde mit wasserfreiem $Na_2SO_4$ getrocknet und eingeengt. Der Rückstand wurde aus Ethanol-Wasser umkristallisiert.
Die Ausbeute betrug 92 mg.

MS (FAB): 993 $(M+H)^+$, 975, 893, 793

NMR (270 MHz, DMSO <$D_6$>): 0,72 (d, 6Hz, 6H); 0,75 (d, 6Hz, 6H); 1,29 (s, 18H); 1,86 (m, 2H); 2,60-2,96 (m, 8H); 3,30 (m, 2H); 4,17 (m, 2H); 4,45 (m, 2H); 4,68 (m, 2H); 7,03 (d, 9Hz, 2H); 7,05-7,30 (m, 22H); 7,53 (d, 9Hz, 2H).

**Beispiel 2**

N,N′-Bis-(L-valyl)-2S,5S-diamino-1,6-diphenyl-hexan-3R,4R-diol-dihydrochlorid

220 mg N,N′-Bis-(tert.-butoxycarbonyl-L-valyl)-2S,5S-diamino-1,6-diphenyl-3,4-O-isopropyliden-hexan-3R,4R-diol wurden in 10 ml einer ca. 3N Lösung von HCl in Dioxan/Methanol 1/1 für 1 h bei RT gerührt. Man entfernte die flüchtigen Bestandteile der Lösung i. Vac. und trocknete im Hochvakuum. Die Substanz wurde ohne weitere Reinigung in die nächste Stufe eingesetzt.
Ausbeute: 184 mg

MS (FAB): 499 $(M+H)^+$, 481, 463

**Beispiel 2a**

N,N-Bis-(tert.-butoxycarbonyl-L-valyl)-2S,5S-diamino-1,6-diphenyl-3,4-O-isopropyliden-hexan-3R,4R-diol

136 mg 2S,5S-Diamino-1,6-diphenyl-3,4-O-isopropyliden-hexan-3R,4R-diol wurden mit 0,54 ml NEM

und 260 mg N-tert.-Butoxycarbonyl-L-valin in 2 ml trockenen EE gelöst. Bei -10 °C wurde 0,97 ml einer 50 %igen PPA-Lösung in EE zugesetzt. Man rührte 1 h bei 0 °C, dann über Nacht bei RT. Die Lösung wurde mit EE verdünnt, mit gesättigter $NaHCO_3$-, 10 %iger $KHSO_4$-Lösung und Wasser extrahiert. Man trocknete die organische Phase über wasserfreiem $MgSO_4$, engte ein und reinigte den Rückstand durch Chromatographie an Kieselgel (Dichlormethan/Methanol 97/3).

Man erhielt als Ausbeute: 230 mg

MS (FAB): 739 $(M+H)^+$, 681, 639, 569, 539

**Beispiel 2b**

2S,5S-Diamino-1,6-diphenyl-3,4-O-isopropyliden-hexan-3R,4R-diol

2,3 g 2S,5S-Diazido-1,6-diphenyl-1,6-O-isopropyliden-hexan-3R,4R-diol wurden in 50 ml Methanol gelöst und mit ca. 0,2 g Palladium auf Kohle (10 %ig) für 2 h unter Normaldruck hydriert. Der Katalysator wurde abfiltriert, nach Einengung der Lösung wurde der Rückstand an Kieselgel chromatographiert (Dichlormethan/Methanol 99/1).

Ausbeute: 1,33 g

MS (FAB): 341 $(M+H)^+$

NMR (270 MHz; DMSO <$D_6$>): 1,29 (m, 4H); 1,37 (s, 6H); 2,71 (dd, 12Hz, 5Hz, 2H); 2,87 (m, 2H); 3,32 (m, 2H); 3,95 (s, 2H); 7,12-7,33 (m, 10H)

**Beispiel 2c**

2S,5S-Diazido-1,6-diphenyl-3,4-O-isopropyliden-hexan-3R,4R-diol

8,5 g 2R,5R-Di-(4-nitrophenylsulfonyloxy)-1,6-diphenyl-3,4-O-isopropyliden-hexan-3S,4S-diol wurden in 300 ml DMF gelöst und mit ca. 9,2 g $NaN_3$ und 6,3 g 18-Krone-6 für 4 h auf 50 °C erhitzt. Das Lösungsmittel wurde überwiegend i. Vac. abrotiert, der Rückstand in Ether aufgenommen und mit wäßriger $NaHCO_3$-Lösung extrahiert. Nach Waschen mit Wasser wurde getrocknet und eingeengt. Der Rückstand wurde an Kieselgel (Toluol/n-Heptan 2/5 bis 2/3) chromatographiert.

Man erhielt als Ausbeute: 2,37 g

NMR (270 MHz, DMSO <$D_6$>): 1,48 (s, 6H); 2,92-3,12 (m, 4H); 3,74 (dd, 10Hz, 5Hz, 2H); 4,15 (s, 2H); 7,21-7,39 (m, 10H)

**Beispiel 2d**

2R,5R-Di-(4-nitrophenylsulfonyloxy)-1,6-diphenyl-3,4-O-isopropyliden-hexan-3S,4S-diol

5,6 g 2R,5R-Dihydroxy-1,6-diphenyl-3,4-O-isopropyliden-hexan-3R,4R-diol wurden zusammen mit 7,9 g DMAP in 300 ml Chloroform gelöst. Man gab bei RT 14,5 g p-Nitrobenzolsulfonylchlorid hinzu und rührte 3 h bei 50 °C. Man versetzte mit Methylenchlorid und extrahierte die Lösung mit Bicarbonat-, $KHSO_4$- und NaCl-Lösung. Nach Trocknung der organischen Phase wurde eingeengt.

Ausbeute: 11,8 g

MS (FAB): 713 $(M+H)^+$, 697, 510

NMR (270 MHz, DMSO <$D_6$>): 1,42 (s, 6H); 2,87 (dd, 15Hz, 9Hz, 2H); 3,11 (dd, 15Hz, 3Hz, 2H); 4,41 (s, 2H); 5,07 (dm, 9Hz, 2H); 6,95-7,11 (m, 10H); 7,73 (d, 9Hz, 4H); 8,18 (d, 9Hz, 4H)

**Beispiel 2e**

2R,5R-Dihydroxy-1,6-diphenyl-3,4-O-isopropyliden-3R,4R-diol

Unter Argon wurden 1,12 g 1,2R-5R,6-Diepoxy-3,4-O-isopropyliden-3R-4R-diol (Y. Le Merrer, A. Dureault, C. Gravier, D. Languin und J.C. Depezay Tetrahedron Lett., 26 (1985), 319-322) bei -78 °C zu einer Lösung von 36 mmol $(C_6H_5)_2CuLi$ in 60 ml trockenem Ether gegeben. Man entfernte das Kältebad und ließ unter Rühren auf RT erwärmen. Die Mischung wurde mit 250 ml EE versetzt und 3x mit einer Mischung aus 25 %igem Ammoniak und Ammoniumchlorid extrahiert. Die EE-Phase wurde mit NaCl-Lösung gewaschen, getrocknet und eingeengt. Der Rückstand wurde über Kieselgel (Dichlormethan/EE 97/3 bis 90/10) gereinigt.

Man erhielt als Ausbeute: 1,86 g

MS (FAB): 343 $(M+H)^+$, 327, 285, 267

NMR (270 MHz, DMSO <$D_6$>): 1,39 (s, 6H); 2,58 (dd, 13Hz, 9Hz, 2H), 3,43 (dd, 13Hz, 3Hz, 2H); 3,68 (m, 2H), 3,83 (m, 2H); 5,05 (d, 6Hz, 2H); 7,14-7,32 (m, 10H)

**Beispiele 3-5**

3) N,N′-Bis-(tert.-butoxycarbonyl)-2S,5S-diamino-1,6-diphenyl-hexan-3R,4R-diol

4) N,N′-Bis-(tert.-butoxycarbonyl)-2S,5S-diamino-1,6-diphenyl-hexan-3S,4S-diol

5) N,N′-Bis-(tert.-butoxycarbonyl)-2S,5S-diamino-1,6-diphenyl-hexan-3R,4S-diol

17 g tert.-Butoxycarbonyl-L-phenylalaninal wurden in 500 ml trockenem THF gelöst und unter Argon auf 0 °C gekühlt. Man gab über ca. 20 min 1 l 0,1 molare $SmI_2$-Lösung in THF hinzu und rührte für 30 min bei RT nach. Mit 0,1 N wäßriger HCl wurde auf pH 1-2 angesäuert. Man verdünnte mit EE, trennte die organische Phase ab und extrahierte diese mit 0,1 N HCl, 2x mit $Na_2S_2O_3$-Lösung und 2x mit Wasser. Nach Trocknung über $MgSO_4$ wurde eingeengt und über Kieselgel chromatographiert (EE/Petrolether 1/2).

Die Fraktion, die das 3R,4R-Isomer enthielt, wurde aus Ethanol/Wasser umkristallisiert.

Aus der Fraktion, die das 3S,4S- und das 3R,4S-Isomer enthielt, konnte das 3S,4S-Isomer durch Kristallisation aus Dichlormethan/Isopropylether/Heptan gewonnen werden. Die Mutterlauge wurde zur Erlangung des 3R,4S-Isomeren an RP18-Kieselgel chromatographiert (Acetonitril/Wasser 4/6).

Ausbeuten: 1,61 g 3R,4R-Isomer
1,00 g 3S,4S-Isomer
0,71 g 3R,4S-Isomer

Rf-Werte: Kieselgel, EE/Hexan 1/2
0,18 3R,4R-Isomer
0,41 3S,4S-Isomer
0,39 3R,4S-Isomer

MS (FAB): 501 $(M+H)^+$, 401, 345, 327, 301 3R,4R-Isomer 501 $(M+H)^+$, 401, 345, 327, 301 3S,4S-Isomer 501 $(M+H)^+$, 401, 345, 327 3R,4S-Isomer

| $^1$H-NMR (270 MHz, DMSO <$D_6$>): | | | |
|---|---|---|---|
| | 3R,4R-Isomer | 3S,4S-Isomer | 3R,4S-Isomer |
| N-H | 6,16; (d; 2H) | 6,60 (d, 2H) | 6,31 (d, 1H)<br>6,28 (d, 1H) |
| O-H | 4,43 (m, 2H) | 4,57 (d, 7Hz,2H) | 4,62 (d,4Hz,1H)<br>4,94 (d,6Hz,1H) |
| $H^3$, $H^4$ | 4,12 (m, 2H) | 3,71 (m, 2H) | 3,91-4,12(m,2H) |
| $H^2$, $H^5$ | 3,24 (m, 2H) | 3,42 (m, 2H) | 3,27-3,46(m,2H) |
| $CH_2$ | 2,54-2,80 (m,2H) | 3,04 (dd, 14Hz, 4Hz, 1H)<br>2,63 (dd, 14Hz, 9Hz, 1H) | 2,62-2,83(m,2H) |
| $C(CH_3)_3$ | 1,30 (s, 18H) | 1,30 (s, 18H) | 1,32 (s, 9H)<br>1,24 (s, 9H) |
| Ar-H | 7,08-7,27 (m,10H) | 7,11-7,29 (m,10H) | 7,08-7,32(m,10H) |

Die Zuordnung der absoluten Stereochemie ergibt sich beim 3R,4S-Isomeren aus dem doppelten Signalsatz, die Unterscheidung zwischen dem 3R,4R- und dem 3S,4S-Isomeren durch Vergleich mit synthetischem Referenzmaterial ausgehend von D-Mannit (s. Beispiel 3.1). Auswertung von Kopplungskonstanten nach Abspaltung der tert.-Butoxycarbonyl-Gruppen und Überführung der Isomere mit Phosgen in doppelte 2-Oxazolidinonsysteme ergab damit konsistene Ergebnisse.

**Beispiel 3.1**

Zuordnung der absoluten Stereochemie der Isomere aus Beispielen 3-5

N,N′-Bis-(tert.-butoxycarbonyl)-2S-5S-diamino-1,6-diphenyl-hexan-3R,4R-diol

140 mg 2S,5S-Diamino-1,6-diphenyl-3,4-O-isopropyliden-hexan-3R,4R-diol wurden in einer Mischung aus 5 ml 1N HCl in Methanol und 5 ml 5N HCl in Dioxan gelöst und 4 h bei RT gerührt. Die flüchtigen Bestandteile wurden i. Vac. entfernt. Man trocknete den Rückstand im Hochvakuum und setzte das erhaltene 2S,5S-Diamino-1,6-diphenyl-hexan-3R,4R-diol-dihydrochlorid (MS (FAB): 301 $(M+H)^+$ der freien Base) direkt in die nächste Reaktion ein.

45 mg 2S,5S-Diamino-1,6-diphenyl-hexan-3R,4R-diol-dihydrochlorid wurden in 5 ml trockenem Dichlormethan gelöst und zusammen mit 40 µl Triethylamin und 75 mg Pyrokohlensäure-di-tert.-butylester 3 h bei

RT gerührt. Man verdünnte mit Dichlormethan und extrahierte mit $KHSO_4$-, $NaHCO_3$- und NaCl-Lösung. Nach Trocknung über wasserfreiem $Na_2SO_4$ wurde eingeengt und der Rückstand über Kieselgel gereinigt (Acetonitril/DCM 1/8).
Ausbeute: 23 mg
MS (FAB) 501 $(M+H)^+$, 401, 345, 327, 301
Die Verbindung war identisch mit dem polarsten Isomer aus den Beispielen 3-5.

**Beispiel 6**

N,N′-Bis-(tert.-butoxycarbonyl-L-phenylalanyl-L-valyl)-2S,5S-diamino-1,6-diphenyl-hexan-3S,4S-diol

38 mg N,N′-Bis-(tert.-butoxycarbonyl-L-valyl)-2S,5S-diamino-1,6-diphenyl-hexan-3S,4S-diol wurden für 30 min mit 5N HCl in Dioxan behandelt. Die flüchtigen Bestandteile wurden i. Vac. entfernt, der Rückstand getrocknet. Das so erhaltene N,N′-Bis-(L-valyl)-2S,5S-diamino-1,6-diphenyl-hexan-3S,4S-diol-dihydrochlorid wurde mit 40 mg tert.-Butyloxycarbonylphenylalanin, 22 mg HOBt und 51 mg TBTU in 1 ml trockenem DMF gelöst. Man setzte 60 $\mu$l Ethyldiisopropylamin hinzu und rührte 15 min bei RT. Das DMF wurde abrotiert, der Rückstand in EE aufgenommen und mit $KHSO_4$-, $NaHCO_3$-Lösung und Wasser extrahiert. Nach Trocknung über $MgSO_4$ konzentrierte man auf, wobei die Substanz auskristallisierte. Man filtrierte den Niederschlag ab, wusch mit Ether und erhielt so eine Ausbeute von: 30 mg
MS (FAB): 1015 $(M+Na)^+$, 993 $(M+H)^+$, 893, 793
NMR (270 MHz, DMSO <$D_6$>): 0,79 (m, 12H); 1,28 (s, 18H); 1,85 (m, 2H); 2,68-2,82 (m, 4H); 2,85-3,03 (m, 4H), 3,37 (m, 2H); 4,00-4,13 (m, 4H); 4,21 (m, 2H); 4,66 (d, 7Hz, 2H); 7,03 (d, 7Hz, 2H); 7,05-7,34 (m, 20H); 7,62 (d, 7Hz, 2H); 7,68 (d, 8Hz, 2H)

**Beispiel 7**

N,N′-Bis-(tert.-butoxycarbonyl-L-phenylalanyl-L-valyl)-2S,5S-diamino-1,6-diphenyl-hexan-3R,4S-diol

Synthese analog zu Beispiel 6 aus N,N′-Bis-(tert.-butoxycarbonyl-L-valyl)-2S,5S-diamino-1,6-diphenyl-hexan-3R,4S-diol
MS (FAB): 1015 $(M+Na)^+$, 993 $(M+H)^+$, 893, 793
NMR (270 MHz, DMSO <$D_6$>): 0,68-0,85 (m, 12H); 1,28 (s, 9H); 1,30 (s, 9H); 1,75-2,03 (m, 2H); ca. 2,5-3,30 (m, 8H); ca. 3,3-3,51 (m, 2H); 4,05-4,30 (m, 5H); 4,43 (m, 1H); 4,74 (d, 4Hz, 1H); 5,32 (d, 7Hz, 1H); 6,93-7,35 (m, 22H); 7,61 (d, 8Hz, 1H); 7,67 (d, 7Hz, 1H); 7,85 (d, 8Hz, 1H); 7,92 (d, 7Hz, 1H)

**Beispiel 8**

N,N′-Bis-(tert.-butoxycarbonyl-L-valyl)-2S,5S-diamino-1,6-diphenyl-hexan-3S,4S-diol

164 mg N,N′-Bis-(tert.-butoxycarbonyl)-2S,5S-diamino-1,6-diphenyl-hexan-3S,4S-diol wurden 1,5 h bei RT mit 10 ml 5N HCl in Dioxan behandelt. Die flüchtigen Bestandteile entfernte man i. Vac., den Rückstand trocknete man. Das so erhaltene 2S,5S-Diamino-1,6-diphenyl-hexan-3S,4S-diol-dihydrochlorid wurde zusammen mit 178 mg tert.-Butoxycarbonyl-L-valin und 0,56 ml NEM in 15 ml trockenem DMF gelöst. Bei -5°C gab man 0,53 ml einer 50 %igen Lösung von PPA in EE hinzu, rührte 1 h bei 0°C und über Nacht bei RT. Das Lösungsmittel wurde abrotiert, der Rückstand in EE aufgenommen und mit Wasser, $NaHCO_3$-, $KHSO_4$-Lösung und Wasser extrahiert. Nach Trocknung über wasserfreiem $Na_2SO_4$, engte man i. Vac. ein. Beim Behandeln des Rückstandes mit Diethylether kristallisierte das Produkt aus. Es wurde aus Ethanol/Wasser umkristallisiert.
Ausbeute: 59 mg
MS (FAB): 699 $(M+H)^+$, 599, 499

**Beispiel 9**

N,N′-Bis-(tert.-butoxycarbonyl-L-valyl)-2S,5S-diamino-1,6-diphenyl-hexan-3R,4S-diol

Synthese analog zu Beispiel 8 aus N,N′-Bis-(tert.-butoxycarbonyl)-2S,5S-diamino-1,6-diphenyl-hexan-3R,4S-diol
MS (FAB): 699 $(M+H)^+$, 599, 499

**Beispiel 10**

N,N′-Bis-(L-lysyl-L-valyl)-2S,5S-diamino-1,6-diphenyl-hexan-3R,4R-diol-tetrahydrochlorid

Synthese analog Beispiel 2 aus Beispiel 11
MS (FAB, LiI): 761 $(M+Li)^+$, 755 $(M+H)^+$, 737

**Beispiel 11**
N,N′-Bis-(Nα-<tert.-butoxycarbonyl>-L-lysyl-L-valyl)-2S,5S-diamino-1,6-diphenyl-hexan-3R,4R-diol-dihydrochlorid

36 mg N,N′-Bis-(<Nω-Benzyloxycarbonyl-Nα-tert.-butoxycarbonyl>L-lysyl-L-valyl)-2S,5S-diamino-1,6-diphenyl-hexan-3R,4R-diol (Synthese analog Beispiel 1 aus Nω-Benzyloxycarbonyl-Nα-tert.-butoxycarbonyl-L-lysin und N,N′-Bis-(L-Valyl)-2S,5S-diamino-1,6-diphenyl-hexan-3R,4R-diol-dihydrochlorid) wurden mit Palladium auf Aktivkohle als Katalysator in Methanol hydriert. Der pH-Wert wurde dabei mit einer Lösung von HCl in Methanol auf etwa 3-4 gehalten. Nach Abfiltrieren des Katalysators und Einengung erhielt man 26 mg Produkt.

MS (FAB, LiI): 961 $(M+Li)^+$

NMR (270 MHz, DMSO <$D_6$>): 0,75 (d, 5Hz, 6H); 0,78 (d, 5Hz, 6H); ca. 1,13-1,60 (m, ca. 12H); 1,38 (s, 18H); 1,88 (m, 2H); ca. 2,50-2,68 (m, 2H); 2,72-2,94 (m, 6H); 3,72 (m, 2H); 4,22 (m, 2H); 4,37 (m, 2H); 4,41-4,55 (m, 4H); 4,72 (m, 2H); 6,76 (m, 2H); 7,05-7,23 (m, 16H); 7,66 (d, 8Hz, 2H); 8,15 (d, 9Hz, 2H)

**Beispiel 12**
N,N′-Bis-(Nα-<tert.-butoxycarbonyl-L-phenylalanyl>-L-lysyl)-2S,5S-diamino-1,6-diphenyl-hexan-3R,4R-diol-dihydrochlorid

Synthese analog zu Beispiel 11.
MS (FAB): 1051 $(M+H)^+$, 951

**Beispiel 13**
N,N′-Bis-<(2S-(1,1-dimethylethyl-sulfonylmethyl)-3-(1-naphthyl)-propionyl)-L-valyl>-2S,5S-diamino-1,6-diphenyl-hexan-3R,4R-diol

57 mg N,N′-Bis-(L-valyl)-2S,5S-diamino-1,6-diphenyl-hexan-3R,4R-diol-dihydrochlorid, 95 mg (2S-(1,1-Dimethylethylsulfonylmethyl)-3-(1-naphthyl)-propionsäure (J. Med. Chem. 31, 1839, (1988)), 41 mg HOBt und 96 mg TBTU wurden in 1 ml trockenem DMF gelöst. Bei RT gab man 0,11 ml N-Ethyldiisopropylamin hinzu und rührte für 1 h. Das Lösungsmittel wurde abrotiert, der Rückstand mit 30 ml EE aufgenommen und mit Bisulfat-, Bicarbonatlösung und Wasser extrahiert. Nach Trocknung über $Na_2SO_4$ engte man ein und reinigte die Substanz durch Chromatographie an Kieselgel (DCM/Methanol 97/3).
Man erhielt als Ausbeute: 31 mg

MS (FAB): 1153 $(M+Na)^+$, 1131 $(M+H)^+$, 716

NMR (270 MHz, DMSO <$D_6$>): 0,69 (d, 7Hz, 6H); 0,76 (d, 7Hz, 6H); 1,10 (s, 18H); 1,86 (m, 2H); 2,63-2,87 (m, 6H); 3,08 (m, 2H); ca. 3,25-3,44 (m, ca. 2H); 3,52-3,63 (m, 2H); 4,08 (m, 2H); 7,32 (d, 8Hz, 2H); 7,38-7,48 (m, 4H); 7,47-7,62 (m, 4H); 7,81 (m, 2H); 7,92 (m, 2H); 8,12-8,25 (m, 4H)

**Beispiel 14**
N,N′-Bis-(L-seryl-L-phenylalanyl-L-valyl)-2S,5S-diamino-1,6-diphenyl-hexan-3R,4R-diol-dihydrochlorid

Synthese analog zu Beispiel 16
MS (FAB, LiI): 973 $(M+Li)^+$, 967 $(M+H)^+$

**Beispiel 15**
N,N′-Bis-(tert.-butoxycarbonyl-L-(O-tert.-butyl-seryl)-L-phenylalanyl-L-valyl)-2S,5S-diamino-1,6-diphenyl-hexan-3R,4R-diol

52 mg N,N′-Bis-(L-phenylalanyl-L-valyl)-2S,5S-diamino-1,6-diphenyl-hexan-3R,4R-diol-dihydrochlorid wurden zusammen mit 18 mg HOBt, 15,3 µl NEM und 35 mg O-tert.-Butyl-N-tert.-butoxycarbonyl-L-serin in 1 ml trockenem DMF gelöst und bei 0° C mit 25,3 mg EDAC versetzt. Man rührte 1h bei 0° C, über Nacht bei RT. Das Lösungsmittel wurde abrotiert, der Rückstand in EE aufgenommen und mit Bisulfat-, Bicarbonatlösung und Wasser extrahiert. Die organische Phase wurde über wasserfreiem Natriumsulfat getrocknet und eingeengt. Der Rückstand wurde durch Chromatographie an Kieselgel gereinigt.
Man erhielt als Ausbeute: 28 mg

MS (FAB): 1301 $(M+Na)^+$, 1279 $(M+H)^+$, 1261, 1179, 1079,

NMR (270 MHz, DMSO<$D_6$>): 0,78 (d, 7Hz; 6H), 0,81 (d, 7Hz; 6H), 1,06 (s; 18H), 1,38 (s, 18H), 1,82 (m; 2H), 2.61-2,98 (m, 8H), ca. 3,15-3,45 (m, ca. 6H), 3,92 (m; 2H), 4,11 (dd, 8Hz, 6Hz; 2H), 4,47 (m; 2H), 4,63 (m; 4H), 6,58 (d, 8Hz; 2H), 7,04-7,25 (m; 20H), 7,46 (d, 9Hz; 2H), 7,77 (d, 8Hz; 2H), 7,83 (d, 8Hz; 2H).

**Beispiel 16**

N,N′-Bis-(L-phenylalanyl-L-valyl)-2S,5S-diamino-1,6-diphenyl-hexan-3R,4R-diol-dihydrochlorid

100 mg N,N′-Bis-(tert.-butyloxycarbonyl-L-phenylalanyl-L-valyl)-2S,5S-diamino-1,6-diphenyl-hexan-3R,4R-diol (Beispiel 1) wurden mit einer Mischung aus 2 ml 5N HCl in Dioxan und 1 ml HCl in Methanol für 30 min bei RT behandelt. Man entfernte die flüchtigen Bestandteile i.Vac., wusch den Rückstand mit Ether und trocknete die Substanz im Hochvakuum.

Ausbeute: 59 mg

MS (FAB): 793 $(M+H)^+$, 775,

**Beispiel 17**

N,N′-Bis-(L-phenylalanyl-L-valyl)-2S,5S-diamino-1,6-diphenyl-hexan-3S,4S-diol-dihydrochlorid

Synthese analog zu Beispiel 16

MS (FAB): 793 $(M+H)^+$, 775

**Beispiel 18**

N,N′-Bis-(L-phenylalanyl-L-valyl)-2S,5S-diamino-1,6-diphenyl-hexan-3R,4S-diol-dihydrochlorid

Synthese analog zu Beispiel 16

MS (FAB): 793 $(M+H)^+$, 775

**Beispiel 19**

N,N′-Bis-(L-seryl-L-phenylalanyl-L-valyl)-2S,5S-diamino-1,6-diphenyl-hexan-3S,4S-diol-dihydrochlorid

Synthese analog zu Beispiel 14

MS (FAB): 967 $(M+H)^+$

**Beispiel 20**

N,N′-Bis-(L-seryl-L-phenylalanyl-L-valyl-2S,5S-diamino-1,6-diphenyl-hexan-3R,4S-diol-dihydrochlorid

Synthese analog zu Beispiel 14

MS (FAB): 967 $(M+H)^+$

**Beispiel 21**

Bis-(N-(L-phenylalanyl-L-valyl)-2S-amino-3-phenylpropyl)-amin-tri-hydrochlorid

Synthese analog zu Beispiel 16 aus Beispiel 22

MS (FAB): 776 $(M+H)^+$

**Beispiel 22**

Bis-(N-(tert.-butoxycarbonyl-L-phenylalanyl-L-valyl)-2S-amino-3-phenylpropyl)-amin

Synthese analog Beispiel 6 aus Beispiel 23

MS (FAB, LiI): 982 $(M+Li)^+$, 976 $(M+H)^+$

NMR (270 MHz, DMSO <$D_6$>): 0,81 (m, 12H); 1,29 (s, 18H); 1,89 (m, 2H); ca. 2,45-2,98 (m, ca. 12H); 3,97 (m, 2H); 4,05-4,25 (m, 4H); 7,03 (d, 9Hz, 2H); 7,10-7,31 (m, 20H); 7,65 (d, 8Hz, 2H); 7,84 (d, 8Hz, 2H)

**Beispiel 23**

Bis-(N-(L-valyl)-2S-amino-3-phenylpropyl)-amin-tri-hydrochlorid

Synthese analog zu Beispiel 16 aus Beispiel 24

MS (FAB): 482 $(M+H)^+$

**Beispiel 24**
Bis-(N-(tert.-butoxycarbonyl-L-valyl)-2S-amino-3-phenylpropyl)-amin

Synthese analog Beispiel 16 aus Beispiel 25

MS (FAB): 682 $(M+H)^+$

NMR (270 MHz, DMSO <$D_6$>): 0,73 (d, 6Hz, 6H); 0,77 (d, 6Hz, 6H); 1,38 (s, 18H); 1,65 (m, 1H); 1,82 (m, 2H); 2,42-ca. 2,53 (m, ca. 4H); 2,64 (dd, 14Hz, 8Hz, 2H); 2.84 (dd, 14Hz, 6,Hz, 2H); 3,68 (m, 2H); 3,93 (m, 2H); 6,50 (d, 9Hz, 2H); 7,12-7,28 (m, 10H); 7,62 (d, 8Hz, 2H)

**Beispiel 25**
Bis-(N-tert.-butoxycarbonyl-2S-amino-3-phenylpropyl)-amin-hydrochlorid

9,6 g tert.-Butoxycarbonyl-L-phenylalaninal wurden zusammen mit 30,5 g $NH_4OAc$ und 1,7 g $NaBH_3CN$ in 300 ml Methanol gelöst und 6h bei RT gerührt. Mit konzentrierter HCl wurde auf pH < 2 angesäuert. Dabei fällt das Produkt aus. Man digerierte mit Diethylether und Wasser, trocknete im Hochvakuum und erhielt als Ausbeute 3,1 g.

MS (FAB): 484 $(M+H)^+$, 428, 372,

NMR (270 MHz; DMSO<$D_6$): 1,33 (s, 18H), 2,55-2,90 (m; 8H), 3,82 (m; 2H), 6,75 (m; 2H), 7,12-7,325 (m; 10H).

**Beispiel 26**
N,N′-Bis-(5S-amino-4S-hydroxy-7-methyl-octanoyl)-2S,5S-diamino-1,6-diphenyl-hexan-3S,4S-diol-dihydrochlorid

Synthese analog Beispiel 16

MS (FAB): 643 $(M+H)^+$, 625,

NMR (270 MHz; DMSO<$D_6$>): 0,92 (m; 12H), 1,43 (m; 4H), 1,60 (m; 4H), 1,74 (m; 2H), 2,15 (m, 2H), 2,26 (m; 2H), 2,72 (dd, 14Hz, 11Hz; 2H), 2,93 (m; 2H), 3,12 (dm; 2H), 3,44 (m; 4H), 4,03 (m; 2H), ca. 4,85 (m; ca. 4H), 7,13-7,38 (m; 20H), 7,82 (m; 6H), 8,13 (d, 9Hz; 2H).

**Beispiel 26a**
N,N′-Bis-(N-tert.-butoxycarbonyl-5S-amino-7-methyl-4S-(tert.-butyl-dimethyl-silyl)oxy-octanoyl)-2S,5S-diamino-1,6-diphenyl-hexan-3S.4S-diol

88,5 mg N,N′-Bis-(tert.-butyloxycarbonyl-2S,5S-diamino-1,6-diphenyl-hexan-3S,4S-diol wurden für 30 min bei RT mit 2 ml 5N HCl in Dioxan behandelt. Man entfernte die flüchtigen Bestandteile i. Vac. und trocknete den Rückstand im Hochvakuum. Das erhaltene 2S,5S-Diamino-1,6-diphenyl-hexan-3S,5S-diol-dihydrochlorid wurde mit 211 mg N-tert.-Butyloxycarbonyl-5S-amino-7-methyl-4S-(tert.-butyl-dimethyl-silyl)-oxy-octansäure (Synthese aus (5S)-5-<(1S)-1-(N-Boc-amino)-3-methylbutyl>dihydrofuran-2(3H)-on (A.H. Fray et al; J. Org. Chem. 51 (1986), 4828-4833) analog der Darstellung von 5-(t-Boc-amino)-4-<tert.-butyldimethylsilyl)-oxy>-6-(phenylmethyl)-hexanoic Acid (B.E. Evans et al., J. Org. Chem. 50, (1985), 4615-4625)), 72 mg HOBt und 28.5 µl NEM in 5 ml trockenem DMF gelöst. Bei 0°C wurden 101 mg EDAC zugesetzt. Die Lösung wurde 1h bei 0°C gerührt, dann über Nacht bei RT. Das Lösungsmittel wurde abrotiert, der Rückstand in EE aufgenommen und mit $KHSO_4$-, $NaHCO_3$- und NaCl-Lösung extrahiert. Nach Trocknung der organischen Phase wurde aufkonzentriert und der Rückstand durch Chromatographie an Kieselgel gereinigt (DCM Acetonitril 5:1).

Ausbeute: 129 mg

MS (FAB): 1093 $(M+H)^+$, 1071 $(M+H)^+$, 971, 871,

NMR (270 MHz, DMSO<$D_6$>): 0,02 (s; 6H), 0,08 (s; 6H), 0,77-0,93 (m; 30H), ca. 1,1-1,4 (m; ca. 6H), 1,45-1,63 (m; 4H), 1,91 (m; 2H), 2,02-2,16 (m; 2H), 2,67 (dd, 11Hz, 14Hz; 2H), 3,36 (m; 2H), 3,42-3,56 (m; 4H), 3,95 (m; 2H), 4,81 (d, 6Hz; 2H), 6,44 (d, 8 Hz; 2H), 7,08-7,30 (m; 10H), 7,79 (d, 9Hz; 1H).

**Beispiel 27**
N,N′-Bis-(tert.-butoxycarbonyl-L-phenylalanyl-L-valyl)-3S,6S-diamino-1,8-di-(4-pyridyl)-octan-4R,5R-diol

Synthese analog zu Beispiel 6 aus 3S,6S-Diamino-1,8-di-(4-pyridyl)-octan-4R,5R-diol-tetrahydrochlorid

NMR (270 MHz, DMSO<$D_6$>): 0,85 (d, 6Hz, 12H); 1,20 (s, 18 H); 1,66 (m, 2H); 1,78 (m, 2H); 2,00 (m, 2H); ca. 2,48 (m, 4H); 2,74 (m, 2H); 2,98 (m, 2H); ca. 3,31 (m, 2H); 4,08 (m, 2H); 4,19 (m, 2H); 4,30 (m, 2H);

4,68 (m, 2H); 7,01 (d, 8Hz, 2H); 7,10-7,30 (m, 14 H); 7,62 (d, 8Hz, 2H); 7,74 (d, 8Hz, 2H); 8,43 (d, 4,8 Hz, 4H)

MS (FAB): 1023 $(M+H)^+$, 923, 823

**Beispiel 27a**

3S,6S-Diamino-1,8-di-(4-pyridyl)-octan-4R,5R-diol-tetrahydrochlorid

Synthese analog Beispiel 2, 2b, 2c und 2e ausgehend von 1,2R-5R,6-Diepoxy-3,4-O-isopropyliden-3R,4R-diol und 4-Picolyllithium

NMR (270 MHz, DMSO<$D_6$>: 1,87-2,20 (m, 4H); 3,10 (m, 4H); 3,29 (m, 2H); 3,84 (d, 6Hz, 2H); ca. 3,3-4,5 (br, ca. 4H); 8,07 (d, 7Hz, 4H); 8,18 (m, 6H); 8,88 (d, 7Hz, 4H)

MS (FAB): 331 $(M+H)^+$

**Beispiel 28**

N,N′-Bis-(2S-<2S-amino-3-phenyl-propyl>-amino-3-methyl-butanoyl)-2S,5S-diamino-1,6-diphenyl-hexan-3S,4S-diol-tetrahydrochlorid

Synthese analog Beispiel 16

MS (FAB): 765 $(M+H)^+$

**Beispiel 29**

N,N′-Bis-(2S-<2S-tert.-butoxycarbonylamino-3-phenyl-propyl>-amino-3-methyl-butanoyl)-2S,5S-diamino-1,6-diphenyl-hexan-3S,4S-diol

Von 50 mg N,N′-Bis-(tert.-butoxycarbonyl)-2S,5S-diamino-1,6-diphenyl-hexan-3S,5S-diol wurden analog Beispiel 8 die Schutzgruppen entfernt. Das resultierende 2S,5S-Diamino-1,6-diphenyl-hexan-3S,5S-diol-dihydrochlorid wurde mit 70 mg 2S-(2S-tert.-Butoxycarbonylamino-3-phenyl-propyl)-amino-3-methyl-butansäure (Synthese durch reduktive Kupplung von tert.-Butoxycarbonyl-L-phenylalaninal und L-Valinmethylester-hydrochlorid mit $NaBH_3CN$ <R.F. Borch et al., J. Am. Chem. Soc., 93 (1971), 2897-2904> gefolgt von üblicher Methylester-Spaltung), 41 mg HOBt und 12,6 µg NEM in 5 ml trockenem DMF gelöst. Bei 0° C, wurden 57 mg EDAC zugesetzt. Man rührte 1 h bei 0° C, über Nacht bei RT. Das DMF wurde i. Vac. entfernt, der Rückstand in DCM aufgenommen und mit $KHSO_4$-, $NaHCO_3$- und NaCl-Lösung gewaschen. Nach Trocknung und Einengung verrieb man den Rückstand mit Diethylether.

Ausbeute: 33 mg

MS (FAB): 965 $(M+H)^+$, 865, 765

NMR (270 MHz; DMSO <$D_6$>): 0,74 (d, 7Hz, 6H); 0,78 (d, 6Hz, 6H); 1,33 (s, 18H); 1,63 (m, 2H); 1,94-2,16 (m, 4H); ca. 2,5 (m, ca. 4H); 2,64 (m, 2H); 2,81 (dd, 14Hz, 5Hz, 2H); 3,13 (dm, 14Hz, 2H); 3,42 (m, 2H); 3,56 (m, 2H); 4,10 (m; 2H); 4,90 (m; 2H); 6,58 (d, 9Hz, 2H); 7,05-7,30 (m, 20H); 7,85 (d, 8Hz, 2H)

**Beispiel 30**

N,N′-Bis-(L-phenylalanyl-L-valyl)-2R,5R-diamino-1,6-diphenyl-hexan-3R,4R-diol-dihydrochlorid

Synthese analog Beispiel 16

MS (FAB): 793 $(M+H)^+$

**Beispiel 31**

N,N′-Bis-(L-phenylalanyl-L-valyl)-2R,5R-diamino-1,6-diphenyl-hexan-3S,4S-diol-dihydrochlorid

Synthese analog Beispiel 16

MS (FAB): 793 $(M+H)^+$

**Beispiel 32**

N,N′-Bis-(L-phenylalanyl-L-valyl)-2R,5R-diamino-1,6-diphenyl-hexan-3R,4S-diol-dihydrochlorid

Synthese analog Beispiel 16

MS (FAB): 793 $(M+H)^+$

**Beispiel 33**

N,N'-Bis-(tert.-butoxycarbonyl-L-phenylalanyl-L-valyl)-2R,5R-diamino-1,6-diphenyl-hexan-3R,4R-diol

Synthese analog Beispiel 6
MS (FAB): 993 $(M+H)^+$, 893, 793
NMR (270 MHz, DMSO <D$_6$>): 0,48 (d, 7Hz, 6H); 0,54 (d, 6Hz, 6H); 1,25 (s, 18H); 1,70 (m, 2H); 2,60 (t, 13Hz, 2H); 2,74 (dd, 14Hz, 11Hz, 2H); 2,96 (dd, 13Hz, 4Hz, 2H); 3,13 (dm, 14Hz, 2H); 3,39 (m, 2H); 4,02-4,25 (m, 6H); 4,88 (d, 4Hz, 2H); 7,02 (d, 9Hz, 2H); 7,07-7,33 (m, 20H); 7,60 (d, 9Hz, 2H); 8,24 (d, 9Hz, 2H);

**Beispiel 34**
N,N'-Bis-(tert.-butoxycarbonyl-L-phenylalanyl-L-valyl)-2R,5R-diamino-1,6-diphenyl-hexan-3S,4S-diol

Synthese analog Beispiel 6
MS (FAB): 993 $(M+H)^+$, 893, 793

**Beispiel 35**
N,N'-Bis-(tert.-butoxycarbonyl-L-phenylalanyl-L-valyl)-2R,5R-diamino-1,6-diphenyl-hexan-3R,4S-diol

Synthese analog Beispiel 6
MS (FAB): 993 $(M+H)^+$, 893, 793

**Beispiele 36-38**
36) N,N'-Bis-(tert.-butoxycarbonyl)-2R,5R-diamino-1,6-diphenyl-hexan-3R,4R-diol
37) N,N'-Bis-(tert.-butoxycarbonyl)-2R,5R-diamino-1,6-diphenyl-hexan-3S,4S-diol
38) N,N'-Bis-(tert.-butoxycarbonyl)-2R,5R-diamino-1,6-diphenyl-hexan-3R,4S-diol

Synthese analog den Beispielen 3-5 aus tert.-Butoxycarbonyl-D-phenylalaninal. Die MS und NMR-Daten entsprechen denen ihrer Enantiomeren aus den Beispielen 3-5.

**Beispiel 39**
N,N'-Bis-(L-(1-naphthyl)alanyl-L-valyl)-2S,5S-diamino-1,6-diphenyl-hexan-3R,4R-diol-dihydrochlorid

Synthese analog Beispiel 16
MS (FAB, LiI): 899 $(M+Li)^+$, 893 $(M+H)^+$, 875

**Beispiel 40**
N,N'-Bis-(tert.-butoxycarbonyl-L-(1-naphthyl)alanyl-L-valyl)-2S,5S-diamino-1,6-diphenyl-hexan-3R,4R-diol

Synthese analog Beispiel 6
MS (FAB): 1093 $(M+H)^+$, 993
NMR (270 MHz, DMSO <D$_6$>): 0,76 (m, 12H); 1,23 (s, 18H); 1,89 (m, 2H); 2,60-2,87 (m, 4H); 3,12 (dd, 14Hz, 10Hz, 2H); ca. 3,33 (m, 2H); 3,52 (dm, 4Hz, 2H); 4,16-4,35 (m, 4H); 4,44 (m, 2H); 4,70 (s, 2H); 7,00-7,27 (m, 12H); 7,37-7,44 (m, 4H); 7,46-7,68 (m, 8H); 7,79 (m, 2H); 7,92 (d, 8Hz, 2H); 8,13 (d, 8Hz, 2H)

**Beispiel 41**
N,N'-Bis-[(2-(2-Hydroxyethyl-sulfonylmethyl)-3-phenylpropionyl)-L-valyl]-2S,5S-diamino-1,6-diphenyl-hexan-3S,4S-diol

Synthese analog Beispiel 13
MS (FAB): 1007 $(M+H)^+$

**Beispiel 42**
N,N'-Bis-[L-phenylalanyl-L-valyl]-2S,5S-diamino-1,6-dicyclohexyl-hexan-3R,4R-diol-dihydrochlorid

Synthese analog Beispiel 16
MS (FAB): 805 $(M+H)^+$, 787

**Beispiel 43**
N,N'-Bis-[L-phenylalanyl-L-valyl]-2S,5S-diamino-1,6-dicyclohexyl-hexan-3S,4S-diol-dihydrochlorid

Synthese analog Beispiel 16
MS (FAB): 805 (M + H)$^+$, 787

**Beispiel 44**
N,N'-Bis-(tert.-butoxycarbonyl-L-phenylalanyl-L-valyl)-2S,5S-diamino-1,6-dicyclohexyl-hexan-3R,4R-diol

Synthese analog Beispiel 6
MS (FAB): 1005 (M + H)$^+$, 987 905, 805

**Beispiel 45**
N,N'-Bis-(tert.-butoxycarbonyl-L-phenylalanyl-L-valyl)-2S,5S-diamino-1,6-dicyclohexyl-hexan-3S,4S-diol

Synthese analog Beispiel 6
MS (FAB): 1005 (M + H)$^+$, 987, 905, 805
NMR (270 MHz, DMSO <$D_6$>): 0,86 (m, 12H); 0,99-1,67 (m, ca. 24H); 1,28 (s, 18H); 1,74 (m, 2H); 1,98 (m, 2H); 2,75 (dd, 14Hz, 11Hz, 2H); 2,96 (dd, 14Hz, 4Hz, 2H); 3,23 (m, 2H); 3,89 (m, 2H); 4,13-4,25 (m, 2H); 4,42 (d, 5Hz, 2H); 7,02 (d, 8Hz, 2H); 7,13-7,32 (m, 10H); 7,69-7,81 (m, 4H)

**Beispiel 46**
N,N'-Bis-(tert.-butoxycarbonyl)-2S,5S-diamino-1,6-dicyclohexyl-hexan-3S,4S-diol

200 mg N,N'-Bis-(tert.-butoxycarbonyl)-2S,5S-diamino-1,6-diphenyl-hexan-3S,4S-diol wurden in 25 ml Eisessig gelöst und mit 100 mg Platindioxid als Katalysator für 18 h bei 60 °C und 120 bar hydriert. Nach Abfiltrieren des Katalysators wurde das Lösungsmittel i. Vac. entfernt und der Rückstand aus Ethanol/Wasser umkristallisiert.
Ausbeute: 150 mg
MS (FAB): 535 (M + Na)$^+$, 513 (M + H)$^+$, 413
NMR (270 MHz, DMSO <$D_6$>): 0,75 (m, 2H); 0,94 (m, 2H); 1,03-1,32 (m, 10H); 1,38 (s, 18H); 1,44 (m, 2H); 1,50-1,73 (m, 8H); 1,80 (m, 2H); 3,22 (m, 2H); 3,53 (m, 2H), 4,28 (d, 6Hz, 2H); 6,48 (d, 9Hz, 2H)

**Beispiel 47**
N,N'-Bis-(tert.-butoxycarbonyl)-2S.5S-diamino-1,6-dicyclohexyl-hexan-3R,4R-diol

Synthese analog Beispiel 46
MS (FAB): 513 (M + H)$^+$. 413
NMR (270 MHz. DMSO <$D_5$>): 0,65-0,96 (m, 4H); 1,03-1,28 (m, 10H); 1,30-1,45 (m, 20H); 1,54-1,70 (m, 8H); 1,82 (m, 2H); 3.11 (m, 2H): 3.89 (m, 2H); 4,22 (m, 2H); 5.88 (d, 9Hz, 2H)

**Beispiel 48**
N,N'-Bis-(tert.-butoxycarbonyl)-2S.5S-diamino-1,6-dicyclohexyl-hexan-3R,4S-diol

Synthese analog Beispiel 46
MS (FAB): 513 (M + H)$^+$. 413

**Beispiel 49**
N,N'-Bis-(4Z-aminocyclohexancarbonyl-L-phenylalanyl-L-valyl)-2S,5S-diamino-1,6-diphenyl-3S,5S-diol-dihydrochlorid

Synthese analog Beispiel 16 bzw. 6
MS (FAB): 1043 (M + H)$^+$, 1025

**Beispiel 50**
N,N'-Bis-(4Z-N-tert.-butoxycarbonylamino)-cyclohexancarbonyl-L-phenylalanyl-L-valyl)-2S,5S-diamino-1,6-diphenyl-3S,5S-diol-dihydrochlorid

Synthese analog Beispiel 6
MS (FAB): 1243 (M + H)$^+$, 1143, 1043

**Beispiel 51**
N,N'-Bis-<(2S-(1,2-dimethylethyl-sulfonylmethyl)-3-(1-naphthyl)-propionyl)-L-valyl>-2S,5S-diamino-1,6-diphenyl-hexan-3S,4S-diol

Synthese analog zu Beispiel 13
MS (FAB): 1131 (M+H)$^+$, 716
NMR (270 MHz, DMSO<D6>): 0,77 (d, 7Hz, 6H); 0,80 (d, 7Hz, 6H); 1,12 (s, 18H); 1,87 (m, 2H); 2,75 (m, 2H); 2,83 (m, 2H); 2,92-3,03 (m, 2H); 3,10-3,22 (m, 2H); ca. 3,27-3,49 (m, 6H); 3,54-3,67 (m, 2H); 4,02-4,15 (m, 4H); 4,66 (d, 6Hz, 2H), 7,01-7,09 (m, 2H): 7,10-7,25 (m, 8H); 7,28-7,43 (m, 4H); 7,48-7,68 (m, 6H); 7,79 (d, 8Hz, 2H); 7,88-7,95 (m 2H); 8,15-8,25 (m, 4H)

**Beispiel 52**
N,N'-Bis-<(2S-(1,1-dimethylethyl-sulfonylmethyl)-3-phenylpropionyl)-L-valyl>-2S,5S-diamino-1,6-diphenyl-hexan-3R,4R-diol

Synthese analog zu Beispiel 13
MS (FAB): 1053 (M+Na)$^+$, 1031 (M+H)$^+$
NMR (270 MHz, DMSO<D6>): 0,72 (d, 7Hz, 6H); 0,78 (d, 7Hz, 6H); 1,14 (s, 18H); 1,85 (m, 2H); 2,62-2,94 (m; 8H); ca. 3,20-3,35 (m, ca. 4H); 3,53 (dd, 10Hz, 14Hz, 2H); 4,02-4,13 (m, 2H); 4,50 (m, 2H); 4,64 (M, 2H); 7,01-7,10 (m, 2H); 7,12-7,39 (m, 22H); 8,05 (d, 8Hz, 2H)

**Beispiel 53**
N,N'-Bis-<(3-(1,1-dimethylethyl-sulfonyl)-propionyl)-L-valyl>-2S,5S-diamino-1,6-diphenyl-hexan-3R,4R-diol

Synthese analog zu Beispiel 13
MS (FAB): 873 (M+Na)$^+$, 851 (M+H)$^+$
NMR (270 MHz, DMSO<D6>): 0,69 (d, 6Hz, 6H); 0,73 (d, 6Hz, 6H); 1,33 (s, 18H); 1,84 (m, 2H); 2,54-2,59 (m, 6H); 2,67 (m, 2H); ca. 3,15-3,30 (m, 6H); 4,05 (dd, 7Hz, 9Hz, 2H); 4,47 (m, 2H); 4,63 (m, 2H); 7,06-7,21 (m, 10H); 7,30 (d, 9Hz, 2H); 7,94 (d, 8Hz, 2H)

**Beispiel 54**
N,N'-Bis-<(2R-(1,1-dimethylethyl-sulfonylmethyl)-3-(2-thienyl)-propionyl)-L-valyl>-2S,5S-diamino-1,6-diphenyl-hexan-3R,4R-diol

Synthese analog zu Beispiel 13
MS (FAB): 1065 (M+Na)$^+$, 1049(M+Li)$^+$
NMR (270 MHz, DMSO<D6>): 0,51(d, 7Hz, 6H); 0,56 (d, 7Hz, 6H); 1,28 (s, 18H); 1,85 (m, 2H); 2,95-3,19 (m, 8H); 3,30-3,60 (m, 8H); 3,95 (dd, 8Hz, 5,2Hz, 2H); 4,06 (m, 2H); 4,62 (d, 7Hz, 2H); 6,93 (d, 3,2Hz, 4H); 7,08-7,25 (m, 10H); 7,34 (m, 2H); 7,43 (d, 8,4Hz, 2H); 8,14 (d, 8Hz, 2H);

**Beispiel 55**
N,N'-Bis-<L-phenylalanyl-L-valyl)-4S,7S-diamino-2,9-dimethyl-decan-5,6-diol-dihydrochlorid

Synthese analog zu Beispiel 16 aus Beispiel 56
MS (FAB): 725 (M+H)$^+$

**Beispiel 56**
N,N'-Bis-(tert.-butoxycarbonyl-L-phenylalanyl-L-valyl)-4S,7S-diamino-2,9-dimethyl-decan-5,6-diol-dihydrochlorid

Synthese analog zu Beispiel 6 bzw. den Beispielen 3-5
MS (FAB): 925 (M+H)$^+$, 826, 725
NMR (270 MHz, DMSO<D6>): 0,75-0,95 (m, 24H); 1,29 (s, 18H); 1,35-1,45 (m, 4H); 1,56 (m, 2H); 1,99 (m, 2H); 2,74 (dd, 10Hz, 13Hz, 2H); 2,95 (dd, 4Hz, 13Hz, 2H); 3,23 (m, 2H); 3,88 (m, 2H); 4,13-4,28 (m, 4H); 4,45 (d, 5Hz, 2H); 7,02 (8d, 8Hz, 2H); 7,13-7,33 (m, 10H); 7,76 (d, 8Hz, 2H); 7,80 (d, 8Hz, 2H)

**Beispiel 57**
N,N'-Bis-<(2S-(1,1-dimethylethyl-sulfonylmethyl)-3-phenylpropionyl)-L-valyl>-4S,7S-diamino-2,9-dimethyl-decan-3,4-diol

Synthese analog zu Beispiel 13 bzw. den Beispielen 3-5
MS (FAB): 985 $(M+Na)^+$, 963 $(M+H)^+$
NMR (270 MHz, DMSO<D6>): 0,78 (d, 7Hz, 6H); 0,80-0,93 (m, 18H); 1,15 (s, 18H); 1,20-1,68 (m, 6H); 1,98 (m, 2H); 2,58 (dd, 10Hz, 14Hz, 2H); 2,73 (dd, 14Hz, 3Hz, 2H); 2,98 (dd, 14Hz, 4Hz, 2H); 3,23 (m, 2H); ca. 3,33 (m, 2H); 3,47-3,61 (m, 2H); 3,85 (m, 2H); 4,14 (m, 2H); 4,44 (d; 5Hz, 2H); 7,15-7,33 (m, 10H); 7,69 (d, 9Hz, 2H); 8,22 (d, 9Hz, 2H)

**Beispiel 58**
N,N'-Bis-<(2-pyridyl)-acetyl-L-valyl>-2S,5S-diamino-1,6-diphenyl-hexan-3R,4R-diol

74 mg 2S,5S-Diamino-1,6-diphenyl-hexan-3R,4R-diol dihydrochlorid und 68 mg 2-Pyridylessigsäure-hydrochlorid wurden in 2 ml DMF gelöst und mit 53 mg HOBt, 125 mg TBTU und 0,221 ml Diisopropylethylamin versetzt. Man rührte 2h bei RT und arbeitete wie üblich auf. Nach Chromatographie an Kieselgel (DCM/MeOH 95/5 bis 90/10) erhielt man 68 mg Produkt.
MS (FAB): 759 $(M+Na)^+$, 737 $(M+H)^+$
NMR (270 MHz, DMSO<D6>): 0,70 (2d, 12H); 1,88 (m, 2H); 2,62 (dd, 14Hz, 5Hz, 2H); 2,77 (dd, 14Hz, 10Hz, 2H); 3,72 (m, 4H); 4,13 (dd, 6Hz, 9Hz, 2H); 4,46 (m, 2H); 7,05-7,23 (m, 10H); 7,28-7,40 (m, 4H); 7,48 (d, 9Hz, 2H); 7,82 (dt, 8Hz, 2Hz, 2H); 7,97 (d, 9Hz, 2H); 8,54 (m, 2H)

**Beispiel 59**
N,N'-Bis-<(4-pyridyl-thio)-acetyl-L-valyl>-2S,5S-diamino-1,6-diphenyl-hexan-3R,4R-diol

74 mg 2S,5S-Diamino-1,6-diphenyl-hexan-3R,4R-diol-dihydrochlorid und 66 mg 4-Pyridylmercaptoessigsäure wurden in 2ml DMF gelöst und mit 53 mg HOBt, 125 mg TBTU und 0,177 ml Diisopropylamin versetzt. Man rührte 2h bei RT, entfernte das Lösungsmittel i. Vac. und verrührte den Rückstand für 30 min zwischen EE und $NaHCO_3$-Lösung. Das Unlösliche wurde abfiltriert und mit EE und Wasser gewaschen. Das Rohprodukt wurde in warmem DMF gelöst, die Lösung filtriert und in EE verrührt. Der Niederschlag wurde abgesaugt und getrocknet. Ausbeute: 76 mg
MS (FAB): 801 $(M+H)^+$
NMR (270 MHz, DMSO<D6>): 0,68 (2d, 12H); 1,84 (m, 2H); 2,62 (dd, 14Hz, 5Hz, 2H); 2,78 (dd, 14Hz, 9Hz, 2H); 3,28 (m, 2H); 3,73 (d, 15Hz,2H); 3,90 (d, 15Hz, 2H); 4,17 (dd, 6Hz, 9Hz, 2H); 4,43 (m, 2H); 4,70 (m, 2H); 7,05-7,20 (m, 10H); 7,30 (m, 4H); 7,58 (d, 9Hz, 2H); 8,03 (d, 9Hz, 2H); 8,34 (m, 4H)

**Beispiel 60**
N,N'-Bis-<L-phenylalanyl-D-valyl>-2S,5S-diamino-1,6-diphenyl-hexan-3S,4S-diol-dihydrochlorid

Synthese analog Beispiel 16
MS (FAB): 793 $(M+H)^+$

**Beispiel 61**
N,N'-Bis-<D-phenylalanyl-L-valyl>-2S,5S-diamino-1,6-diphenyl-hexan-3S,4S-diol-dihydrochlorid

Synthese analog Beispiel 16
MS (FAB): 793 $(M+H)^+$

**Beispiel 62**
N,N'-Bis-<tert.-butoxycarbonyl-L-phenylalanyl-D-valyl>-2S,5S-diamino-1,6-diphenyl-hexan-3S,4S-diol

Synthese analog Beispiel 6
MS (FAB): 993 $(M+H)^+$ ,893, 793
NMR (270 MHz, DMSO<D6>): 0,42 (d, 7Hz, 6H); 0,47 (d, 7Hz, 6H); 1,26 (s, 18H); 2,58 (m, 2H); 2,73 (m, 2H); 2,98 (dd, 13Hz, 5Hz, 2H); 3,16 (m, 2H); 3,40 (m, 2H); 4,00-4,32 (m, 6H); 4,85 (d, 5Hz, 2H); 6,86 (d, 9Hz, 2H); 7,07-7,30 (m, 20H); 7,74 (d, 9Hz, 2H); 8,19 (d, 9Hz, 2H)

**Beispiel 63**
N,N'-Bis-<tert.-butoxycarbonyl-D-phenylalanyl-L-valyl>-2S,5S-diamino-1,6-diphenyl-hexan-3S,4S-diol

Synthese analog Beispiel 6
MS (FAB): 1015 $(M+Na)^+$ ,993 $(M+H)^+$, 893, 793
NMR (270 MHz, DMSO<$D_6$>): 0,72, (d, 7Hz, 12Hz); 1,30 (s, 18H); 1,84 (s, 2H); 2,65-2,82 (m, 4H); 2,88-3,02 (m, 4H); 3,37 (m, 2H); 4,00-4,13 (m, 4H); 4,28 (m, 2H); 4,63 (d, 7Hz, 2H); 6,96 (d, 8Hz, 2H); 7,05-7,35 (m, 20H); 7,59 (d, 8Hz,2H); 7,82 (d,9Hz, 2H)

**Beispiel 64**
N,N′-Bis-<L-phenylalanyl-glycyl>-2S,5S-diamino-1,6-diphenyl-hexan-3S,4S-diol-dihydrochlorid

Synthese analog Beispiel 16
MS (FAB): 709 $(M+H)^+$

**Beispiel 65**
N,N′-Bis-<L-phenylalanyl-L-isoleucyl>-2S,5S-diamino-1,6-diphenyl-hexan-3S,4S-diol-dihydrochlorid

Synthese analog Beispiel 16
MS (FAB): 821 $(M+H)^+$

**Beispiel 66**
N,N′-Bis-<L-leucyl-glycyl>-2S,5S-diamino-1,6-diphenyl-hexan-3S,4S-diol-dihydrochlorid

Synthese analog Beispiel 20
MS (FAB): 641 $(M+H)^+$

**Beispiel 67**
N,N′-Bis-<tert.-butoxycarbonyl-L-phenylalanyl-glycyl>-2S,5S-diamino-1,6-diphenyl-hexan-3S,4S-diol

Synthese analog Beispiel 6
MS (FAB): 931 $(M+Na)^+$, 909 $(M+H)^+$, 809, 709
NMR (270 MHz, DMSO<$D_6$>): 1,38 (s, 18H); 2,58-2,78 (m, 4H); 2,92-3,09 (m, 4H); 3,43-3,62 (m, 4H); 3,78 (dd, 16Hz, 5Hz, 2H); 4,05 (m, 2H); 4,19 (m, 2H); 4,83 (d, 5Hz,2H); 6,92 (d, 9Hz, 2H); 7,10-7,29 (m, 10H); 7,90 (d, 9Hz, 2H); 8,01 (m, 2H)

**Beispiel 68**
N,N′-Bis-<tert.-butoxycarbonyl-L-phenylalanyl-L-isoleucyl>-2S,5S-diamino-1,6-diphenyl-hexan-3S,4S-diol

Synthese analog Beispiel 6
MS (FAB): 1021 $(M+H)^+$, 921, 821
NMR (270 MHz, DMSO<$D_6$>): 0,70-0,85 (m, 12H); 1,03 (m, 2H); 1,29 (s, 18H); 1,37 (m, 2H); 1,65 (m, 2H); 2,68-2,80 (m, 4H); 2,84-3,04 (m, 4H); 3,39 (m, 2H); 4,00-4,13 (m, 4H); 4,20 (m, 2H); 4,64 (d, 7Hz, 2H); 7,02 (d, 9Hz, 2H); 7,05-7,33 (m, 20H); 7,62-7,73 (m, 4H)

**Beispiel 69**
N,N′-Bis-<tert.-butoxycarbonyl-L-leucyl-glycyl>-2S,5S-diamino-1,6-diphenyl-hexan-3S,4S-diol

Synthese analog Beispiel 6
MS (FAB): 863 $(M+Na)^+$, 841 $(M+H)^+$, 741, 641
NMR (270 MHz, DMSO<$D_6$>): 0,83 (d, 6Hz, 6H); 0,87 (d, 6Hz, 6H); 1,38 (s, 18H); ca. 1,42 (m, 4H); 1,60 (m, 2H); 2,62 (dd, 14Hz, 10Hz, 2H); 3,03 (dm, 14Hz, 2H); 3,44 (m, 2H); 3,52 (dd, 16Hz, 5Hz, 2H); 3,72 (dd, 16Hz, 5Hz,2H); 3,90-4,08 (m, 4H); 4,79 (d, 5Hz, 2H); 6,93 (d, 9Hz, 2H); 7,10-7,28 (m, 10H); 7,78-7,90 (m, 4H)

**Beispiel 70**
N,N′-Bis-<L-phenylalanyl-L-seryl>-2S,5S-diamino-1,6-diphenyl-hexan-3S,4S-diol-dihydrochlorid

Synthese analog Beispiel 16
MS (FAB):769 $(M+H)^+$

**Beispiel 71**
N,N'-Bis-<5S-amino-4S-hydroxy-7-methyl-2R-propyl-octanoyl>-2S,5S-diamino-1,6-diphenyl-hexan-3S,4S-diol-dihydrochlorid

56 mg 2S,5S-Diamino-1,6-diphenyl-hexan-3S,4S-diol-dihydrochlorid und 134 mg N-tert.-Butoxycarbonyl-5S-amino-7-methyl-2R-propyl-4S-(tert.-butyl-dimethylsilyl-oxy)-octansäure wurden in 3 ml DMF gelöst und mit 43 mg HOBt, 101 mg TBTU und 155 mg Diisopropylethylamin versetzt. Man rührte 4h bei RT, entfernte das Lösungsmittel i. Vac. und verteilte den Rückstand zwischen DCM und Wasser. Die organische Phase wurde mit $KHSO_4$-, $NaHCO_3$-Lösung und Wasser extrahiert. Nach Trocknung über wasserfreiem Natriumsulfat engte man ein und chromatographierte den Rückstand an Kieselgel (Cyclohexan/EE 3/1). Man erhielt als Ausbeute 157 mg N,N'-Bis-<N-tert.-butoxycarbonyl-5S-amino-7-methyl-2R-propyl-4S-(tert.-butyl-dimethylsilyl-oxy)-octanoyl>-2S,5S-diamino-1,6-diphenyl-hexan-3S,4S-diol-dihydrochlorid. Behandlung mit HCl in Dioxan analog Beispiel 16 ergab das Produkt.

Die Kupplungskomponente N-tert.-Butoxycarbonyl-5S-amino-7-methyl-2R-propyl-4S-(tert.-butyl-dimethylsilyl-oxy)-octansäure wurde analog der Beschreibung in Beispiel 27 dargestellt.

Das Ausgangsmaterial (5S)-5-<(1S)-1-(N-Boc-amino)-3-methylbutyl>dihydrofuran-2(3H)-on wurde dafür zusätzlich mit Allylbromid alkyliert und dann hydriert (analog der Darstellung von Verbindung 11 bei Fray et al.).

MS (FAB): 727 $(M+H)^+$

NMR (270 MHz, DMSO<D6>): 0,80-0,88 (m, 18H); 1,08-1,74 (m, 18H); ca. 2,55 (m, 2H); 2,72-2,88 (m, 4H); 3,02-3,18 (m, 4H); 3,48 (d, 7Hz, 2H); 3,99 (m, 2H); 7.10-7,19 (m, 2H); 7,20-7,32 (m, 10H); 7,74 (m, 6H); 8,16 (d, 9Hz, 2H)

**Beispiel 72**
N,N'-Bis-< L-phenylalanyl-L-cyclohexylglycyl >-2S,5S-diamino-1,6-diphenyl-hexan-3R,4R-diol-dihydrochlorid

Synthese analog Beispiel 16
MS (FAB): 873 $(M+H)^+$

**Beispiel 73**
N,N'-Bis-<tert.-butoxycarbonyl-L-phenylalanyl-L-cyclohexylglycyl >-2S,5S-diamino-1,6-diphenyl-hexan-3R,4R-diol

Synthese analog Beispiel 6
MS (FAB): 1073 $(M+H)^+$, 973, 873
NMR (270 MHz, DMSO<$D_6$>): 0,82-1,66 (m, ca 22H); 1,29 (s, 18H); 2,56-2,97 (m, 8H); ca. 3,30 (m, 2H); 4,08-4,22 (m, 4H); 4,50 (m, 2H); 4,63 (m, 2H); 7,02 (d, 9Hz, 2H); 7,04-7,32 (m, 20H); 7,47 (d, 9Hz, 2H); 7,56 (d, 9Hz, 2H)

**Beispiel 74**
N,N'-Bis-<L-methionyl-L-valyl>-2S,5S-diamino-1,6-diphenyl-hexan-3S,4S-diol-dihydrochlorid

Synthese analog Beispiel 16
MS (FAB): 761 $(M+H)^+$

**Beispiel 75**
N,N'-Bis-<tert.-butoxycarbonyl-L-methionyl-L-valyl>-2S,5S-diamino-1,6-diphenyl-hexan-3S,4S-diol

Synthese analog Beispiel 6
MS (FAB): 961 $(M+H)^+$, 861, 761
NMR (270 MHz, DMSO<$D_6$>): 0,75 (d, 6Hz, 12H); 1,38 (s, 18H); 1,70-1,90 (m, 6H); 2,02 (s, 6H); ca. 2,37-2,5 (m, 4H); ca 3,32 (m, 2H); 3,94-4,10 (m, 6H); 4,63 (d, 7Hz, 2H); 7,04-7,20 (m, 12H); 7,49-7,59 (m, 4H)

**Beispiel 76**
N,N'-Bis-<(O-methyl-tyrosyl)-L-valyl>-2S,5S-diamino-1,6-diphenyl-hexan-3S,4S-diol-dihydrochlorid

Synthese analog Beispiel 16

MS (FAB): 853 $(M+H)^+$

**Beispiel 77**
N,N′-Bis-<tert.-butoxylcarbonyl-(O-methyl-tyrosyl)-L-valyl>-2S,5S-diamino-1,6-diphenyl-hexan-3S,4S-diol

Synthese analog Beispiel 16
MS (FAB): 1053 $(M+H)^+$, 953, 853
NMR (270 MHz DMSO<$D_6$>): 0,73-0,83 (m, 12H); 1,29 (s, 12H); 1,84 (m, 2H); 2,60-3,02 (m, 8H); 3,36 (m, 2H); 3,70 (s, 6H); 3,99-4,18 (m, 6H); 4,64 (d, 6H, 2H); 6,82 (d, 9Hz, 4H); 6,98 (d, 9Hz, 2H); 7,05-7,22 (m, 14H); 7,59 (d, 9Hz, 2H); 7,65 (d, 9Hz, 2H)

**Beispiel 78**
N,N′-Bis-<L-tyrosyl-L-valyl>-2S,5S-diamino-1,6-diphenyl-hexan-3S,4S-diol-dihydrochlorid

Synthese analog Beispiel 16
MS (FAB): 825 $(M+H)^+$

**Beispiel 79**
N,N′-Bis-<(N-tert.-butoxycarbonyl-O-tert.-butyl-L-tyrosyl)-L-valyl>-2S,5S-diamino-1,6-diphenyl-hexan-3S,4S-diol

Synthese analog Beispiel 6
MS (FAB): 1137 $(M+H)^+$, 1037, 937
NMR (270 MHz DMSO<$D_6$>): 0,72-0,85 (m, 12H); 1,25 (s, 18H); 1,28 (s, 18H); 1,85 (m, 2H); 262-2,82 (m, 4H); 2,84-3,01 (m, 4H); 3,36 (m, 2H); 3,98-4,12 (m, 4H); 4,19 (m, 2H); 4,64 (d, 7Hz, 2H); 6,85 (d,8Hz, 4H); 7,02 (d, 9Hz, 2H); 7,05-7,21 (m, 18H); 7,60 (d, 8Hz, 2H); 7,66 (d, 9Hz, 2H)

**Beispiel 80**
N,N′-Bis-<$N^6$-benzyloxycarbonyl-$N^2$-(tert.-butoxycarbonyl-L-lysyl)-L-valyl>-2S,5S-diamino-1,6-diphenyl-hexan-3R,4R-diol

Synthese analog Beispiel 6
MS (FAB/LiI): 1229 $(M+H)^+$

**Beispiel 81**
N,N′-Bis-<$N^6$-benzyloxycarbonyl-$N^2$-(tert.-butoxycarbonyl-L-phenylalanyl)-L-lysyl>-2S,5S-diamino-1,6-diphenyl-hexan-3R,4R-diol

Synthese analog Beispiel 6
MS (FAB): 1319 $(M+H)^+$, 1219, 1185
NMR (270 MHz, DMSO<D6>): 1,08-1,47 (m, 30H); 2,60-2,82 (m, 6H); 2,87-3,00 (m, 6H); 3,23 (m, 2H); 4,08-4,23 (m, 4H); 4,36 (m, 2H); 4,69 (m, 2H); 4,99 (s, 4H); 6,94 (d, 9Hz, 2H); 7,04-7,40 (m, 32H); 7,46 (d, 8Hz, 2H); 7,69 (d, 9Hz, 2H)

**Beispiel 82**
N,N′-Bis-<L-glutamyl-L-valyl>-2S,5S-diamino-1,6-diphenyl-hexan-3S,4S-diol-dihydrochlorid

Synthese analog Beispiel 16
MS (FAB/LiI): 763 $(M+Li)^+$, 757 $(M+H)^+$
NMR (270 MHZ, DMSO<$D_6$>): 0,81 (d, 6Hz, 6H); 0,85 (d, 6Hz, 6H); 0,78-1,98 (m, 6H); 2,20-2,38 (m, 4H); 2,76 (m, 2H); 2,97 (m, 2H); ca. 3,35 (m, ca 2H); 3,89 (m, 2H); 4,01-4,14 (m, 4H); 4,68 (d, 7Hz, 2H); 7,06-7,21 (m, 10H); 7,68 (d, 8Hz,2H); 8,22 (m, 6H); 8,46 (d, 9Hz, 2H)

**Beispiel 83**
N,N′-Bis-<tert.-butoxycarbonyl-L-glutamyl-L-valyl>-2S,5S-diamino-1,6-diphenyl-hexan-3S,4S-diol

Synthese aus Beispiel 84 durch katalytische Hydrierung an Pd/Kohle in Eisessig/Wasser 9/1.
MS (FAB): 979 $(M+Na)^+$, 958( $M+H)^+$

NMR (270 MHZ, DMSO<$D_6$>): 0,70-0,82 (m, 12H); 1,38 (s, 18H); 1,62-1,93 (m, 6H); 2,17-2,29 (m, 4H); 2,74 (m, 2H); 2,95 (dm, 13Hz, 2H); ca. 3,35 (m, 2H); 3,90-4,09 (m, 6H); 4,12 (m, 2H); 7,00-7,20 (m, 12H); 7,48-7,62 (m, 4H)

**Beispiel 84**
N,N'-Bis-<(N-tert.-butoxycarbonyl-O-benzyl-L-glutamyl)-L-valyl>-2S,5S-diamino-1,6-diphenyl-hexan-3S,4S-diol

Synthese analog Beispiel 6
MS (FAB): 1159 $(M+Na)^+$, 1137 $(M+H)^+$, 1037
NMR (270 MHZ, DMSO<$D_6$>): 0,75 (d, 6Hz, 12H); 1,37 (s, 18H); 1,70-1,98 (m,6H); 2,33-2,45 (m, 2H); 2,76 (m, 2H); 2,93 (m, 2H); ca. 3,3 (m, 2H); 3,94-4,08 (m, 6H); 4,60 (s, 7Hz, 2H); 5,08 (s,4H); 7,03-7,17 (m, 12H); 7,30-7,48 (m, 10H); 7,50 (d, 8Hz, 2H); 7,58 (d, 9Hz, 2H)

**Beispiel 85**
N,N'-Bis-<glycyl-L-valyl>-2S,5S-diamino-1,6-diphenyl-hexan-3R,4R-diol-dihydrochlorid

Synthese analog Beispiel 16
MS (FAB): 635 $(M+Na)^+$, 613 $(M+H)^+$

**Beispiel 86**
N,N'-Bis-<tert.-butoxycarbonyl-glycyl-L-valyl>-2S,5S-diamino-1,6-diphenyl-hexan-3R,4R-diol

Synthese analog Beispiel 6
MS (FAB): 835 $(M+Na)^+$, 813 $(M+H)^+$
NMR (270 MHz, DMSO<$D_6$>): 0,70 (d, 7Hz, 12H); 1,38 (s, 18H); 1,84 (m, 2H); 2,62 (dd, 14Hz, 4Hz, 2H); 2,87 (dd, 14Hz, 10Hz, 2H); 3,26 (m, 2H); 3,52 (d, 6Hz, 4H); 4,13 (m, 2H); 4,42 (m, 2H); 4,69 (m, 2H); 7,03 (m, 2H); 7,08-7,21 (m, 10H); 7,38 (d, 9Hz, 2H); 7,50 (d, 9Hz, 2H)

**Beispiel 87**
N,N'-Bis-<L-leucyl-L-valyl>-2S,5S-diamino-1,6-diphenyl-hexan-3S,4S-diol-dihydrochlorid

Synthese analog Beispiel 16
MS (FAB): 747 $(M+Na)^+$, 725 $(M+H)^+$

**Beispiel 88**
N,N'-Bis-<tert.-butoxycarbonyl-L-leucyl-L-valyl>-2S,5S-diamino-1,6-diphenyl-hexan-3S,4S-diol

Synthese analog Beispiel 6
MS (FAB): 947 $(M+Na)^+$, 925 $(M+H)^+$, 825, 725
NMR (270 MHz, DMSO<$D_6$>): 0,72-0,80 (m, 12H); 0,85 (d, 7Hz, 6H); 0,89 (d, 7Hz, 6H); 1,28-1,54 (m, 22H); 1,60 (m, 2H); 1,81 (m, 2H); 2,76 (dd, 13Hz, 9Hz, 2H); 2,93 (dd, 13Hz, 4Hz, 2H); ca. 3,33 (m, 2H); 3,92-4,09 (m, 6H); 4,60 (d, 7Hz, 2H); 7,04 (d, 8Hz, 2H); 7,05-7,20 (m, 10H); 7,48 (d, 9Hz, 4H)

**Beispiel 89**
N,N'-Bis-<L-(S-dioxo)methionyl-L-valyl>-2S,5S-diamino-1,6-diphenyl-hexan-3R,4R-diol-dihydrochlorid

Synthese analog Beispiel 16
MS (FAB): 847 $(M+Na)^+$, 825 $(M+H)^+$

**Beispiel 90**
N,N'-Bis-<tert.-butoxycarbonyl-L-(S-dioxo)methionyl-L-valyl>-2S,5S-diamino-1,6-diphenyl-hexan-3R,4R-diol

Synthese analog Beispiel 6
MS (FAB): 1074 $(M+Na)^+$
NMR (270 MHz, DMSO<$D_6$>): 0,65-0,78 (m, 12H); 1,39 (s, 18H); 1,74-2,07 (m, 6H); 2,63 (m, 2H); 2,78 (m, 2H); 3,07 (m, 4H); 3,26 (m, 2H); 3,98-4,17 (m, 4H); 4,44 (m, 2H); 4,67 (m, 2H); 7,07-7,23 (m, 12H); 7,49 (d, 9Hz, 2H); 7,53 (d, 9Hz, 2H)

**Beispiel 91**

N,N'-Bis-<(2S-(1,1-dimethylethyl-sulfonylmethyl)-3-phenylpropionyl)-L-tert.-butylglycyl>-2S,5S-diamino-1,6-diphenyl-hexan-3R,4R-diol

Synthese analog Beispiel 13

MS (FAB): 1081 (M + Na)$^+$, 1059 (M + H)$^+$

NMR (270 MHz, DMSO<$D_6$>): 0,83 (s, 18H); 1,12 (s, 18H); 2,39 (dd, 11Hz, 14Hz, 2H); 2,56-2,72 (m, 4H); 2,73-2,90 (m, 4H); ca. 3,25-3,40 (m, ca 4H); 3,53 (dd, 10Hz, 14Hz, 2H); 4,20 (d, 9Hz, 2H); 4,54 (m, 2H); 4,62 (m, 2H); 6,98 (m, 2H); 7,07-7,36 (m, 18H); 7,47 (d, 9Hz, 2H); 7,98 (d, 9Hz,2H)

**Beispiel 92**

N,N'-Bis-<(2S-(1,1-dimethylethyl-sulfonylmethyl)-3-phenylpropionyl)-L-neopentylglycyl>-2S,5S-diamino-1,6-diphenyl-hexan-3R,4R-diol

Synthese analog Beispiel 13

MS (FAB): 1109 (M + Na)$^+$, 1087 (M + H)$^+$

NMR (270 MHz, $CDCl_3$): 0,86 (s, 18H); 1,08 (dd, 8Hz, 14Hz, 2H); 1,35 (s; 18H); 1,58 (dd, 14Hz, 4Hz, 2H); 2,75-3,45 (m, ca. 8H); 3.80 (m, 2H); 4,12 (m, 2H); 5,80 (d, 8Hz, 2H); 6,27 (d, 8Hz, 2H); 7,10-7,36 (m, ca. 10H)

**Beispiel 93**

N,N'-Bis-<(2-S-hydroxy-3-phenylpropionyl)-L-valyl>-2S,5S-diamino-1,6-diphenyl-hexan-3S,4S-diol

Zu 0,065 mmol N,N'-Bis-<-L-valyl>-2S,5S-diamino-1,6-diphenyl-hexan-3S,4S-diol-hydrochlorid und 33 mg S-Phenylmilchsäure in 4 ml DMF wurden 27 mg HOBt, 64 mg TBTU und dann langsam 0,088 ml Diisopropylethylamin gegeben. Nach 15 min bei RT entfernte man das DMF im Vacuum, nahm den Rückstand in EE auf und extrahierte mit $KHSO_4$-, $NaHCO_3$-Lösung und Wasser. Die org. Phase wurde mit $MgSO_4$ getrocknet und eingeengt, der Rückstand mit Ether verrieben und abgesaugt.

Ausbeute: 43 mg

MS (FAB): 795 (M + H)$^+$

NMR (270 MHz, DMSO<$D_6$>): 0,63 (d, 7Hz, 6H); 0,67 (d, 7Hz, 6H); 1,82 (m, 2H); 2,64-2,79 (m, 4H); 2,91-3,04 (m, 4H); 3,38 (m, 2H); 3,97-4,17 (m, 6H); 4,72 (d, 6Hz, 2H); 5,77 (d, 6Hz, 2H); 7,08-7,29 (m, 20H); 7,38 (d, 9Hz, 2H); 7,85 (d, 8Hz, 2H)

**Beispiel 94**

N,N'-Bis-<(2S-hydroxy-4-phenylbutyryl)-L-valyl>-2S,5S-diamino-1,6-diphenyl-hexan-3S,4S-diol

Synthese analog Beispiel 93

MS (FAB): 845 (M + Na)$^+$, 823 (M + H)$^+$

NMR (270 MHz, DMSO<$D_6$>): 0,73 (d, 5Hz, 6H); 0,76 (d, 5Hz, 6H); 1,76-2,00 (m, 6H); 2,55-2,78 (m, 6H); 2,98 (dm, 14Hz, 2H); 3,39 (m, 2H); 3,89 (m, 2H); 4,00-4,18 (m, 4H); 4,75 (d, 6Hz,2H); 5,88 (d, 6Hz, 2H); 7,05-7,32 (m, 20H); 7,45 (d, 9Hz, 2H); 7,88 (d,8Hz, 2H)

**Beispiel 95**

N,N'-Bis-<(2-(1-imidazolylmethyl)-3-phenyl-propionyl)-L-valyl>-2S,5S-diamino-1,6-diphenyl-hexan-3S,4S-diol (aus "Diastereomer 1")

35,8 mg 2S,5S-Diamino-1,6-diphenyl-hexan-3S,4S-dioldihydrochlorid wurden mit 90 mg 2-(1-Imidazolylmethyl)-3-phenyl-propionyl-L-valin ("Diastereomer 1") in 2 ml DMF gelöst und mit 32 mg HOBt, 77 mg TBTU und anschließend mit 0,163 ml Diisopropylethylamin bei RT versetzt. Man rührte 3h, entfernte das Lösungsmittel i. Vac. und verteilte den Rückstand zwischen EE und $NaHCO_3$-Lösung. Die org. Phase wurde mit halbkonz. NaCl-Lösung gewaschen, getrocknet und eingeengt. Der Rückstand wurde mit Diethylether verrieben, abgesaugt und anschließend an Kieselgel chromatographiert (EE/MeOH 85/15). Man erhielt 57 mg Produkt.

MS (FAB): 923 (M + H)$^+$

Die Darstellung des 2-(1-Imidazolylmethyl)-3-phenylpropionyl-L-valin verlief folgendermaßen: 1,53 g Benzylacrylsäureester (J.Med.Chem. 31,1839, (1988)) und 550 mg Imidazol wurden in 30 ml EtOH gelöst und unter Argon bei RT mit 40 mg NaH versetzt. Nach 7d wurde die Reaktionslösung in 50 ml $KH_2PO_4$-

Lösung gegossen und 3x mit 50 ml Methyl-tert.-butylether extrahiert. Die org. Phase wurde 2x mit $NaHSO_4$ extrahiert, die wässrige Phase mit $K_2CO_3$ alkalisch gestellt und erneut 2x mit 50 ml Methyl-tert.-butylether extrahiert. Man erhält nach Einengung 390 mg 2-Benzyl-3-(1-imidazolyl)propansäureethylester. Dieser wurde mit NaOH verseift und nach der PPA-Methode mit Valinmethylester gekuppelt. Die Diastereomere wurden mit EE/MeOH 10/1 getrennt.

0,34 = Diastereomer 1

0,18 = Diastereomer 2

Verseifung mit NaOH in Dioxan/Wasser führte zu den Kupplungskomponenten für Beispiele 95 und 96.

**Beispiel 96**

N,N'-Bis-<(2-(1-imidazolylmethyl)-3-phenyl-propionyl)-L-valyl>-2S,5S-diamino-1,6-diphenyl-hexan-3S,4S-diol (aus "Diastereomer 2")

Darstellung siehe Beispiel 95

MS (FAB): 923 $(M+H)^+$

**Beispiel 97**

N,N'-Bis-<3-(4-amino-1-piperidyl-sulfonyl)-2-benzylpropionyl-L-valyl>-2S,5S-diamino-1,6-diphenyl-hexan-3R,4R-diol

Synthese analog Beispiel 16

MS (FAB): 1115 $(M+H)^+$

**Beispiel 98**

N,N'-Bis-<2-benzyl-3-(4-tert.-butoxycarbonyl-amino-1-piperidyl-sulfonyl)-propionyl-L-valyl>-2S,5S-diamino-1,6-diphenyl-hexan-3R,4R-diol

57 mg N,N'-Bis-<L-valyl>-2S,5S-diamino-1,6-diphenyl-hexan-3R,4R-diol dihydrochlorid und 129 mg 2-Benzyl-3-(4-tert.-butoxycarbonyl-amino-1-piperidyl-sulfonyl)- propionsäure wurden in 1 ml DMF gelöst und mit 41 mg HOBt, 96 mg TBTU und 135 µl Diisopropylethylamin versetzt. Nach 20 min entfernte man das Lösungsmittel i. Vac., nahm den Rückstand in DCM auf und extrahierte ihn mit $KHSO_4$-, $KHCO_3$-Lösung und Wasser. Nach Trocknung und Einengung wurde der zähe Rückstand in wenig DCM/MeOH gelöst und mit Diethylether ausgefällt. Ausbeute 64 mg.

MS (FAB): 1337 $(M+Na)^+$, 1315 $(M+H)^+$, 1237, 1215, 1137, 1115

2-Benzyl-3-(4-tert.-butoxycarbonyl-amino-1-piperidylsulfonyl)-propionsäure wurde in Analogie zu Beispiel 13 synthetisiert nach: J. Med. Chem. 31 1839 (1988). Die Zwischenstufe des Benzylacrylsäureesters wurde mit Thioessigsäure umgesetzt zu 3-Acetylthio-2-benzylpropionsäurebenzylester. Anschließende Oxidation mit Chlor lieferte 2-Benzyl-3-Chlorsulfonyl-propionsäurebenzylester, der durch Kupplung mit 4-tert.-butoxycarbonylamino-piperidin und anschließende Hydrierung in obige Kupplungskomponente umgesetzt wurde.

**Beispiel 99**

N,N'-Bis-<3-(4-amino-1-piperidyl-carbonyl)-2R-benzylpropionyl-L-valyl>-2S,5S-diamino-1,6-diphenyl-hexan-3R,4R-diol-dihydrochlorid

Synthese analog Beispiel 16

MS (FAB): 1043 $(M+H)^+$

**Beispiel 100**

N,N'-Bis-<2R-benzyl-3-(4-tert.-butoxycarbonyl-amino-1-piperidyl-carbonyl)-propionyl-L-valyl>-2S,5S-diamino-1,6-diphenyl-hexan-3R,4R-diol-dihydrochlorid

57 mg N,N'-Bis-<L-valyl>-2S,5S-diamino-1,6-diphenyl-hexan-3R,4R-diol-dihydrochlorid und 129 mg 2R-Benzyl-3-(4-tert.-butoxycarbonyl-amino-1-piperidyl-carbonyl)-propionsäure (Synthese durch Kupplung von 4-tert.-Butoxycarbonylamino-piperidin auf 2-R-Benzyl-3-carboxypropionsäurebenzylester <s. Literaturstelle in Beispiel 102>) wurden in 1 ml DMF gelöst und mit 41 mg HOBt, 96 mg TBTU und dann langsam 0,135 ml Diethylisopropylamin versetzt. Nach 20 min wurde das Lösungsmittel i. Vac. entfernt, der Rückstand in EE aufgenommen und mit $KHSO_4$-, $NaHCO_3$-Lösung und Wasser extrahiert. Die org. Phase wurde über $MgSO_4$

getrocknet und eingeengt. Der Rückstand wurde in wenig DCM gelöst, mit Diethylether gefällt und abfiltriert.
Ausbeute 64 mg
MS (FAB): 1265 $(M+Na)^+$, 1243 $(M+H)^+$

**Beispiel 101**
N,N'-Bis-<(2R-benzyl-3-carboxyl)-propionyl-L-valyl>-2S,5S-diamino-1,6-diphenyl-hexan-3R,4R-diol

Synthese aus Beispiel 102 durch Behandlung mit Trifluoressigsäure
MS (FAB): 901 $(M+Na)^+$, 879 $(M+H)^+$

**Beispiel 102**
N,N'-Bis-<(2R-benzyl-3-tert.-butoxycarbonyl)-propionyl-L-valyl>-2S,5S-diamino-1,6-diphenyl-hexan-3R,4R-diol

45 mg N,N'-Bis-<L-valyl>-2S,5S-diamino-1,6-diphenyl-hexan-3R,4R-diol-dihydrochlorid wurden mit 75 mg 2R-Benzyl-3-tert.-butoxycarbonyl-propionsäure in 2 ml DMF gelöst und mit 37 mg HOBt, 87 mg TBTU und 112 µl Ethyldiisopropylamin versetzt. Man rührte 15 min bei RT, entfernte das DMF i. Vac. nahm den Rückstand in EE auf und extrahierte mit $KHSO_4$-, $NaHCO_3$-Lösung und Wasser. Die org. Phase wurde über $MgSO_4$ getrocknet und eingeengt. Der Rückstand wurde mit Diethylether verrieben und abfiltriert.
Ausbeute: 44mg
MS (FAB): 1013 $(M+Na)^+$ 991 $(M+H)^+$
NMR (270 MHz, DMSO<$D_6$>): 0,69 (d, 6Hz, 6H); 0,74 (d, 6Hz, 6H); 1,31 (s, 18H); 1,83 (m, 2H); 1,95 (m, 2H); 2,32-2,47 (m, 4H); 2,60-2,87 (m, 6H); 2,98 (m, 2H); 3,29 (m, 2H); 4,09 (dd, 8Hz, 7Hz, 2H); 4,46 (m, 2H); 4,64 (m, 2H); 7,02-7,31 (m, 10H); 7,38 (d, 9Hz, 2H); 7,80 (d, 8Hz, 2H)
Die Herstellung des carboxygeschützten Bernsteinsäurederivates in enantiomerenreiner Form wurde nach Evans durchgeführt (D.A.Evans et.al. J. Am. Chem. Soc. 104, 1737 (1982), J.J.Plattner et.al. J. Med. Chem. 31, 2277 (1988)).

**Beispiel 103**
N,N'-Bis-<(3-amino-2-benzyl)-propionyl-L-valyl>-2S,5S-diamino-1,6-diphenyl-hexan-3R,4R-diol-dihydrochlorid (aus "Diastereomer 1")

Synthese analog Beispiel 16 aus Beispiel 105
MS (FAB): 843 $(M+Na)^+$ 821 $(M+H)^+$

**Beispiel 104**
N,N'-Bis-<(3-amino-2-benzyl)-propionyl-L-valyl>-2S,5S-diamino-1,6-diphenyl-hexan-3R,4R-diol-dihydrochlorid (aus "Diastereomer 2")
Synthese analog Beispiel 16 aus Beispiel 106
MS (FAB): 843 $(M+Na)^+$ 821 $(M+H)^+$

**Beispiel 105**
N,N'-Bis-<(2-benzyl-3-tert.-butoxycarbonyl-amino)-propionyl-L-valyl>-2S,5S-diamino-1,6-diphenyl-hexan-3R,4R-diol (aus "Diastereomer 1")

37 mg 2S,5S-Diamino-1,6-diphenyl-hexan-3R,4R-dioldihydrochlorid wurden mit 98 mg N,N'-Bis-(2-benzyl-3-tert.-butoxycarbonyl-amino)-propionyl-L-valin nach der TBTU-Methode gekuppelt. Nach üblicher Aufarbeitung und Chromatographie (DCM/Methanol 98/2 auf 95/5) erhielt man 28 mg Produkt.
MS (FAB): 1043 $(M+Na)^+$ 1021 $(M+H)^+$, 921, 821
Der Baustein N,N'-Bis-(2-benzyl-3-tert.butoxycarbonylamino)-propionyl-L-valin wurde wie folgt dargestellt: 2,3 g Natrium wurden in 170 ml EtOH gelöst und mit 32 ml Cyanessigsäureethylester versetzt. Unter Rühren wurden 11,5 ml Benzylchlorid zugetropft. Die Lösung stand über Nacht bei RT. Man filtrierte das NaCl ab und destillierte das Lösungsmittel ab. Der Rückstand wurde in EE gelöst und mit $H_2O$ extrahiert. Die org. Phase wurde eingeengt, der Rückstand i. Vac. destilliert (0,5 mm Hg/120-125 °C).
Ausbeute: 8,1 g
Der erhaltene Benzylcyanessigsäureethylester wurde in 200 ml EtOH gelöst und über Raney-Nickel hydriert. Nach Absaugen des Katalysators und Einengen erhielt man 8,2 g Öl, nach Chromatographie über

Kieselgel (EE nach EE/MeOH 5/1) 5,5 g an 3-Amino-2-benzylpropionsäureethylester.
Diese Verbindung wurde mit $Boc_2O$ zu 2-Benzyl-3-(tert.-butoxycarbonylamino)-propionsäureethylester umgesetzt, verseift und mit H-Val-OMe nach der PPA-Methode gekuppelt. Die erhaltene Diastereomere wurden per Chromatographie (Toluol/Diisopropylether 1/1) getrennt.
Rf = 0,140 = Diastereomer 1
Rf = 0,097 = Diastereomer 2
Verseifung mit NaOH in Dioxan/Wasser führte zu den Kupplungskomponenten für Beispiel 105 und 106.

**Beispiel 106**
N,N′-Bis-<(2R-benzyl-3-tert.butoxycarbonyl-amino)-propionyl-L-valyl>-2S,5S-diamino-1,6-diphenyl-hexan-3R,4R-diol (aus "Diastereomer 2")

Synthese analog Beispiel 105
MS (FAB): 1043 $(M+Na)^+$, 1021 $(M+H)^+$, 921, 821

**Beispiel 107**
N,N′-Bis-<O-(D-mannofuranosyl)-2S-hydroxy-3-phenyl-propionyl-L-valyl>-2S,5S-diamino-1,6-diphenyl-hexan-3R,4R-diol

20 mg der Verbindung aus Beispiel 108 wurde für 30 min bei RT mit methanolischer Salzsäure gerührt. Die flüchtigen Bestandteile wurden i. Vac. abdestilliert, der Rückstand mit Diethylether digeriert, abgesaugt und getrocknet.
Ausbeute: 13 mg
NMR (270 MHz, DMSO <$D_6$>): 0,58 (d, 6Hz, 6H); 0,62 (d, 6Hz, 6H); 1,82 (m, 2H); 2,60 (dd, 4Hz, 14Hz, 2H); 2,71-2,82 (m, 4H); 2,98 (dd, 14Hz, 3Hz, 2H); ca. 3,25 (m, 2H); 3,30-3,49 (m, 6H); 3,58 (m, 2H); 3,67 (dd, 11Hz, 3Hz, 2H); ca 3,70-4,30 (m, ca 16H); 4,43 (m, 2H); 4,49 (d, 2Hz, 2H); 7,05-7,29 (m, 20H); 7,35 (d, 9Hz, 2H); 7,67 (d, 9Hz, 2H)

**Beispiel 107a**
N,N′-Bis-<O-(2,3-5,6-diisopropyliden-D-mannofuranosyl)-2S-hydroxy-3-phenyl-propionyl-L-valyl>-2S,5S-diamino-1,6-diphenyl-hexan-3R,4R-diol

57 mg N,N′-Bis-<L-valyl>-2S,5S-diamino-1,6-diphenyl-hexan-3R,4R-diol-dihydrochlorid wurden mit 90 mg O-(2,3-5,6-diisopropyliden-D-mannofuranosyl)-2S-hydroxy-3-phenylpropionsäure in 1 ml DMF gelöst und mit der TBTU-Methode gekuppelt. Ausbeute: 60 mg
MS (FAB): 1279 $(M+H)^+$, 1261, 1221
NMR (270 MHz, DMSO <$D_6$>): 0,63 (d, 6Hz, 6H); 0,69 (d, 6HZ, 6H); 1,19 (s, 6H); 1,21 (s, 6H); 1,30 (s, 12H); 1,79 (m, 2H); 2,60-2,82 (m, 8H); 3,29 (m, 2H); 3,73 (dd, 8Hz, 6Hz, 2H); 3,85-3,98 (m, 4H); 4,01-4,18 (m, 4H); 4,23 (dd, 8Hz, 3Hz, 2H); 4,40 (d, 6Hz, 2H); 4,45 (m, 2H); 4,62-4,72 (m, 4H); 7,03-7,32 (m, ca 22H); 7,40 (d, 9Hz, 2H); 7,59 (d, 9Hz, 2H)
O-(2,3-5,6-Diisopropyliden-D-mannofuranosyl)-2S-hydroxy-3-phenyl-propionsäure wurde mit der Imidatmethode nach R.R. Schmidt aus 2,3-5,6-Diisopropyliden-D-mannofuranose und 2S-Hydroxy-3-phenyl-propionsäure dargestellt (R.R. Schmidt und I. Michel Angew.Chem. 92,763 (1980); Angew. Chem. Int. Ed. Engl. 19, 731 (1980)).
405 mg O-(2,3-5,6-Diisopropyliden-D-mannofuranosyl-trichloracetimidat wurden zusammen mit 194 mg Phenylmilchsäureethylester in 15 ml abs $CH_2Cl_2$ gelöst. Man kühlte auf 0 °C und setzte 100 µl einer 1M $BF_3$-Etheratlösung in $CH_2Cl_2$ zu. Die Lösung rührte 1h bei 0 °C, wurde in 100 ml $NaHCO_3$-Lösung gegossen und mit $CH_2Cl_2$ extrahiert. Die org. Phase wurde mit $Na_2SO_4$ getrocknet und eingeengt. Nach Chromatographie mit Kieselgel
(Laufmittel: Methyltert.-butylether/Heptan (1/1)) erhielt man 195 mg Produkt.

**Beispiel 108**
N,N′-Bis-(L-phenylalanyl-L-valyl)-3S,6S-diamino-1,8-di-(4-pyridyl)-octan-4R,5R-diol-tetrahydrochlorid

Synthese analog zu Beispiel 16 aus 27
MS (FAB): 823 $(M+H)^+$

**Beispiel 109**

N,N'-Bis-<N-(β-D-1-desoxyfructos-1-yl)-L-phenylalanyl-L-valyl>-2S,5S-diamino-1,6-diphenyl-hexan-3S,4S-diol-diacetat

69 mg N,N'-Bis-<L-phenylalanyl-L->valyl>-2S,5S-diamino-1,6-diphenyl-hexan-3S,4S-diol-dihydrochlorid wurden mit 79 mg D-Glucose in 6 ml MeOH und 2ml Pyridin suspendiert und 4,5 h gekocht. Das Lösungsmittel wurde i. Vac. entfernt, der Rückstand durch Chromatographie über ®Sephadex LH20 mit 10 % wässriger Essigsäure getrennt.
Ausbeute: 71 mg
MS (FAB): 1139 (M + Na)$^+$, 1117 (M + H)$^+$

**Beispiel 110**
N,N'-Bis-<D-gluconyl-L-phenylalanyl-L-valyl>-2S,5S-diamino-1,6-diphenyl-hexan-3R,4R-diol

Synthese Behandlung der Verbindung aus Beispiel 111 mit Ammoniak-gesättigtem Methanol.
MS (FAB): 1171 (M + Na)$^+$

**Beispiel 111**
N,N'-Bis-<2,3,4,5,6-penta-O-acetyl-D-gluconyl-L-phenylalanyl-L-valyl>-2S,5S-diamino-1,6-diphenyl-hexan-3R,4R-diol

Synthese: durch Kupplung von 2,3,4,5,6-Penta-O-acetyl-D-gluconsäure (C.E. Braun und C.D. Cook, Organic Synthesis, Vol. 5, 1973, 887-889) auf N,N'-Bis-<L-phenylalanyl-L-valyl>-2S,5S-diamino-1,6-diphenyl-hexan-3R,4R-diol-dihydrochlorid mit der TBTU-Methode.
MS (FAB): 1569 (M + H)$^+$

**Beispiel 112**
N,N'-Bis-<tert.-butoxycarbonyl-L-phenylalanyl-L-valyl>-1,4-diamino-butan-2R,3R-diol

Synthese analog zu Beispiel 6 aus 1,4-Diamino-butan-2R,3R-diol-dihydrochlorid
NMR (270 MHz, DMSO <$D_6$>): 0,83 (d, 6Hz, 12H); 1,31 (s, 18H); 1,93 (m, 2H); 2,73 (m, 2H); 2,91-3,07 (m, 4H); 3,28 (m, 2H); 3,42 (m, 2H); 4,18 (m, 4H); 4,57 (m, 2H); 7,02 (d, 8Hz, 2H); 7,13-7,32 (m, 10H); 7,66 (d, 8,4Hz, 2H); 8,04 (m, 2H)
MS (FAB): 835 (M + Na)$^+$, 813 (M + H)$^+$, 713, 613

**Beispiel 112a**

1,4-Diamino-butan-2R,3R-diol-dihydrochlorid
Synthese aus (+)-1,4-Di-O-tosyl-2,3-O-isopropyliden-D-threitol analog Beispiel 2, 2b und 2c
NMR (60 MHZ, DMSO <$D_6$>): 2,9 (m, 4H); 3,73 (m, 2H); ca. 5,7-4,5 (br, ca. 2H); 8,1 (m, ca. 6H)
MS (DCI): 121 (M + H)$^+$, 104

**Beispiel 113**
N,N'-Bis-<L-phenylalanyl-L-valyl>-1,4-diamino-butan-2R,3R-diol-dihydrochlorid

Synthese analog zu Beispiel 16 aus 112
MS (FAB): 635 (M + Na)$^+$, 613 (M + H)$^+$

**Beispiel 114**
N,N'-Bis-<tri-benzyloxycarbonyl-L-arginyl-L-valyl>-2S,5S-diamino-1,6-diphenyl-hexan-3R,4R-diol

Synthese analog Beispiel 6
MS (FAB): 1637 (M + Na)$^+$, 1615 (M + H)$^+$
NMR (270 MHz, DMSO<$D_6$>): 0,71 (d, 7Hz, 12H); 1,57 (m, 8H); 1,80 (m, 2H); 2,73 (m, 2H); 2,94 (m, 2H); 3,30 (m, 2H); 3,70-4,12 (m, 10H); 4,58 (d, 7Hz, 2H); 4,92-5,18 (m, 8H); 5,19 (s, 4H); 7,00-7,42 (m, 40H); 7,49 (d, 8Hz, 4H); 7,64 (d, 8,4Hz, 2H); 9,13 (br.s, 4H)

**Beispiel 115**
N,N'-Bis-<tert.-butyloxycarbonyl-L-cyclohexylalanyl-L-valyl>-2S,5S-diamino-1,6-diphenyl-hexan-3R,4R-diol

Synthese analog Beispiel 6
MS (FAB): 1005 $(M+H)^+$, 905
NMR (270 MHz, DMSO<$D_6$>): 0,67 (d, 7Hz, 6H); 0,80 (d, 7Hz, 6H); 0,80-1,84 (m, 26H); 1,42 (s, 18H); 2,13 (sept., 7Hz, 2H); 2,80 (dd, 15Hz, 9Hz, 2H); 3,35 (m, 4H); 4,03 (m, 4H); 4,30 (qd, 9Hz, 4Hz, 2H); 4,96 (d, 4Hz, 2H); 6,57 (d, 8Hz, 4H); 7,10-7,30 (m, 12H)

**Beispiel 116**
N,N′-Bis-<L-cyclohexylalanyl-L-valyl>-2S,5S-diamino-1,6-diphenyl-hexan-3R,4R-diol-dihydrochlorid

Synthese analog Beispiel 16
MS (FAB): 805 $(M+H)^+$, 553, 531
NMR (270 MHz, DMSO<$D_6$>): 0,79 (d, 7Hz, 6H); 0,85 (d, 7Hz, 6H); 1,00-1,95 (m, 28H); 2,77 (dd, 14Hz, 7Hz, 2H); 2,93 (m, 2H); 3,37 (m, 2H); 3,89 (m, 2H); 4,09 (m, 4H); 4,70 (d, 7Hz, 2H); 7,16 (m, 10H); 7,66 (d, 8Hz, 2H); 8,17 (s, 6H); 8,47 (d, 9Hz, 2H)

**Beispiel 117**
N,N′-Bis-<benzyloxycarbonyl-L-tryptophyl-L-valyl>-2S,5S-diamino-1,6-diphenyl-hexan-3R,4R-diol

Synthese analog Beispiel 6
MS (FAB): 1139 $(M+H)^+$, 720
NMR (270 MHz, DMSO<$D_6$>): 0,75 (m, 12H); 1,96 (m, 2H); 2,76 (dd, 13Hz, 7Hz, 2H); 2,90-3,13 (m, 6H); 3,40 (m, 2H); 4,07 (m, 4H); 4,38 (m, 2H); 4,65 (d, 7Hz, 2H); 4,88 (d, 14Hz, 2H); 4,97 (d, 14Hz, 2H); 6,90-7,35 (m, 28H); 7,47 (d, 8Hz, 2H); 7,58 (d, 8Hz, 2H); 7,65 (d, 8Hz, 2H); 7,83 (d, 8Hz, 2H); 10,80 (s, 2H)

**Beispiel 118**
N,N′-Bis-<L-tryptophyl-L-valyl>-2S,5S-diamino-1,6-diphenyl-hexan-3R,4R-diol-dihydrochlorid

Synthese analog Beispiel 11
MS (FAB): 871 $(M+H)^+$
NMR (270 MHz, DMSO<$D_6$>): 0,75 (m, 12H); 1,88 (m, 2H); 2,75 (m, 4H); 2,98 (dd, 14Hz, 2Hz, 2H); 3,13 (dd, 14Hz, 3Hz,2H); 3,42 (m, 2H); 3,73 (m, 2H); 4,10 (m, 4H); 4,73 (d, 6Hz, 2H); 6,09-7,24 (m,18H); 7,35 (d, 8Hz, 2H); 7,63 (d, 8Hz, 2H); 7,80 (d, 8Hz, 2H); 8,22 (s, 6H); 10,90 (s, 2H)

**Beispiel 119**
N,N′-Bis-<benzyloxycarbonyl-L-1,2,3,4-tetrahydro-isochinolin-3-yl-carbonyl-L-valyl>-2S,5S-diamino-1,6-diphenyl-hexan-3R,4R-diol

Synthese analog Beispiel 6
MS (FAB): 1107 $(M+Na)^+$, 1085 $(M+H)^+$
NMR (270 MHz, DMSO<$D_6$>): 0,55 (m, 12H); 1,70 (m, 2H); 2,60-3,81 (m, 10H); 3,90 (m, 2H); 4,03 (m, 2H); 4,38-4,80 (m, 8H); 4,91-5,20 (m, 4H); 7,00-7,53 (m, 28H); 7,58 (d, 8Hz, 2H); 7,72 (d, 8Hz, 2H)

**Beispiel 120**
N,N′-Bis-<L-1,2,3,4-tetrahydro-isochinolin-3-yl-carbonyl-L-valyl>-2S,5S-diamino-1,6-diphenyl-hexan-3R,4R-diol-diacetat

Synthese analog Beispiel 11
MS (FAB): 839 $(M+Na)^+$, 817 $(M+H)^+$.
NMR (270 MHz, DMSO<$D_6$>): 0,70 (d, 7Hz, 12H); 1,86 (m, 2H); 1,92 (s, 6H); 2,64-2,89 (m, 4H); 2,92 (dd, 16Hz, 5Hz, 2H); 3,02 (dd, 13Hz, 3Hz, 2H); 3,39 (m, 2H); 3,47 (dd, 9Hz, 5Hz, 2H); 3,90 (s, 4H); 4,03 (m, 2H); 4,10 (dd, 9Hz, 5Hz, 2H); 4,74 (br.s, 2H); 7,02-7,26 (m, 18H); 7,77 (d,9Hz, 2H); 7,85 (d, 8Hz, 2H)

**Beispiel 121**
N,N′-Bis-<(2-(benzyl-sulfinyl-methyl)-3-phenyl-propionyl)-L-valyl>-2S,5S-diamino-1,6-diphenyl-hexan-3R,4R-diol

Synthese analog Beispiel 13

Die Synthese des Bausteins (2-(Benzyl-sulfinyl-methyl)-3-phenyl-propionsäure erfolgte analog Literatur: J. Med. Chem. 31, 1839, (1988).

MS (FAB): 1089 (M+Na)$^+$, 1067 (M+H)$^+$, 710

NMR (270 MHz, DMSO<$D_6$>): 0,45 (m, 6H); 0,72 (m, 6H); 1,80 (m, 2H); 2,53-2,95 (m, 12H); 3,22-3,36 (m, 4H); 3,55 (m, 2H); 3,73-4,26 (m, 6H); 4,48 (m, 2H); 7,00-7,40 (m, 30H); 7,85 -8,07 (m, 4H)

**Beispiel 122**

N,N′-Bis-<(2-(p-chlorbenzyl-thio-methyl)-3-phenyl-propionyl)-L-valyl>-2S,5S-diamino-1,6-diphenyl-hexan-3R,4R-diol

Synthese analog Beispiel 13

Die Synthese des Bausteins (2-(p-Chlorbenzyl-thio-methyl)-3-phenyl-propionsäure erfolgte analog Literatur: J. Med. Chem. 31, 1839, (1988).

MS (FAB): 1125 (M+Na)$^+$

NMR (270 MHz, DMSO<$D_6$>): 0,49 (m, 6H); 0,57 (m, 6H); 1,80 (m, 2H); 2,10-2,33 (m, 2H); 2,38-2,60 (m, 4H); 2,62-2,83 (m, 6H); 2,95 (m, 2H); 3,28 (m, 2H); 3,65 (s, 4H); 4,03-4,17 (m, 2H); 4,45 (m, 2H); 4,54-4,67 (m, 2H); 7,00-7,50 (m, 28H); 7,64 (m, 2H); 7,88 (m, 2H)

**Beispiel 123**

N,N′-Bis-<(2-(p-chlorbenzyl-sulfonyl-methyl)-3-phenylpropionyl)-L-valyl>-2S,5S-diamino-1,6-diphenyl-hexan-3R,4R-diol

Synthese analog Beispiel 13

Die Synthese des Bausteins 2-(p-Chlorbenzyl-sulfonyl-methyl)-3-phenyl-propionsäure erfolgte analog Literatur: J. Med. Chem. 31, 1839, (1988).

MS (FAB): 1191 (M+2H+Na)$^+$ 1189 (M+Na)$^+$

NMR (270 MHz, DMSO<$D_6$>): 0,52 (m, 6H); 0,74 (m, 6H); 1,83 (m, 2H); 2,42-2,95 (m, 10H); 3,28-3,54 (m, 6H); 3,90-4,70 (m, 10H); 6,98-7,47 (m,30H); 8,03 (m, 2H)

**Beispiel 124**

N,N′-Bis-<N-tosyl(-β-naphthyl-alanyl-L-valyl>-2S,5S-diamino-1,6-diphenyl-hexan-3R,4R-diol

Synthese analog Beispiel 6

MS (FAB): 1223 (M+Na)$^+$

NMR (270 MHz, DMSO<$D_6$>): 0,66 (m, 12H); 1,80 (m, 2H); 2,13 (s, 6H); 2,50-2,90 (m, 8H); 3,30 (m, 2H); 3,98-4,67 (m, 8H); 6,70-8,00 (m, 38H)

**Beispiel 125**

N,N′-Bis-<N-mesyl-β-naphthyl-alanyl-L-valyl>-2S,5S-diamino-1,6-diphenyl-hexan-3R,4R-diol

Synthese analog Beispiel 6

MS (FAB): 1072 (M+Na)$^+$, 838

NMR (270 MHz, DMSO<$D_6$>): 0,74 (m, 12H); 1,82 (s, 6H); 1,87 (m, 2H); 2,55-3,08 (m, 8H); 3,25 (m, 2H); 4,02 (m, 2H); 4,22 (m, 2H); 4,47 (m, 2H); 4,70 (m, 2H); 7,00-8,00 (m, 30H)

**Beispiel 126**

N-<(2R-(1,1-Dimethylethyl-sulfonylmethyl)-3-phenylpropionyl)-L-valyl>-N′-<(2S-(1,1-dimethylethylsulfonylmethyl)-3-phenyl-propionyl)-L-valyl>-2S,5S-diamino-1,6-diphenyl-hexan-3R,4R-diol

Nebenprodukt aus der Synthese von Beispiel 52

Beispiel 126 Rf = 0,17 (EE)

Beispiel 52 Rf = 0,35 (EE)

MS (FAB): 1053 (M+Na)$^+$

NMR (270 MHz, DMSO<$D_6$>: 0,47 (d, 7Hz, 3H); 0,48 (d, 7Hz, 3H); 0,70 (d, 7Hz, 3H); 0,75 (d, 7Hz, 3H); 1,14 (s, 9H); 1,27 (s, 9H); 1,82 (m, 2H); 2,60-3,00 (m, ca. 10H); 3,08-3,35 (m, ca. 3H); 3,38-3,58 (m, 3H); 3,91 (dd, 8Hz, 6Hz, 1H); 4,06 (m, 1H); 4,27 (d, 5Hz, 1H); 4,35 4,54 (m, 3H); 7,00-7,38 (m, 22H); 7,93 (d, 8Hz, 2H); 8,04 (d, 8Hz, 2H)

Die nachfolgenden Verbindungen der Beispiele 127-134 wurden analog der Synthesen gemäß den Beispielen 6 bzw. 16 erhalten.

**Beispiel 127**
N,N'-Bis-<tert.-butoxycarbonyl-L-valyl>-2R,5R-diamino-1,6-diphenyl-hexan-3R,4R-diol

MS (FAB): 699 $(M+H)^+$, 599, 499

**Beispiel 128**
N,N'-Bis-<tert.-butoxycarbonyl-L-valyl>-2S,5S-diamino-1,6-dicyclohexyl-hexan-3S,4S-diol

MS (FAB/LiI): 717 $(M+Li)^+$

**Beispiel 129**
N,N'-Bis-<tert.-butoxycarbonyl-L-cyclohexylglycin>-2S,5S-diamino-1,6-diphenyl-hexan-3R,4R-diol

MS (FAB): 801 $(M+Na)^+$, 779 $(M+H)^+$, 679

**Beispiel 130**
N,N'-Bis-<tert.-butoxycarbonyl-L-asparaginyl>-2S,5S-diamino-1,6-diphenyl-hexan-3R,4R-diol

MS (FAB): 729 $(M+H)^+$, 629

**Beispiel 131**
N,N'-Bis-<L-valyl>-2S,5S-diamino-1,6-dicyclohexyl-hexan-3S,4S-diol-dihydrochlorid

**Beispiel 132**
N,N'-Bis-<$N^6$-benzoxycarbonyl-L-lysyl>-2S,5S-diamino-1,6-diphenyl-hexan-3R,4R-diol-dihydrochlorid

**Beispiel 133**
N,N'-Bis-<glycyl>-2S,5S-diamino-1,6-diphenyl-hexan-3S,4S-diol-dihydrochlorid

MS (FAB): 415 $(M+H)^+$.

**Beispiel 134**
N,N'-Bis-<tert.-butoxycarbonyl-glycyl>-2S,5S-diamino-1,6-diphenyl-hexan-3S,4S-diol

MS (FAB): 615 (M+H).

Die nachfolgenden Verbindungen der Beispiele 135-140 wurden analog der Synthesen gemäß den Beispielen 23 bzw. 24 erhalten.

**Beispiel 135**
Bis-<N-(($N^2$-tert.-butoxycarbonyl-L-lysyl)-L-leucyl)-2S-amino-3-phenyl-propyl>-amin-trihydrochlorid

MS (FAB): 966 $(M+H)^+$

**Beispiel 136**
Bis-<N-(tert.-butoxycarbonyl-2S-amino-3-cyclohexyl-propyl>-amin-hydrochlorid

MS (FAB): 496 $(M+H)^+$

**Beispiel 137**
Bis-<N-(L-leucyl)-2S-amino-3-phenyl-propyl>-amintrihydrochlorid

MS (FAB): 510 $(M+H)^+$

**Beispiel 138**
Bis-<N-(tert.-butoxycarbonyl-L-leucyl)-2S-amino-3-phenylpropyl>-amin

MS (FAB): 710 $(M+H)^+$

**Beispiel 139**
Bis-<2S-amino-3-phenyl-propyl>-amin-trihydrochlorid

MS (FAB): 284 $(M+H)^+$

**Beispiel 140**
Bis-<N-(benzyloxycarbonyl-L-valyl)-2S-amino-3-phenyl-propyl>-amin

MS (FAB): 750 $(M+H)^+$

**Beispiel 141**
Bis-<N-tert.-butoxycarbonyl-2S-amino-3-methyl-butyl>-amin-hydrochlorid

Synthese analog Beispiel 25
MS (FAB): 388 $(M+H)^+$

**Beispiel 142**
N,N′-Bis-<(2S-(1,1-dimethylethyl-sulfonylmethyl)-3-(1-naphthyl)-propionyl)-L-valyl>-3S,6S-diamino-1,8-di-(4-pyridyl)-octan-4R,5R-diol

Synthese analog zu Beispiel 13 aus 27a
NMR (270 MHz, DMSO <$D_6$>): 0,83 (m, 12H); 1,14 (s, 18H); 1,66 (m, 2H); 1,82 (m, 2H); 2,00 (m, 2H); 2,50-2,78 (m, 4H); 2,86 (m, 2H); 3,06-3,63 (m, 10H); 4,02 (m, 2H); 4,14 (m, 2H); 4,69 (m, 2H); 7,30-7,60 (m, 14H); 7,74 (d, 8Hz, 2H); 7,87 (m, 2H); 8,16 (m, 2H); 8,32 (d, 8Hz, 2H); 8,58 (m, 4H)
MS (FAB): 1161 $(M+H)^+$

**Beispiel 143**
N,N′-Bis-<(2S-(1,1-dimethylethyl-sulfonylmethyl)-3-(1-naphthyl)-propionyl)-L-valyl>-1,4-diamino-butan-2R,3R-diol

Synthese analog zu Beispiel 13 aus 112a
NMR (270 MHZ, DMSO <$D_6$>): 0,82 (d, 6Hz, 12H); 1,17 (s, 18H); 1,92 (m, 2H); 2,92-3,08 (m, 4H); 3,16-3,53 (m, 10H); 3,53 (dd, 12,8Hz, 8,8Hz, 2H); 4,11 (dd, 8,0Hz, 7,2Hz, 2H); 4,55 (d, 4,8Hz, 2H); 7,38-7,67 (m, 10H); 7,80 (m, 2H); 7,92 (m, 2H); 8,12 (d, 8,4Hz, 2H); 8,20 (d, 8Hz, 2H)
MS (FAB): 973 $(M+Na)^+$ ; 951 $(M+H)^+$

**Beispiel 144**
N,N′-Bis-<tert.-butoxycarbonyl-L-phenylalanyl-L-valyl>-1,4-diamino-butan

Synthese analog zu Beispiel 6
NMR (270 MHZ, DMSO <$D_6$>): 0,83 (d, 6Hz, 12H); 1,28 (s, 18H); 1,39 (m, 4H); 1,91 (m, 2H); 2,74 (dd, 12,8Hz, 9,6HZ, 2H); 2,89-3,16 (m, 6H); 4,08-4,23 (m, 4H); 7,02 (d, 8Hz, 2H); 7,14-7,30 (m, 10H); 7,63 (d, 8,4Hz, 2H); 7,95 (m, 2H)
MS (FAB): 781 $(M+H)^+$, 681, 581

**Beispiel 145**
N,N′-Bis-<L-phenylalanyl-L-valyl>-1,4-diamino-butandihydrochlorid

Synthese analog zu Beispiel 16 aus 144
MS (FAB): 581 $(M+H)^+$

**Beispiel 146**
N,N′-Bis-<(2S-(1,1-dimethylethyl-sulfonylmethyl)-3-(1-naphthyl)-propionyl)-L-valyl>-1,4-diamino-butan

Synthese analog zu Beispiel 13
NMR (270 MHz, DMSO <$D_6$>): 0,82 (d, 6Hz, 12H); 1,19 (s, 18H); 1,32 (m, 4H); 1,89 (m, 2H); 2,98 (m, 4H); 3,32 (m, 2H); 3,42 (m, 6H); 3,54 (dd, 12,8Hz, 8Hz, 2H); 4,04 (t, J=8Hz, 2H); 7,38 (m, 4H); 7,53 (m, 6H); 7,79 (m, 2H); 7,92 (m, 2H); 8,08 (d, 8Hz, 2H); 8,21 (m, 2H)
MS (FAB): 941 $(M+Na)^+$, 919 $(M+H)^+$

**Beispiel 147**

N,N'-Bis-(tert.butoxycarbonyl-L-phenylalanyl-L-valyl)-3S,6S-diamino-1,8-diphenyl-octan-4R,5R-diol

Synthese analog Beispiel 6 aus 3S,6S-Diamino-1,8-diphenyl-octan-4R,5R-diol-dihydrochlorid (Letztere Verbindung wurde analog Beispiel 2, 2b, 2c und 2e aus 1,2R-5R,6-Diepoxy-3,4-O-isopropyliden-3R,4R-diol und Benzyllithium dargestellt)

MS (FAB(LiI)): 1027 (M + Li)$^+$, 927, 827

NMR (270 MHz, DMSO<D6>) 0,88 (m,12H); 1,28 (s, 18H); 1,57-1,86 (m, 4H); 2.01 (m, 2H); ca 2,4-2,6 (m, ca 4H); 2,75 (dd, 11Hz, 14Hz, 2H); 2,98 (dd, 14 Hz, 4Hz, 2H); ca 3,32 (m, ca 2H); 4,06-4,26 (m, 4H); 4,32 (dd; 6Hz, 8Hz, 2H); 4,62 (m, 2H); 7,0 (d, 8Hz, 2H); 7,10-7,32 (m, 20H); 7,62 (d, 10Hz, 2H); 7,75 (d, 8Hz, 2H);

**Beispiel 148**

N,N'-Bis-(L-phenylalanyl-L-valyl)-3S,6S-diamino-1,8-diphenyl-octan-4R,5R-diol-dihydrochlorid

Synthese analog zu Beispiel 16 aus 147

MS (FAB): 821 (M + H) +, 843 (M + Na) +, 803

**Beispiel 149**

N,N'-Bis-(<2S-(1,1 dimethylethyl-sulfonylmethyl)-3-(1-naphthyl)-propionyl>-L-valyl)-3S,6S-diamino-1,8-diphenyl-octan-4R,5R-diol

Synthese analog zu Beispielen 13 und 147

MS (FAB(LiI)): 1165 (M + Li) +

NMR (270 MHz, DMSO<D6>): 0,92 (d, 7Hz, 12H); 1,13 (s, 18H); 1,6-1,85 (m, 4H); 2,04 (m, 2H); 2,40-2,64 (m, 4H); 2,82 (dm, 14Hz, 2H); 3,18 (m, 2H); 3,32-3,52 (m, 6H); 3,58(m, 2H); 4,08 (m, 2H); 4,22 (t, 8Hz, 2H); 7,1-7,56 (m, 20H); 7,72 (dd, 4Hz, 2H); 7,88 (m, 2H); 8,14 (m, 2H); 8,32 (d, 8Hz, 2H)

**Beispiel 150**

N,N'-Bis-(tert.butoxycarbonyl-L-phenylalanyl-L-valyl)-6S,9S-diamino-tetradecan-7R,8R-diol

Synthese analog Beispiel 6 aus 6S,9S-Diamino-tetradecan-7R,8R-diol-dihydrochlorid (Letztere Verbindung wurde analog Beispiel 2, 2b, 2c und 2e aus 1,2R-5R,6-Diepoxy-3,4-O-isopropyliden-3R,4R-diol und n-Butyllithium dargestellt)

MS (FAB(LiI)): 959 (M + Li)$^+$

NMR (270 MHz, DMSO<D6>): 0,76-0,91 (m, 18H); 1,12-1,54 (m, 16H); 1,28 (s, 18H); 1,98 (m, 2H); 2,74 (dd, 12Hz, 14Hz, 2H); 2,87 (dd, 14Hz, 4Hz, 2H); 3,22 (m, 2H); 3,98 (m, 2H); 4,14-4,32 (m, 4H); 4,46 (s, 2H); 7,0 (d, 8Hz, 2H); 7,14-7,31 (d, 4Hz, 10H); 7,38 (d, 9Hz, 2H); 7,70 (d, 9Hz, 2H)

**Beispiel 151**

N,N'-Bis-(L-phenylalanyl-L-valyl)-6S,9S-diamino-tetradecan-7R,8R-diol-dihydrochlorid

Synthese analog zu Beispiel 16 aus 150

MS (FAB): 753 (M + H) +, 775 (M + Na) +, 735

**Beispiel 152**

N,N'-Bis-(<2S-(1,1 dimethylethyl-sulfonylmethyl)-3-(1-naphthyl)-propionyl>-L-valyl)-6S,9S-diamino-tetradecan-7R,8R-diol

Synthese analog zu Beispiel 13 und 150

MS (FAB(LiI)): 1097 (M + Li)$^+$

NMR (270 MHz, DMSO<D6>) 0,76 (m, 6H); 0,88 (d, 7Hz, 12H); 1,12 (s, 18H); ca 1,10-1,54 (m, 16H); 2,02 (m, 2H); 2,82 (dd, 12Hz, 2Hz, 2H); 3,16 (dd, 12Hz, 16Hz, 2H); 3,24 (m, 2H); 3,36-3,52 (m, 4H); 3,58 (dd, 8Hz, 13Hz, 2H); 3,98 (m, 2H); 4,16 (t, 6Hz, 2H); 4,44 (s, 2H); 7,18 (d, 10Hz, 2H); 7,42-7,48 (m, 4H); 7,49-7,62 (m, 4H); 7,81 (m, 2H); 7,92 (m, 2H); 8,20 (d, 8HZ, 2H); 8,30 (d, 8,4Hz, 2H)

**Beispiel 153**

N,N'-Bis-(tert.-butoxycarbonyl-L-phenylalanyl-L-valyl)-2S,5S-diamino-1,6-bis-(3,4-methylendioxyphenyl)-hexan-3R,4R-diol

Synthese analog Beispiel 6 aus 2S,5S-Diamino-1,6-bis-(3,4-methylendioxyphenyl)-hexan-3R,4R-diol-dihydrochlorid (Letztere Verbindung wurde analog Beispiel 2, 2b, 2c und 2e aus 1,2R-5R,6-Diepoxy-3,4-O-isopropyliden-3R,4R-diol und 3,4-Methylendioxyphenyllithium dargestellt)

MS (FAB): 1103 $(M+Na)^+$,1081 $(M+H)^+$

NMR (270 MHz, DMSO<D6> :0,73 (d, 6Hz, 6H); 0,76 (d, 6HZ, 6H); 1,28 (s, 18H); 1,87 (m, 2H); 2,52-2,78 (m, 6H); 2,91 (dd, 14Hz, 4Hz, 2H); 3,26 (m, 2H); 4,11-4,22 (m, 4H); 4,35 (m, 2H); 4,66 (m, 2H); 5,84 (s, 2H); 5,86 (s, 2H); 6,63 (d, 8Hz, 2H); 6,69 (d, 8Hz, 2H); 6,75 (s, 2H); 6,99 (d, 9Hz, 2H); 7,13-7,33 (m, 10H); 7,45 (d, 9Hz, 2H); 7,59 (d, 9Hz, 2H)

**Beispiel 154**

N,N'-Bis-(L-phenylalanyl-L-valyl)-2S,5S-diamino-1,6-bis-(3,4-methylendioxyphenyl)-hexan-3R,4R-diol-dihydrochlorid

Synthese analog zu Beispiel 16 aus 153

MS (FAB): 881 $(M+H)^+$,863

**Beispiel 155**

N,N'-Bis-(<2S-(1,1-dimethylethyl-sulfonylmethyl)-3-(1-naphthyl)-propionyl>-L-valyl)-2S,5S-diamino-1,6-bis-(3,4-methylendioxyphenyl)-hexan-3R,4R-diol

Synthese analog zu Beispielen 13 und 153

MS (FAB): 1241 $(M+Na)^+$,1219 $(M+H)^+$

NMR (270 MHz, DMSO<D6>):0,73 (d, 7HZ, 6H); 0,78 (d, 7Hz, 6H); 1,10 (s, 18H); 1,89 (m, 2H); 2,55-2,72 (m, 4H); 2,79 (dm 14Hz, 2H); 3,08 (dd, 14Hz, 10Hz, 2H); ca 3,22-3,43 (m, ca 6H); 3,58 (dd, 14Hz, 10Hz, 2H); 4,07 (m, 2H); 4,45 (m, 2H); 4,49 (m, 2H); 5,75 (s, 2H); 5,78 (s, 2H); 6,68 (s, 2H); 6,80 (s, 2H); 7,25 (d, 9HZ, 2H); 7,39-7,45 (m, 4H); 7,54 (m, 6H); 7,80 (m, 2H); 7,92 (m, 2H); 8,15-8,25 (m, 4H)

**Beispiel 156**

N,N'-Bis-(<2S-(1,1-dimethylethyl-sulfonylmethyl)-3-(1-naphthyl)-propionyl>-L-isoleucyl)-2S,5S-diamino-1,6-diphenyl-hexan-3R,4R-diol

Synthese analog Beispiel 13

MS (FAB): 1181 $(M+Na)^+$

NMR (270 MHz, DMSO<D6>):0,63 (d, 7Hz, 6H); 0,73 (t, 7Hz, 6H); 0,99 (m, 2H); 1,11 (s, 18H); 1,32 (m, 2H); 1,64 (m, 2H); 2,63-2,88 (m, 6H); 3,07 (dd, 15Hz, 11Hz, 2H); ca 3,28-3,43 (m, ca 6H); 3,58 (dd, 14Hz, 9Hz, 2H); 4,09 (t, 8Hz, 2H); 4,48-4,62 (m, 4H); 7,03 (m, 2H); 7,12-7,31 (m, 10H); 7,43 (m, 4H); 7,54 (m, 4H); 7,81 (m, 2H); 7,92 (m, 2H); 8,15-8,25 (m, 4H)

**Beispiel 157**

N,N'-Bis-($N^2$-<hexadecyl-sulfonyl>L-lysyl-L-valyl)-2S,5S-diamino-1,6-diphenyl-hexan-3R,4R-diol

Synthese analog Beispielen 11 und 58

MS (FAB): 1330 $(M+H)^+$

**Beispiel 158**

N,N'-Bis-($N^2$-<tetradecanoyl>L-lysyl-L-valyl)-2S,5S-diamino-1,6-diphenyl-hexan-3R,4R-diol

Synthese analog Beispielen 11 und 58

MS (FAB): 1174 $(M+H)^+$

**Beispiel 159**

N,N'-Bis-(tert.-butoxycarbonyl-L-phenylalanyl-L-asparaginyl)-2S,5S-diamino-1,6-diphenyl-hexan-3R,4R-diol

Synthese analog Beispiel 6

MS (FAB): 1045$(M+Na)^+$

NMR (270MHz, DMSO<D6>): 1,27 (s, 18H); 2,20-2,78 (m, 10H); 2,90 (m, 2H); 3,30 (m, 2H); 4,14 (m,

2H); 4,28 (m, 2H); 4,45 (m, 2H); 4,64 (s, 2H); 6,88 (s, 4H); 7,02-7,37 (m, 24H); 8,04 (d, 8Hz, 2H)

**Beispiel 160**

N,N′-Bis-(L-phenylalanyl-L-asparaginyl)-2S,5S-diamino-1,6-diphenyl-hexan-3R,4R-diol

Synthese analog Beispiel 16 aus 159
MS(FAB): 823 (M+H)$^+$

**Beispiel 161**

N,N′-Bis-(<2S-(1,1-dimethylethyl-sulfonylmethyl)-3-(1-naphthyl)-propionyl>L-asparaginyl)-2S,5S-diamino-1,6-diphenyl-hexan-3R,4R-diol

Synthese analog Beispiel 13
MS (FAB): 1183 (M+Na)$^+$
NMR (270MHz, DMSO<D6>): 1,17 (s, 18H); 2,22 (m, 2H); 2,37-2,76 (m, 10H); 2,90 (m, 2H); 3,25 (m, 4H); 3,58 (m, 2H); 4,25 (m, 2H); 4,40 (m, 2H); 4,62 (m, 2H); 6,93-7,60 (m, 24H); 7,77 (m, 2H); 7,90 (m, 2H); 8,22 (d, 8Hz, 2H); 8,33 (d, 8Hz, 2H);

**Beispiel 162**

N,N′-Bis-(<2-(1,1-dimethylethyl-sulfonylmethyl)-3-(4-pyridyl)-propionyl>L-valyl)-2S,5S-diamino-1,6-diphenyl-hexan-3R,4R-diol-dihydrochlorid

Synthese analog Beispiel 13.
2-(1,1-Dimethylethyl-sulfonylmethyl)-3-(4-pyridyl)-propionsäure wurde als Racemat in die Kupplung eingesetzt; die diastereomeren Produkte wurden chromatographisch getrennt.

Rf-Werte: Laufmittel Essigester/Methanol/Eisessig 60/40/1

a) Rf = 0,50
b) Rf = 0,44
c) Rf = 0,33

MS (FAB):

a) Isomer 1: 1055(M+Na)$^+$ 1033 (M+H)$^+$
b) Isomer 2: 1055(M+Na)$^+$ 1033 (M+H)$^+$
c) Isomer 3: 1055(M+Na)$^+$

NMR (270 MHz, DMSO<$D_6$>):

a) Isomer 1: 0,68 (d, 7Hz, 6H); 0,74 (d, 7Hz, 6H); 1,19 (s, 18H); 1,83 (m, 2H); 2,53-2,94 (m, 10H); ca. 3,2-3,45 (m, ca. 10H); 3,53 (dd, 14Hz, 9Hz, 2H); 4.06 (dd, 9hz, 7Hz, 2H); 4,52(m, 2H); 7.05 (m, 2H); 7,10-7,25 (m, 8H); 7,28 (d, 6Hz, 4H), 7,53 (d, 9Hz, 2H); 8,19 (d, 9Hz, 2H); 8,46 (d, 6Hz, 4H)

b) Isomer 2: 0,38, 0,44, 0,65, 0,73 (4d, je 7Hz, je 3H); 1,18 1,28 (2s, je 9H); 1,70-1,88 (m, 2H); 2,54-3,05 (m, ca.11H); 3,15-3,60 (m, ca. 10H); 3,87 (dd, 8Hz, 6Hz, 1H); 4,03 (dd, 9Hz, 7Hz, 1H); 4,36-4,52 (m, 2H); ca. 4,4-5,0 (1H); 7,00-7,30 (m, 14H); 7,41, 7,58, 8,18 8,27 (4d, je 9Hz, je 1H); 8,43, 8,46 (2d, je 6Hz, je 1H)

c) Isomer 3: 0,34 (d, 7Hz, 6H), 0,40 (d, 7Hz, 6H); 1,31 (s, 18H); 1,73 (m, 2H); 2,60-3,07 (m, 12H); 3,26 (s, 2H); 3,38-3,58 (m, 4H); 3,81 (dd, 8Hz, 6Hz, 2H); 4,42 (m, 2H); ca.4,3-5,3 (2H); 7,03-7,30 (m, 14H); 7,43 (d, 9Hz, 2H); 8,28 (d, 9Hz, 2H); 8,43 (d, 6Hz, 4H)

**Beispiel 163**

N,N′-Bis-(<2-(1,1-dimethylethyl-sulfonylmethyl)-3-(N-oxido-4-pyridyl)-propionyl>L-valyl)-2S,5S-diamino-1,6-diphenyl-hexan-3R,4R-diol

Synthese analog Beispiel 162.
2-(1,1-Dimethylethyl-sulfonylmethyl)-3-(N-oxido-4-pyridyl)-propionsäure ensteht aus der Vorstufe 2-(1,1-Dimethylethyl-thio-methyl)-3-(4-pyridyl)-propionsäure durch Oxidation mit drei anstatt mit zwei Equivalenten an Kaliumperoxomonosulfat (Oxone®), wie im Beispiel 162.
MS(FAB): 1065 (M+H)$^+$

**Beispiel 164**
N,N'-Bis-(<bis-(1,1-dimethylethyl-thio-methyl)-acetyl>L-valyl)-2S,5S-diamino-1,6-diphenyl-hexan-3R,4R-diol

Synthese analog Beispiel 13. Die Synthese der Bis-(1,1-dimethylethyl-thio-methyl)-essigsäure erfolgte aus Bis-(hydroxymethyl)-malonsäurediethylester durch Reaktion mit Bromwasserstoff und nachfolgender Substitution der erhaltenen $\beta,\beta'$-Dibromisobuttersäure mit Kalium-tert.-butylsulfid.

MS (FAB): 990 (M+H)$^+$

NMR (270 MHz, CDCl3): 0,59 (d, 7Hz, 6H); 0,85 (d, 7Hz, 6H); 1,29 (s, 18H); 1,33 (s, 18H); 2,16 (m, 2H); 2,42 (m, 2H); 2,70-3,02 (m, 14H); 3,48 (br.s, 2H); 4,13 (m, 2H); 4,28 (m, 2H); 5,33 (d, 8Hz, 2H); 6,47 (d, 8Hz, 2H); 7,20-7,28 (m, 10H)

**Beispiel 165**
N,N'-Bis-(<bis-(1,1-dimethylethyl-sulfonylmethyl)-acetyl>L-valyl)-2S,5S-diamino-1,6-diphenyl-hexan-3R,4R-diol

Synthese analog Beispielen 164 und 13

MS (FAB): 1118 (M+H)$^+$

**Beispiel 166**
N,N'-Bis-(<1-naphthyl>-acetyl-L-valyl)-2S,5S-diamino-1,6-diphenyl-hexan-3R,4R-diol

Synthese analog Beispiel 58

MS (FAB): 834 (M+H)$^+$

**Beispiel 167**
N,N'-Bis-(<1-naphthyloxy>-acetyl-L-valyl)-2S,5S-diamino-1,6-diphenyl-hexan-3R,4R-diol

Synthese analog Beispiel 58

MS (FAB): 866 (M+H)$^+$

**Beispiel 168**
N,N'-Bis-(<2S-(1,1-dimethylethyl-sulfonylmethyl)-3-(1-naphthyl)-propionyl>L-valyl)-2S,5S-diamino-1,6-bis-(4-tert.-butylphenyl)-hexan-3R,4R-diol

Synthese analog Beispiel 6 aus 2S,5S-Diamino-1,6-bis-(4-tert.-butylphenyl)-hexan-3R,4R-diol-dihydrochlorid (Letztere Verbindung wurde analog Beispiel 2, 2b, 2c und 2e aus 1,2R-5R,6-Diepoxy-3,4-O-isopropyliden-3R,4R-diol und 4-Tert.-butylphenyllithium dargestellt)

MS (FAB): 1265 (M+H)$^+$

NMR (270 MHz, DMSO<D6>): 0,67 (d, 7Hz, 6H); 0,76 (d, 7Hz, 6H); 1,09 (s, 18H); 1,11 (s, 18H); 1,87 (m, 2H); 2,60-2,85 (m, 6H); 3,08 (dd, 14Hz, 12Hz, 2H); 3,25-3,50 (m, 8H); 3,60 (dd, 14Hz, 9Hz, 2H); 4,06 (m, 2H); 4,52 (m, 2H); 7,10-7,22 (m, 8H); 7,27 (d, 9Hz, 2H); 7,34-7,62 (m, 8H); 7,80 (m, 2H); 7,92 (m, 2H); 8,22 (d, 8Hz, 4H)

**Beispiel 169**
N,N'-Bis-(<2S-(1,1-dimethylethyl-sulfonylmethyl)-3-(1-naphthyl)-propionyl>L-valyl)-2S,5S-diamino-1,6-bis-(2,4-dimethoxyphenyl)-hexan-3R,4R-diol

Synthese analog Beispiel 6 aus 2S,5S-Diamino-1,6-bis-(2,4-dimethoxyphenyl)-hexan-3R,4R-diol-dihydrochlorid (Letztere Verbindung wurde analog Beispiel 2, 2b, 2c und 2e aus 1,2R-5R,6-Diepoxy-3,4-O-isopropyliden-3R,4R-diol und 2,4-Dimethoxyphenyllithium dargestellt)

MS (FAB): 1250 (M+H)$^+$

**Beispiel 170**
N,N'-Bis-(2-<4-pyridyl>ethylsulfonyl-L-valyl)-2S,5S-diamino-1,6-diphenyl-hexan-3R,4R-diol

Synthese analog Beispiel 58

MS (FAB): 836 (M+H)$^+$

**Beispiel 171**
N,N'-Bis-(12-amino-dodecanoyl-L-valyl)-2S,5S-diamino-1,6-diphenyl-hexan-3R,4R-diol

Synthese analog Beispiel 58
MS (FAB): 892 $(M+H)^+$

**Beispiel 172**
N,N'-Bis-(<2-chinolylcarbonyl>-L-valyl)-2S,5S-diamino-1,6-diphenyl-hexan-3R,4R-diol

Synthese analog Beispiel 58
MS (FAB): 831 $(M+Na)^+$, 809 $(M+H)^+$
NMR (270 MHz, DMSO<D6>): 0,80 (d, 7Hz, 6H); 0,84 (d, 7Hz, 6H); 2,65 (dd, 14Hz, 4Hz, 2H); 2,83 (dd, 14Hz, 10Hz, 2H); 3,34 (m, 2H); 4,43 (dd, 6Hz, 9Hz, 2H); 4,55 (m, 2H); 4,80 (m, 2H); 6,86 (m, 2H); 7,07 (t, 8Hz, 4H); 7,22 (d, 8Hz, 4H); 7,74 (m, 2H); 7,89 (m, 4H); 7,89 (m, 4H); 8,12 (d, 8Hz, 2H); 8,19 (m, 4H); 8,57 (d, 9Hz, 2H); 8,61 (d, 9Hz, 2H)

**Beispiel 173**
N,N'-Bis-(<2-chinolylcarbonyl>-L-asparaginyl)-2S,5S-diamino-1,6-diphenyl-hexan-3R,4R-diol

Synthese analog Beispiel 58
MS (FAB): 861 $(M+Na)^+$
NMR (270 MHz, DMSO<D6>): 2,33-2,78 (m, 8H); 3,30 (m, 2H); 4,33 (m, 2H); 4,70 (m, 4H); 4,70 (m, 4H); 6,80-8,22(m, 26H); 8,59 (d, 8Hz, 2H), 8,92 (d, 8Hz, 2H)

**Beispiel 174**
N,N'-Bis-(tert.-butoxycarbonyl)-2S,4-diamino-1,5-diphenyl-pentan-3-ol

2,3 g Tert.-butoxycarbonyl-L-phenylalanal wurden in 10 ml Ethanol gelöst. Nach Zugabe (bei 0 °C) von 0,05 ml Tetramethylguanidin und einer Lösung von 2,42 g 2-Nitro-1-phenylethan in 2 ml Ethanol ließ man auf RT erwärmen und rührte über Nacht. Die Lösung wurde eingeengt, das zurückbleibende helle Öl (4,8 g) direkt weiter verwendet.

4,7 g des oben erhaltenen Öls wurden in 70 ml Ethanol gelöst. Nach Zusatz von 0,1 ml Eisessig und 1 g Raney-Nickel wurde die Lösung 16 h bei 50 °C und 25 atm Wasserstoff im Glaseinsatz im Autoklaven geschüttelt. Der Katalysator wurde abfiltriert, das Eluat zu einem Öl eingedampft. Den Rückstand löste man in Wasser/1n HCl und extrahierte 4x mit Essigester. Der Essigester-Extrakt wurde eingeengt und direkt weiter verwendet (2,6 g).

2,57 g der oben erhaltenen Aminoverbindung wurden in 25 ml Dioxan bei RT gelöst. Nach Zusatz von 0,86 ml Triethylamin und 1,7 g Di-tert.-butyldicarbonat rührte man weitere 30 min.. Die Lösung wurde aufkonzentriert, der Rückstand mit Eiswasser, Essigester und $KHSO_4$-Lösung bis pH2 versetzt. Die Essigester-Phase wurde mit wässriger NaCl-Lösung gewaschen, über wasserfreiem $Na_2SO_4$ getrocknet und eingeengt. Man erhielt 3,3 g eines Öls. Dieses wurde durch Chromatographie an Kieselgel ($CH_2Cl_2$/Methanol/Eisessig 100/3/0,3) weiter gereinigt. Man erhielt 2,2 g Produkt als Gemisch der Diastereomeren.

MS (FAB): 471 $(M+H)^+$, 371, 315

**Beispiel 175**
N,N'-Bis-(tert.-butoxycarbonyl)-1,3-diaminopropan

MS (FAB): 495 $(M+Na)^+$; 473 $(M+H)^+$
NMR (270 MHz, $CDCl_3$): 0,97 (d, 6Hz, 12H); 1,45 (s, 18H); 1,70 (t, 6Hz, 2H); 2,03 (m, 2H); 3,08 (m, 2H); 3,58 (m, 2H); 3,88 (dd, 2H); 5,09 (d, 2H); 7,21 (s, 2H)

**Beispiel 176**
N,N'-Bis-(tert.-butoxycarbonyl)-1,3-diaminopropan-2-ol

MS (FAB/LiCl): 495 $(M+Li)^+$;
NMR (270)MHz, $CDCl_3$): 0,97 (dd, 12H); 1,45 (s, 18H); 2,04 (m, 2H); 3,20 (m, 2H); 3,61 (m, 2H); 3,90 (dd, 2H), 3,95 (m, 1H); 5,16 (dd, 2H); 7,18 (s, 1H); 7,49 (s, 1H)

**Beispiel 177**
N,N'-Bis-(tert.-butoxycarbonyl)-1,3-diaminoaceton

MS (FAB/LiCl): 493 $(M+Li)^+$
NMR (270 MHz, $CDCl_3$): 0,98 (dd, 12H); 1,45 (s, 18H); 2,09 (m, 2H); 3,94 (dd, 2H); 4,10 (s, 2H); 4,18 (s, 2H); 5,20 (d, 2H); 7,50 (s, 2H)

**Beispiel 178**
N,N'-Bis-(tert.-butoxycarbonyl)-1,4-diaminobutan-2-on

MS (FAB): 523 $(M+Na)^+$; 501 $(M+H)^+$
NMR (270 MHz, $CDCl_3$): 0,95 (m, 12H); 1,43 (d, 18H); 2,09 (m, 2H); 2,69 (m, 2H); 3,45 (m, 1H); 3,86 (m, 1H); 3,90 (m, 1H); 3,99 (m, 1H); 4,18 (m, 1H); 5,23 (d, 2H); (d, 2H); 6,91 (s, 1H); 7,17 (s, 1H)

## Ansprüche

1. Verbindung der Formel I

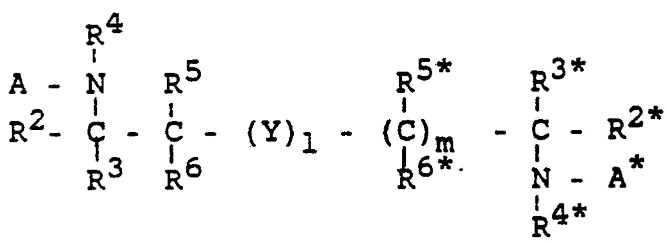

(I)

worin
Y für Sauerstoff, Schwefel, einen Rest der Formel II oder einen Rest der Formel III

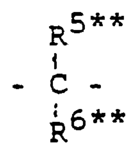

(II)

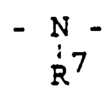

(III)

steht;
l und m unabhängig voneinander 0 oder 1 sind;
A einen Rest der Formel IV und A* einen Rest der Formel IV* bedeuten

$D - (E)_n - (F)_o - (G)_p -$ (IV)

$D^* - (E^*)_{n^*} - (F^*)_{o^*} - (G^*)_{p^*} -$ (IV*)

wobei
E, E*, F, F*, G und G* unabhängig voneinander für eine natürliche oder unnatürliche Aminosäure, Azaaminosäure oder Iminosäure stehen;
n, n*, o, o*, p und p* unabhängig voneinander 0 oder 1 bedeuten;
D für $R^1$ oder einen Rest der Formeln V, VI oder VII und
D* für $R^{1*}$ oder einen Rest der Formeln V*, VI* oder VII* steht

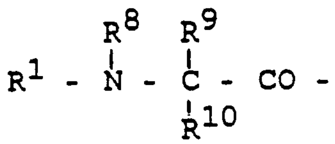 (V) 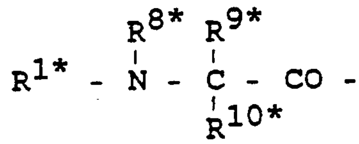 (V*)

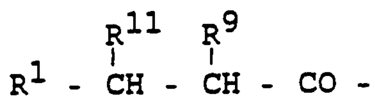 (VI) 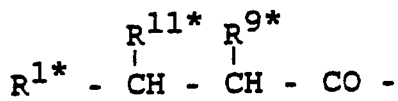 (VI*)

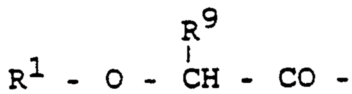 (VII) 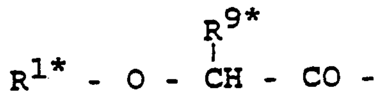 (VII*)

und worin $R^1$ und $R^{1*}$ unabhängig voneinander stehen für

$a_1$)

- Wasserstoff,
- Carboxyl,
- $(C_1-C_{18})$-Alkyl, das gegebenenfalls einfach oder zweifach ungesättigt ist und das gegebenenfalls durch bis zu 3 gleiche oder verschiedene Reste aus der Reihe
- Mercapto,
- Hydroxy,
- $(C_1-C_7)$-Alkoxy,
- Carbamoyl,
- $(C_1-C_8)$-Alkanoyloxy,
- Carboxy,
- $(C_1-C_7)$-Alkoxycarbonyl,
- F, Cl, Br, I,
- Amino,
- Amidino, das gegebenenfalls durch einen, zwei oder drei $(C_1-C_8)$-Alkylreste substituiert sein kann,
- Guanidino, das gegebenenfalls durch einen oder zwei Benzyloxycarbonylreste oder durch einen, zwei, drei oder vier $(C_1-C_8)$-Alkylreste substituiert sein kann,
- $(C_1-C_7)$-Alkylamino,
- Di-$(C_1-C_7)$-Alkylamino,
- $(C_1-C_6)$-Alkoxycarbonylamino,
- $(C_7-C_{15})$-Aralkoxycarbonyl,
- $(C_7-C_{15})$-Aralkoxycarbonylamino,
- Phenyl-$(C_1-C_4)$-alkoxy,
- 9-Fluorenylmethoxycarbonylamino,
- $(C_1-C_6)$-Alkylsulfonyl,
- $(C_1-C_6)$-Alkylsulfinyl,
- $(C_1-C_6)$-Alkylthio,
- Hydroxamino,
- Hydroximino,
- Sulfamoyl,
- Sulfo,
- Carboxamido,
- Formyl,
- Hydrazono,
- Imino,
- einen Rest $CONR^{12}R^{13}$ bzw. $CONR^{12*}R^{13*}$,
- durch bis zu sechs Hydroxy oder
- durch bis zu fünf $(C_1-C_8)$-Alkanoyloxy substituiert ist;
- mono-, bi- oder tricyclisches $(C_3-C_{18})$-Cycloalkyl,
- $(C_3-C_{18})$-Cycloalkyl-$(C_1-C_6)$-alkyl wobei der Cycloalkylteil jeweils gegebenenfalls durch einen oder zwei gleiche oder verschiedene Reste aus der Reihe

- F, Cl, Br, I,
- Carboxy,
- Carbamoyl,
- Carboxymethoxy,
- Hydroxy,
- $(C_1-C_7)$-Alkoxy,
- $(C_1-C_7)$-Alkyl,
- $(C_1-C_7)$-Alkyloxycarbonyl,
- Amino,
- $(C_1-C_6)$-Alkylamino-$(C_1-C_6)$-alkyl,
- Di-$(C_1-C_6)$-Alkylamino-$(C_1-C_6)$-alkyl,
- Amidino,
- Hydroxamino,
- Hydroximino,
- Hydrazono,
- Imino,
- Guanidino,
- $(C_1-C_6)$-Alkoxysulfonyl,
- $(C_1-C_6)$-Alkoxysulfinyl,
- $(C_1-C_6)$-Alkoxycarbonylamino,
- $(C_6-C_{12})$-Aryl-$(C_1-C_4)$-Alkoxycarbonylamino,
- $(C_1-C_7)$-Alkylamino,
- Di-$(C_1-C_7)$-alkylamino und
- Trifluormethyl

substituiert ist;

- $(C_6-C_{14})$-Aryl,
- $(C_6-C_{14})$-Aryl-$(C_1-C_6)$-alkyl,
- $(C_6-C_{14})$-Aryloxy-$(C_1-C_6)$-alkyl oder
- $(C_6-C_{14})$-Aryl-$(C_3-C_8)$-cycloalkyl, worin der Arylteil jeweils gegebenenfalls durch einen, zwei oder drei gleiche oder verschiedene Reste aus der Reihe
- F, Cl, Br, I,
- Hydroxy,
- Mono-, Di- oder Trihydroxy-$(C_1-C_4)$-alkyl,
- Trifluormethyl,
- Formyl,
- Carboxamido,
- Mono- oder Di-$(C_1-C_4)$-alkylaminocarbonyl,
- Nitro,
- $(C_1-C_7)$-Alkoxy,
- $(C_1-C_7)$-Alkyl,
- $(C_1-C_7)$-Alkoxycarbonyl,
- Amino,
- $(C_1-C_7)$-Alkylamino,
- Di-$(C_1-C_7)$-alkylamino,
- Carboxy,
- Carboxymethoxy,
- Amino-$(C_1-C_7)$-alkyl,
- $(C_1-C_7)$-Alkylamino-$(C_1-C_7)$-alkyl,
- Di-$(C_1-C_7)$-alkylamino-$(C_1-C_7)$-alkyl,
- $(C_1-C_7)$-Alkoxycarbonylmethoxy,
- Carbamoyl,
- Sulfamoyl,
- $(C_1-C_7)$-Alkoxysulfonyl,
- $(C_1-C_8)$-Alkylsulfonyl,
- Sulfo-$(C_1-C_8)$-alkyl,
- Guanidino-$(C_1-C_8)$-alkyl und
- $(C_1-C_6)$-Alkoxycarbonylamino

substituiert ist;

- Het,
- Het-($C_1$-$C_6$)-alkyl,
- Het-($C_3$-$C_8$)-cycloalkyl,
- Het-($C_3$-$C_8$)-cycloalkyl-($C_1$-$C_4$)-alkyl,
- Het-($C_3$-$C_8$)-cycloalkoxy-($C_1$-$C_4$)-alkyl,
- Het-thio-($C_1$-$C_6$)-alkyl,
- Het-thio-($C_3$-$C_8$)-cycloalkyl,
- Het-thio-($C_3$-$C_8$)-cycloalkyl-($C_1$-$C_4$)-alkyl,

wobei Het jeweils für den Rest eines 5- bis 7-gliedrigen monocyclischen oder 8- bis 10-gliedrigen bicyclischen Ringsystems steht, das benzanelliert, aromatisch, teilhydriert oder vollständig hydriert sein kann, das als Heteroelemente einen, zwei, drei oder vier verschiedene Reste aus der Gruppe N, O, S, NO, SO, $SO_2$ enthalten kann, das mit 1 bis 6 Hydroxy substituiert sein kann und das gegebenenfalls wie bei ($C_6$-$C_{14}$)-Aryl unter $a_1$) definiert und/oder mit Oxo, mono-, di- oder trisubstituiert ist,
oder einen Rest $NR^{12}R^{13}$ bzw. $NR^{12*}R^{13*}$ bedeuten oder,

$a_2$)
- einen Rest der Formel VIII beziehungsweise VIII*
$R^{1a}$ - W (VIII)
$R^{1a*}$ - W * (VIII*)
worin $R^{1a}$ und $R^{1a*}$ wie $R^1$ bzw. $R^{1*}$ unter $a_1$) definiert sind und W bzw. W* für -CO-, -CS-, O-CO-, -$SO_2$-, -SO-, -S-, -$NHSO_2$-, -NHCO-, -CH(OH)-, -N(OH)- oder -CO-V- wobei V ein Peptid mit 1 bis 10 Aminosäuren bedeutet, stehen;
oder worin $R^1$ und $R^{1*}$ unabhängig voneinander zusammen mit $R^{11}$ bzw. $R^{11*}$ und den diese tragenden Atomen mono- oder bicyclische, gesättigte oder teilweise ungesättigte Ringsysteme mit 5-12 Ringgliedern bilden, die außer Kohlenstoff noch 1 Schwefelatom enthalten können, welches gegebenenfalls zum Sulfoxid oder Sulfon oxidiert sein kann;

$a_3$)
- einen Glycosylrest, bevorzugt einen Glucofuranosyl- oder Glucopyranosyl-Rest, der sich von natürlich vorkommenden Aldotetrosen, Aldopentosen, Aldohexosen, Ketopentosen, Ketohexosen, Desoxyaldosen, Aminoaldosen und Oligosacchariden sowie deren Stereoisomeren ableitet;

$R^2$ und $R^{2*}$
unabhängig voneinander definiert sind wie $R^1$ bzw. $R^{1*}$ unter $a_1$) oder $a_2$) oder
zusammen mit $R^4$ bzw. $R^{4*}$ und den diese tragenden Atomen mono- oder bicyclische, gesättigte oder teilweise ungesättigte Ringsysteme mit 5 bis 12 Ringgliedern bilden, oder zusammen mit $R^3$ bzw. $R^{3*}$ und den diese tragenden Atomen cyclische, gesättigte oder teilweise ungesättigte Ringsysteme mit 3 bis 12 Ringgliedern bilden;

$R^3$ und $R^{3*}$
unabhängig voneinander
- Wasserstoff oder
- ($C_1$-$C_3$)-Alkyl bedeuten;

$R^4$ und $R^{4*}$
unabhängig voneinander
- Wasserstoff oder
- ($C_1$-$C_8$)-Alkyl bedeuten;

$R^5$, $R^{5*}$ und $R^{5**}$
unabhängig voneinander
- Wasserstoff,
- Hydroxy,
- Amino oder
- Carboxy bedeuten, oder

mit $R^6$, $R^{6*}$ bzw. $R^{6**}$ zusammen mit den sie tragenden Kohlenstoffatomen jeweils unabhängig voneinander eine Ketogruppe bilden;

$R^6$, $R^{6*}$ und $R^{6**}$
unabhängig voneinander
- Wasserstoff oder
- ($C_1$-$C_6$)-Alkyl bedeuten oder

im Falle l=0, $R^6$ und $R^{6*}$ gegebenenfalls eine gemeinsame Bindung ausbilden können;

$R^7$
- Wasserstoff,

- Hydroxy oder
- $(C_1-C_6)$-Alkyl bedeutet;

$R^8$ und $R^{8*}$
unabhänging voneinander
- Wasserstoff oder
- $(C_1-C_8)$-Alkyl bedeuten, oder

zusammen mit $R^9$ bzw. $R^{9*}$ und den diese tragenden Atomen mono- oder bicyclische, gesättigte oder teilweise ungesättigte Ringsysteme mit 5-12 Ringgliedern bilden;

$R^9$ und $R^{9*}$
unabhängig voneinander definiert sind wie $R^1$ bzw. $R^{1*}$ unter $a_1$), für Hydroxy oder $(C_1-C_4)$-Alkanoyloxy stehen oder zusammen mit $R^{10}$ bzw. $R^{10*}$ und den diese tragenden Atomen cyclische, gesättigte oder teilweise ungesättigte Ringsysteme mit 3 bis 12 Ringgliedern bilden;
oder
zusammen mit $R^{11}$ bzw. $R^{11*}$ und den diese tragenden Atomen ein mono- oder bicyclisches, gesättigtes oder teilweise ungesättigtes Ringsystem mit 5-12 Ringgliedern bilden, das außer Kohlenstoff noch 1 Schwefelatom enthalten kann,
welches gegebenenfalls zum Sulfoxid oder Sulfon oxidiert sein kann; oder 1 Stickstoffatom enthalten kann, wobei das Ringsystem gegebenenfalls durch Amino substituiert sein kann;

$R^{10}$ und $R^{10*}$
unabhängig voneinander
- Wasserstoff oder
- $(C_1-C_6)$-Alkyl bedeuten;

$R^{11}$ und $R^{11*}$
unabhängig voneinander
- Wasserstoff,
- Hydroxy,
- $(C_1-C_4)$-Alkanoyloxy oder
- $(C_1-C_8)$-Alkyl bedeuten;

$R^{12}$, $R^{12*}$, $R^{13}$ und $R^{13*}$
unabhängig voneinander
- Wasserstoff,
- $(C_1-C_8)$-Alkyl, das durch
- Amino,
- $(C_1-C_4)$-Alkylamino,
- Di-$(C_1-C_4)$-alkylamino,
- Mercapto,
- Carboxy,
- Hydroxy oder
- $(C_1-C_4)$-Alkoxy substituiert sein kann,
- $(C_3-C_7)$-Cycloalkyl,
- $(C_1-C_4)$-Alkoxycarbonyl,
- $(C_6-C_{14})$-Aryl, $(C_6-C_{14})$-Aryl-$(C_1-C_4)$-Alkoxycarbonyl, die im Arylteil wie bei $R^1$ bzw. $R^{1*}$ beschrieben, substituiert sein können,
- Het oder
- Het-$(C_1-C_4)$-alkyl, wobei Het wie bei $R^1$ bzw. $R^{1*}$ beschrieben definiert ist, bedeuten

oder wobei $R^{12}$ und $R^{13}$ bzw. $R^{12*}$ und $R^{13*}$ zusammen mit dem sie tragenden Stickstoffatomen monocyclische oder bicyclische, gesättigte, teilweise ungesättigte oder aromatische Ringsysteme bilden, die als weitere Ringglieder neben Kohlenstoff noch 1 oder 2 Stickstoffatome, 1 Schwefelatom oder 1 Sauerstoffatom enthalten und durch $(C_1-C_4)$-Alkyl substituiert sein können,
wobei
in den vorstehenden Verbindungen der Formel I eine oder mehrere Amidgruppen (-CONH-) der Hauptkette ersetzt sein können durch $-CH_2NR^{14}-$, $-CH_2S-$, $-CH_2O-$, $-OCH_2-$, $-CH_2CH_2-$, -CH=CH-(cis und trans), $-COCH_2-$, $-CH(OH)CH_2-$, $-CH_2SO-$, $-CH_2SO_2-$, -COO-, $-P(O)(OR^{15})CH_2-$ und $-P(O)(OR^{15})NH-$, oder auch durch eine Amidgruppe mit umgekehrter Polarität (-NHCO-);
worin $R^{14}$ und $R^{15}$
unabhängig voneinander stehen für
- Wasserstoff oder
- $(C_1-C_4)$-Alkyl;

sowie deren physiologisch verträgliche Salze.

2. Verbindung der Formel I gemäß Anspruch 1, dadurch gekennzeichnet, daß die Reste und Symbole mit und ohne Stern jeweils identisch sind.

3. Verbindung der Formel I gemäß den Ansprüchen 1 bis 2, dadurch gekennzeichnet, daß die Verbindung $C_2$-symmetrisch ist.

4. Verbindung der Formel I gemäß den Ansprüchen 1 bis 3, dadurch gekennzeichnet, daß

Y für einen Rest der Formel II oder einen Rest der Formel III steht;

l, m, A, A*, D, D*, n, n*, o, o*, p und p* wie in Anspruch 1 definiert sind;

E, E*, F, F*, G und G* unabhängig voneinander für eine natürliche oder unnatürliche α-Aminosäure oder α-Iminosäure stehen;

$R^1$ und $R^{1*}$

unabhängig voneinander stehen für

$a_1$) - Wasserstoff;

- Carboxyl,
- $(C_1\text{-}C_{16})$-Alkyl, das gegebenenfalls einfach ungesättigt ist und das gegebenenfalls durch bis zu 2 gleiche oder verschiedene Reste aus der Reihe
- Hydroxy,
- $(C_1\text{-}C_4)$-Alkoxy,
- Carbamoyl,
- $(C_1\text{-}C_8)$-Alkanoyloxy,
- Carboxy,
- $(C_1\text{-}C_4)$-Alkoxycarbonyl,
- F,
- Amino,
- $(C_1\text{-}C_7)$-Alkylamino,
- Di-$(C_1\text{-}C_7)$-Alkylamino,
- $(C_1\text{-}C_6)$-Alkoxycarbonylamino,
- Benzyloxycarbonyl
- Benzyloxycarbonylamino,
- 9-Fluorenylmethoxycarbonylamino,
- $(C_1\text{-}C_4)$-Alkylsulfonyl,
- einen Rest $CONR^{12}R^{13}$ bzw. $CONR^{12*}R^{13*}$,
- durch bis zu sechs Hydroxy oder
- durch bis zu vier $(C_1\text{-}C_8)$-Alkanoyloxy substituiert ist;
- mono- oder bicyclisches $(C_3\text{-}C_{12})$-Cycloalkyl,
- $(C_3\text{-}C_{12})$-Cycloalkyl-$(C_1\text{-}C_6)$-alkyl wobei der Cycloalkylteil jeweils gegebenenfalls durch einen oder zwei gleiche oder verschiedene Reste aus der Reihe
- F,
- Carboxy,
- Hydroxy,
- $(C_1\text{-}C_7)$-Alkoxy,
- $(C_1\text{-}C_4)$-Alkyl,
- $(C_1\text{-}C_4)$-Alkyloxycarbonyl,
- Amino,
- $(C_1\text{-}C_6)$-Alkoxycarbonylamino,
- Benzyloxycarbonylamino
- $(C_1\text{-}C_4)$-Alkylamino und
- Di-$(C_1\text{-}C_4)$-alkylamino

substituiert ist;

- $(C_6\text{-}C_{10})$-Aryl,
- $(C_6\text{-}C_{10})$-Aryloxy-$(C_1\text{-}C_6)$-alkyl oder
- $(C_6\text{-}C_{10})$-Aryl-$(C_1\text{-}C_6)$-alkyl, worin der Arylteil jeweils gegebenenfalls durch einen, zwei oder drei gleiche oder verschiedene Reste aus der Reihe
- F, Cl, Br,
- Hydroxy,
- Hydroxy-$(C_1\text{-}C_4)$-alkyl,
- Carboxamido,
- Mono- oder Di-$(C_1\text{-}C_4)$-alkylaminocarbonyl,

- $(C_1-C_4)$-Alkoxy,
- $(C_1-C_4)$-Alkyl,
- $(C_1-C_4)$-Alkoxycarbonyl,
- Amino,
- $(C_1-C_4)$-Alkylamino,
- Di-$(C_1-C_4)$-alkylamino,
- Carboxy,
- Carbamoyl,
- $(C_1-C_4)$-Alkoxycarbonylamino

substituiert ist;

- Het,
- Het-$(C_1-C_6)$-alkyl,
- Het-$(C_5-C_6)$-cycloalkyl,
- Het-thio-$(C_1-C_4)$-alkyl,
- Het-thio-$(C_5-C_6)$-cycloalkyl,

wobei Het jeweils für den Rest eines 5- bis 6-gliedrigen monocyclischen oder 8- bis 10-gliedrigen bicyclischen Ringsystems steht, das aromatisch, teilhydriert oder vollständig hydriert sein kann, das als Heteroelemente einen, zwei, drei oder vier verschiedene Reste aus der Gruppe N, O, S, NO, SO, $SO_2$ enthalten kann, das mit 1 bis 4 Hydroxy substituiert sein kann und das gegebenenfalls wie bei $(C_6-C_{10})$-Aryl unter $a_1$) definiert und/oder mit Oxo mono-, oder di-substituiert ist,

oder einen Rest $NR^{12}R^{13}$ bzw. $NR^{12*}R^{13*}$ bedeuten oder,

$a_2$) - einen Rest der Formel VIII beziehungsweise VIII*

$R^{1a}$ - W (VIII)

$R^{1a*}$ - W* (VIII*)

worin $R^{1a}$ und $R^{1a*}$ wie $R^1$ bzw. $R^{1*}$ unter $a_1$) definiert sind und W bzw. W* für -CO-, -O-CO-, $-SO_2-$, -SO-, -S-, -NHCO- oder-CH(OH)-, stehen;

oder worin $R^1$ und $R^{1*}$ unabhängig voneinander zusammen mit $R^{11}$ bzw. $R^{11*}$ und den diese tragenden Atomen monocyclische, gesättigte oder teilweise ungesättigte Ringsysteme mit 5-8 Ringgliedern bilden, die außer Kohlenstoff noch 1 Schwefelatom enthalten können, welches gegebenenfalls zum Sulfoxid oder Sulfon oxidiert sein kann;

$a_3$) - einen Glycosylrest, der wie in Anspruch 1 definiert ist;

$R^2$ und $R^{2*}$

unabhängig voneinander

$b_1$) - Wasserstoff,

- Carboxy,
- $(C_1-C_{10})$-Alkyl, das gegebenenfalls ein- oder zweifach ungesättigt ist und das gegebenenfalls durch bis zu 3 gleiche oder verschiedene Reste aus der Reihe
- Hydroxy,
- $(C_1-C_7)$-Alkoxy,
- $(C_1-C_7)$-Alkylthio,
- $(C_1-C_7)$-Alkylsulfinyl,
- $(C_1-C_7)$-Alkylsulfonyl,
- $(C_1-C_7)$-Alkanoyloxy,
- Carboxy,
- $(C_1-C_7)$-Alkoxycarbonyl,
- Cl, Br,
- Amino,
- Amidino,
- Guanidino,
- N,N′-Di-(benzyloxycarbonyl)-guanidino,
- Carbamoyl,
- $(C_7-C_{15})$-Aralkoxycarbonyl,
- $(C_1-C_5)$-Alkoxycarbonylamino,
- $(C_7-C_{15})$-Aralkoxycarbonylamino oder
- 9-Fluorenylmethoxycarbonylamino substituiert ist;
- $(C_3-C_{12})$-Cycloalkyl,
- $(C_3-C_{12})$-Cycloalkyl-$(C_1-C_3)$-alkyl,
- $(C_6-C_{14})$-Aryl,

- $(C_6-C_{14})$-Aryl-$(C_1-C_3)$-alkyl, wobei der Aryl-Teil jeweils gegebenenfalls durch einen, zwei oder drei gleiche oder verschiedene Reste aus der Reihe
- F, Cl, Br, I,
- Hydroxy,
- $(C_1-C_7)$-Alkoxy,
- $(C_1-C_7)$-Alkyl,
- $(C_1-C_7)$-Alkoxycarbonyl,
- Amino und
- Trifluormethyl substituiert ist; oder
- Het-$(C_1-C_6)$-alkyl, wobei Het für den Rest eines 5-oder 6-gliedrigen monocyclischen oder 9- bis 10-gliedrigen bicyclischen, gegebenenfalls teilweise oder vollständig hydrierten Heteroaromaten, mit mindestens 1 C-Atom, 1-4 N-Atomen und/oder 1-2 S-Atomen und/oder 1-2 O-Atomen als Ringglieder steht, der gegebenenfalls wie in Anspruch 1 für den Arylteil beschrieben mono- oder disubstituiert ist, bedeuten; oder

$b_2$) zusammen mit $R^4$ bzw. $R^{4*}$ und den diese tragenden Atomen Pyrrolidin oder Piperidin, die jeweils noch mit Cyclopentyl, Cyclohexyl oder Phenyl annelliert sein können, bilden,
oder zusammen mit $R^3$ bzw. $R^{3*}$ und den diese tragenden Atome cyclische, gesättigte oder teilweise ungesättigte Ringsysteme mit 3-8 Ringgliedern bilden;

$R^3$ und $R^{3*}$
unabhängig voneinander
- Wasserstoff,
- Methyl oder
- Ethyl bedeuten;

$R^4$ und $R^{4*}$
unabhängig voneinander
- Wasserstoff,
- $(C_1-C_4)$-Alkyl bedeuten;

$R^5$, $R^{5*}$ und $R^{5**}$ unabhängig voneinander wie in Anspruch 1 definiert sind;

$R^6$, $R^{6*}$ und $R^{6**}$
unabhängig voneinander
- Wasserstoff,
- $(C_1-C_4)$-Alkyl bedeuten;

$R^7$
- Wasserstoff,
- Hydroxy oder
- $(C_1-C_4)$-Alkyl bedeutet.

$R^8$ und $R^{8*}$
unabhängig voneinander
- Wasserstoff,

$(C_1-C_8)$-Alkyl bedeuten oder zusammen mit $R^9$ bzw. $R^{9*}$ und den diese tragenden Atomen Pyrrolidin oder Piperidin, die jeweils zusätzlich mit Cyclopentyl, Cyclohexyl oder Phenyl annelliert sein können, bilden;

$R^9$ und $R^{9*}$
unabhängig voneinander definiert sind wie $R^2$ bzw. $R^{2*}$ unter $b_1$), oder
$(C_1-C_8)$-Alkanoyloxy bedeutet oder
zusammen mit $R^{10}$ bzw $R^{10*}$ und den diese tragenden Atomen cyclische, gesättigte oder teilweise ungesättigten Ringsysteme mit 5 bis 12 Ringgliedern bilden;
oder
zusammen mit $R^{11}$ bzw. $R^{11*}$ und den diese tragenden Atomen ein mono- oder bicyclisches, gesättigtes oder teilweise ungesättigtes Ringsystem mit 5-12 Ringgliedern bilden, das außer Kohlenstoff noch 1 Schwefelatom enthalten kann, welches gegebenenfalls zum Sulfoxid oder Sulfon oxidiert sein kann;

$R^{10}$ und $R^{10*}$
unabhängig voneinander
- Wasserstoff oder
- $(C_1-C_4)$-Alkyl bedeuten;

$R^{11}$ und $R^{11*}$
unabhängig voneinander
- Wasserstoff,
- Hydroxy,
- $(C_1-C_4)$-Alkanoyloxy oder

- $(C_1-C_4)$-Alkyl bedeuten;
$R^{12}$, $R^{12*}$, $R^{13}$ und $R^{13*}$
unabhängig voneinander
- Wasserstoff,
- $(C_1-C_8)$-Alkyl, das durch
- Amino,
- $(C_1-C_4)$-Alkylamino,
- Di-$(C_1-C_4)$-alkylamino,
- Carboxy,
- Hydroxy oder
- $(C_1-C_4)$-Alkoxy substituiert sein kann,
- $(C_1-C_{14})$-Alkoxycarbonyl,
- $(C_6-C_{10})$-Aryl, das wie bei $R^1$ bzw. $R^{1*}$ beschreiben substituiert sein kann,
- $(C_6-C_{10})$-Aryl-$(C_1-C_4)$-alkoxycarbonyl,
- Het oder
- Het-$(C_1-C_4)$-alkyl bedeuten, wobei Het wie bei $R^1$ bzw. $R^{1*}$ beschreiben definiert ist,
wobei
in den vorstehenden Verbindungen der Formel I eine oder mehrere Amidgruppen (-CONH-) der Hauptkette ersetzt sein können durch eine Gruppe bestehend aus $-CH_2NR^{14}-$, $-CH_2O-$, $-OCH_2-$, $-CH_2CH_2-$, $-COCH_2-$, $-CH(OH)CH_2-$, -COO- oder auch durch eine Amidgruppe mit umgekehrter Polarität (-NHCO-);
$R^{14}$ für
- Wasserstoff oder
- $(C_1-C_4)$-Alkyl steht;
sowie deren physiologisch verträgliche Salze.

5. Verbindung der Formel I gemäß den Ansprüchen 1 bis 4, dadurch gekennzeichnet, daß
Y für einen Rest der Formel II oder einen Rest der Formel III steht;
l, m, A, A*, D, D*, n, n*, o, o* wie in Anspruch 1 definiert sind,
p und p* für 1 stehen;
$R^1$ und $R^{1*}$
unabhängig voneinander stehen für
- Wasserstoff,
- Carboxyl,
- $(C_1-C_{10})$-Alkyl,
- $(C_3-C_8)$-Cycloalkyl,
- $(C_3-C_8)$-Cycloalkyl-$(C_1-C_{10})$-alkyl,
- Phenyl-$(C_1-C_8)$-alkyl, das wie in Anspruch 4 im Phenylteil substituiert sein kann,
- gegebenenfalls geschütztes Mono- oder Di-Amino-$(C_1-C_{12})$-alkyl oder Amino-$(C_6-C_{10})$-aryl-$(C_1-C_4)$-alkyl oder Amino-$(C_3-C_{10})$-cycloalkyl-$(C_1-C_4)$-alkyl,
- Mono-, Di-, Tri-, Tetra-, Penta- oder Hexahydroxy-$(C_1-C_{10})$-alkyl oder -alkanoyl,
- $(C_1-C_4)$-Alkoxy-$(C_1-C_{10})$-alkyl,
- $(C_1-C_4)$-Alkoxycarbonyl-$(C_1-C_{10})$-alkyl,
- $(C_1-C_{16})$-Alkylsulfonyl,
- $(C_1-C_8)$-Alkylsulfinyl,
- Mono-, Di-, Trihydroxy-$(C_1-C_8)$-alkylsulfonyl,
- Mono-, Di-, Trihydroxy-$(C_1-C_8)$-alkylsulfinyl,
- Mono-, Di-, Tri oder Tetra-$(C_1-C_8)$-alkanoyloxy-$(C_1-C_{10})$-alkyl,
- $(C_1-C_{14})$-Alkanoyl,
- gegebenenfalls geschütztes Amino-$(C_1-C_{11})$-alkanoyl,
- Di-$(C_1-C_7)$-alkylamino-$(C_2-C_{11})$-alkanoyl,
- $(C_1-C_9)$-Cycloalkylcarbonyl,
- Aminosubstituiertes $(C_3-C_9)$-Cycloalkylcarbonyl,
- Aminosubstituiertes $(C_3-C_9)$-Cycloalkylsulfonyl,
- $(C_6-C_{10})$-Aryl-$(C_2-C_{11})$-alkanoyl,
- $(C_6-C_{10})$-Aryloxy-$(C_2-C_{11})$-alkanoyl,
- gegebenenfalls durch Amino, Halogen, $(C_1-C_7)$-Alkyl, $(C_1-C_7)$-Alkoxy oder $(C_1-C_7)$-Alkoxycarbonyl substituiertes Benzoyl, Benzolsulfonyl oder $(C_6-C_{10})$-Aryl-$(C_1-C_4)$-alkylcarbonyl bzw. -sulfonyl,
- $(C_1-C_{10})$-Alkoxycarbonyl,
- substituiertes $(C_1-C_{10})$-Alkoxycarbonyl,

- $(C_6-C_{14})$-Aryl-$(C_1-C_6)$-alkoxycarbonyl,
- durch gegebenenfalls geschütztes Amino und Hydroxy substituiertes $(C_6-C_{10})$-Aryl-$(C_1-C_8)$-alkyl, $(C_3-C_{10})$-Cycloalkyl-$(C_1-C_8)$- alkyl oder $(C_1-C_{10})$-Alkyl,
- 9-Fluorenylmethoxycarbonyl,
- Ketohexosyl,
- Ketopentosyl,
- Desoxyhexoketosyl,
- Desoxypentoketosyl,
- Aldohexosyl,
- Aldopentosyl,
- Desoxyhexoaldosyl,
- Desoxypentoaldosyl,
- 2-Amino-2-desoxyhexosyl,
- 2-Acetamido-2-desoxyhexosyl,
- Lactosyl oder
- Maltosyl wobei die verknüpften Zucker in der Pyranose-oder Furanose-Form vorliegen können,
- Het-$(C_1-C_6)$-alkyl
- Het-carbonyl oder -sulfonyl,
- Het-$(C_1-C_6)$-alkylcarbonyl oder -sulfonyl,
- Het-mercapto-$(C_1-C_6)$-alkylcarbonyl oder -sulfonyl,

wobei Het jeweils für

Furyl, Thienyl, Benzothienyl, Benzdioxolanyl, Pyrrolyl, Imidazolyl, Isoxazolyl, Thiazolyl, Pyrazolyl, Triazolyl, Pyridyl, Pyrimidyl, Pyrazinyl, Pyridazinyl, Triazinyl, Pyrrolidyl, Piperidyl, Piperazinyl, Morpholino, Thiomorpholino, Tetrahydrofuryl, Tetrahydropyryl, Tetrahydrothienyl, Indolyl, Chinolyl oder Isochinolyl, wobei diese auch durch eine oder zwei gleiche oder verschiedene Reste aus der Gruppe $(C_1-C_4)$-Alkyl, $(C_1-C_4)$- Alkoxy, $(C_1-C_4)$-Alkoxycarbonyl, $(C_1-C_4)$-Alkoxycarbonylamino, Hydroxy, Amino, Mono- oder Di-$(C_1-C_4)$-Alkylamino und Oxido substituiert sein können;

$R^2$ und $R^{2*}$

unabhängig voneinander

- Wasserstoff,
- Carboxyl,
- $(C_1-C_8)$-Alkyl, das gegebenenfalls durch bis zu 2 gleiche oder verschiedene Reste aus der Reihe
- Hydroxy,
- $(C_1-C_4)$-Alkoxy,
- $(C_1-C_4)$-Alkylthio,
- $(C_1-C_4)$-Alkylsulfinyl,
- $(C_1-C_4)$-Alkylsulfonyl,
- $(C_1-C_4)$-Alkanoyloxy,
- Carboxy,
- $(C_1-C_4)$-Alkoxycarbonyl,
- Amino,
- Amidino,
- Guanidino,
- N,N′-Di-(benzyloxycarbonyl)-guanidino,
- Carbamoyl,
- $(C_6-C_{10})$-Aryl-$(C_1-C_3)$-alkoxycarbonyl,
- $(C_1-C_5)$-Alkoxycarbonylamino,
- $(C_6-C_{10})$-Aryl-$(C_1-C_3)$-alkoxycarbonylamino substituiert ist oder
- $(C_3-C_{10})$-Cycloalkyl,
- $(C_3-C_{10})$-Cycloalkyl-$(C_1-C_3)$-alkyl,
- $(C_1-C_4)$-Alkyl-$(C_3-C_{10})$-cycloalkyl-$(C_1-C_3)$-alkyl,
- $(C_6-C_{10})$-Aryl,
- $(C_6-C_{10})$-Aryl-$(C_1-C_3)$-alkyl, wobei der Arylteil jeweils gegebenenfalls durch einen, zwei oder drei gleiche oder verschiedene Reste aus der Reihe
- F, Cl, Br,
- Hydroxy,
- $(C_1-C_4)$-Alkoxy,
- $(C_1-C_4)$-Alkyl,

- $(C_1-C_4)$-Alkoxycarbonyl und
- Amino substituiert ist, oder
- Het-$(C_1-C_4)$-alkyl, wobei Het wie bei $R^1$ bzw $R^{1*}$ definiert ist, bedeuten;

$R^3$ und $R^{3*}$
unabhängig voneinander
- Wasserstoff oder
- Methyl bedeuten;

$R^4$ und $R^{4*}$
unabhängig voneinander
- Wasserstoff oder
- Methyl bedeuten;

$R^5$, $R^{5*}$ und $R^{5**}$
unabhängig voneinander
- Wasserstoff,
- Hydroxy,
- Amino oder
- Carboxy bedeuten;

$R^6$, $R^{6*}$ und $R^{6**}$
unabhängig voneinander
- Wasserstoff oder
- Methyl bedeuten;

$R^7$
- Wasserstoff,
- Hydroxy oder
- Methyl bedeutet;

$R^8$ und $R^{8*}$
unabhängig voneinander
- Wasserstoff,
- Methyl, Ethyl oder n-Propyl bedeuten oder zusammen mit $R^9$ bzw $R^{9*}$ und den diese tragenden Atomen ein 1,2,3,4-Tetrahydroisochinolin oder ein 2-Azabicyclooctan-Gerüst bilden;

$R^9$ und $R^{9*}$
unabhängig voneinander definiert sind wie $R^2$ bzw $R^{2*}$ in Anspruch 4, oder
$(C_1-C_8)$-Alkanoyloxy bedeuten oder
zusammen mit $R^{10}$ bzw. $R^{10*}$ und den diese tragenden Atomen cyclische Ringsysteme mit 5 bis 7 Ringgliedern bilden;
oder zusammen mit $R^{11}$ bzw $R^{11*}$ ein Thiochromansystem bilden, dessen Schwefelatom gegebenenfalls zum Sulfon oxidiert sein kann;

$R^{10}$ und $R^{10*}$
unabhängig voneinander
- Wasserstoff oder
- Methyl bedeuten;

$R^{11}$ und $R^{11*}$ wie in Anspruch 4 definiert sind;
wobei
in den vorstehenden Verbindungen der Formel I eine oder mehrere Amidgruppen (-CONH-) der Hauptkette ersetzt sein können wie in Anspruch 4 definiert;

$R^{14}$ für
- Wasserstoff oder
- Methyl steht;

sowie deren physiologisch verträglichen Salze.

6. Verbindung der Formel I gemäß den Ansprüchen 1 bis 5, dadurch gekennzeichnet, daß
$R^1$ und $R^{1*}$
unabhängig voneinander stehen für
$a_1$) - Wasserstoff,
- Carboxyl,
- $(C_1-C_{16})$-Alkylsulfonyl,
- $(C_1-C_8)$-Alkylsulfinyl,
- $(C_1-C_8)$-Mono-, -Di- oder -Tri-Hydroxyalkylsulfonyl,
- Hydroxy-$(C_1-C_{10})$-alkanoyl,

- Mono-, Di-, Tri- oder Tetra-Hydroxy-($C_1$-$C_4$)-alkyl,
- ($C_1$-$C_8$)-Alkanoyloxy-($C_1$-$C_{10}$)-alkyl,
- 1,2-Diacetoxyethyl,
- 1,2,3-Triacetoxypropyl,
- ($C_1$-$C_{14}$)-Alkanoyl,
- Amino-($C_1$-$C_{12}$)-alkanoyl,
- N-($C_1$-$C_4$)-Alkoxycarbonylamino-($C_1$-$C_8$)-alkyl,
- Di-($C_1$-$C_7$)-alkylamino-($C_2$-$C_{11}$)-alkanoyl,
- ($C_3$-$C_9$)-Cycloalkylcarbonyl,
- Amino-($C_3$-$C_8$)-Cycloalkylcarbonyl,
- Amino-($C_3$-$C_8$)-Cycloalkylsulfonyl,
- Phenyl,
- ($C_6$-$C_{10}$)-Aryl-($C_2$-$C_{11}$)-alkanoyl,
- ($C_6$-$C_{10}$)-Aryloxy-($C_2$-$C_{11}$)-alkanoyl,
- gegebenenfalls durch Halogen, Amino, ($C_1$-$C_7$)-Alkyl, ($C_1$-$C_7$)-Alkoxy oder ($C_1$-$C_7$)-Alkoxycarbonyl substituiertes Benzoyl oder Benzolsulfonyl,
- gegebenenfalls durch Halogen, Amino, ($C_1$-$C_7$)-Alkyl, ($C_1$-$C_7$)-Alkoxy oder ($C_1$-$C_7$)- Alkoxycarbonyl substituiertes Benzylsulfonyl, Benzylsulfinyl oder Benzylthio,
- Amino,
- ($C_1$-$C_4$)-Alkoxycarbonylamino,
- ($C_1$-$C_{12}$)-Alkanoyl, das durch Hydroxy, Amino und gegebenenfalls durch Phenyl oder Cyclohexyl substituiert ist,
- gegebenenfalls geschütztes aminosubstituiertes ($C_6$-$C_{10}$)-Aryl- oder ($C_3$-$C_{10}$)-Cycloalkyl-($C_1$-$C_4$)-alkyl oder ($C_1$-$C_8$)-Alkyl,
- ($C_1$-$C_{10}$)-Alkoxycarbonyl,
- substituiertes ($C_1$-$C_{10}$)-Alkoxycarbonyl,
- ($C_6$-$C_{14}$)-Aryl-($C_1$-$C_6$)-alkoxycarbonyl,
- 9-Fluorenylmethoxycarbonyl,
- 1-Desoxyhexoketosyl oder 1-Desoxypentoketosyl,
- Hexosyl oder Pentosyl,
- 6-Desoxyhexosyl,
- Aminozuckerreste,
- Lactosyl
- Maltosyl

wobei die verknüpften Zucker in der Pyranose- oder der Furanose-Form vorliegen können,

- Het,
- Het-carbonyl oder Het-sulfonyl,
- Het-($C_1$-$C_6$)-alkyl,
- Het-($C_1$-$C_6$)-alkanoyl,
- Het-($C_1$-$C_6$)-alkylsulfonyl oder
- Het-mercapto-($C_1$-$C_3$)-alkylcarbonyl,

wobei Het jeweils steht für

- Pyrrolyl,
- Imidazolyl,
- Pyridyl,
- Pyrimidyl,
- Pyrrolidyl,
- Piperidyl,
- Morpholino,
- Chinolyl oder
- Isochinolyl,

wobei dieser auch durch einen oder zwei gleiche oder verschiedene Reste aus der Gruppe ($C_1$-$C_4$)-Alkyl, ($C_1$-$C_4$)-Alkoxycarbonyl, ($C_1$-$C_4$)-Alkoxycarbonylamino, Hydroxy, Amino, Mono- oder Di-($C_1$-$C_4$)-Alkylamino substituiert sein kann;

$R^2$ und $R^{2*}$ unabhängig voneinander stehen für

- Wasserstoff,
- Carboxyl,
- Methyl, Ethyl, Isopropyl, n-Propyl, n-Butyl, Isobutyl, sec.-Butyl, n-Pentyl, n-Hexyl,

- Cyclohexyl,
- Cyclopentylmethyl, Cyclohexylmethyl, Cycloheptylmethyl,
- 4-Methylcyclohexylmethyl,
- 1-Dekahydronaphthylmethyl, 2-Dekahydronaphthylmethyl,
- Phenyl,
- Benzyl,
- 2-Phenylethyl,
- 1-Naphthylmethyl, 2-Naphthylmethyl,
- 2-Methylbenzyl, 3-Methylbenzyl, 4-Methylbenzyl,
- 2,4,6-Trimethylbenzyl,
- 4-tert.-Butylbenzyl,
- 4-tert.-Butoxybenzyl
- 4-Hydroxybenzyl,
- 4-Methoxybenzyl,
- 2,4-Dimethoxybenzyl,
- 3,4-Dihydroxybenzyl,
- 3,4-Dimethoxybenzyl,
- (Benzdioxolan-5-yl)methyl,
- 4-Chlorbenzyl,
- Hydroxymethyl,
- 1-Hydroxyethyl,
- 4-Pyridyl, 4-(N-Oxidopyridyl),
- 2-Pyridylmethyl, 3-Pyridylmethyl, 4-Pyridylmethyl, 2-(4-Pyridyl)ethyl,
- 2-Thienylmethyl, 3-Thienylmethyl,
- 2-(2-Thienyl)ethyl, 2-(3-Thienyl)ethyl,
- Indol-2-yl-methyl, Indol-3-yl-methyl,
- (1-Methyl-imidazol-4-yl)methyl,
- Imidazol-4-yl-methyl, Imidazol-1-yl-methyl,
- 2-Thiazolylmethyl,
- 3-Pyrazolylmethyl,
- 4-Pyrimidylmethyl,
- 2-Benzo[b]thienylmethyl, 3-Benzo[b]thienylmethyl,
- 2-Furylmethyl,
- 2-(Methylthio)ethyl,
- 2-(Methylsulfinyl)ethyl,
- 2-(Methylsulfonyl)ethyl,

$R^3$, $R^{3*}$, $R^4$, $R^{4*}$, $R^6$, $R^{6*}$, $R^{10}$ und $R^{10*}$
Wasserstoff bedeuten;
$R^5$ und $R^{5*}$ unabhängig voneinander stehen für
- Wasserstoff
- Hydroxy oder
- Amino;

$R^7$ - Wasserstoff,
- Hydroxy oder
- Methyl bedeutet;

$R^8$ und $R^{8*}$
unabhängig voneinander bedeuten
- Wasserstoff oder

zusammen mit $R^9$ bzw. $R^{9*}$ und den diese tragenden Atomen ein 1,2,3,4-Tetrahydroisochinolin oder 2-Azabicyclooctan-Gerüst bilden;
$R^9$ und $R^{9*}$
unabhängig voneinander
wie $R^2$ bzw. $R^{2*}$ definiert sind oder
- Hydroxy,
- Acetoxy,
- tert.-Butoxymethyl,
- 3-Guanidinopropyl,
- Carbamoylmethyl, Carbamoylethyl,

- Carboxymethyl, Carboxyethyl,
- Mercaptomethyl,
- (1-Mercapto-1-methyl)ethyl,
- Aminomethyl, 2-Aminoethyl, 3-Aminopropyl,
4-Aminobutyl,
- N,N,-Dimethylamino,
- N,N'-Di-(benzyloxycarbonyl)-guanidino-propyl,
- 2-Benzyloxycarbonylethyl, Benzyloxycarbonylmethyl
- tert.- Butylsulfonylmethyl oder
- 4-Benzylcarbonylaminobutyl bedeuten;

$R^{11}$ und $R^{11*}$ unabhängig voneinander
- Wasserstoff,
- Hydroxy oder
- Acetoxy bedeuten;

wobei
in den vorstehenden Verbindungen dieser Erfindung eine oder mehrere Amidgruppen (-CONH-) der Hauptkette ersetzt sein können durch $-CH_2NR_{14}-$ oder $-CH(OH)CH_2-$;
$R^{14}$ für
- Wasserstoff oder
- Methyl steht;

sowie deren physiologisch verträgliche Salze.

7. Verbindung der Formel I gemäß den Ansprüchen 1 bis 6, dadurch gekennzeichnet, daß
$R^1$ und $R^{1*}$
unabhängig voneinander stehen für
$a_1$) - Wasserstoff,
- Carboxyl,
- $(C_1-C_{16})$-Alkylsulfonyl,
- $(C_1-C_8)$-Mono- oder -Dihydroxyalkylsulfonyl,
- Mono-, Di- oder Trihydroxy-$(C_1-C_3)$-alkyl,
- $(C_1-C_8)$-Alkoxycarbonyl,
- $(C_1-C_{14})$-Alkanoyl,
- Amino-$(C_1-C_{12})$-alkanoyl
- $(C_6-C_{10})$-Aryl-$(C_1-C_4)$-alkoxycarbonyl,
- $(C_6-C_{10}$-Aryl-$(C_1-C_4)$-alkylcarbonyl,
- 9-Fluorenylmethoxycarbonyl,
- $(C_1-C_4)$-Alkanoyloxy-$(C_1-C_6)$-alkyl,
- 1,2-Diacetoxyethyl,
- 1,2,3-Triacetoxypropyl,
- Phenyl
- gegebenenfalls durch Halogen, Amino, $(C_1-C_4)$-Alkyl oder Methoxy substituiertes Benzolsulfonyl,
- gegebenenfalls durch Halogen, Amino $(C_1-C_4)$-Alkyl, oder Methoxy substituiertes Benzylsulfonyl, -sulfinyl oder -thio,
- Het oder Het-sulfonyl,
- Het-$(C_1-C_4)$-alkanoyl,
- Het-mercapto-$(C_1-C_3)$-alkylcarbonyl,

wobei Het jeweils steht für
- Pyrrolyl,
- Imidazolyl,
- Pyridyl,
- Pyrimidyl,
- Pyrrolidyl,
- Chinolyl,
- Isochinolyl,
- Piperidyl oder
- Morpholino,

wobei dieser Rest auch durch durch einen oder zwei gleiche oder verschiedene Reste aus der Gruppe Methyl, Amino und $(C_1-C_4)$-Alkoxycarbonylamino substituiert sein kann,
- Amino-$(C_3-C_6)$-Cycloalkylcarbonyl,

- $(C_1-C_8)$-Alkanoyl, das durch Hydroxy und Amino und gegebenenfalls durch Phenyl oder Cyclohexyl substituiert ist,
- gegebenenfalls geschütztes aminosubstituierte Phenyl-oder Cyclohexyl-$(C_1-C_6)$-alkyl,
- Amino,
- $(C_1-C_4)$-Alkoxycarbonylamino,
- Benzyloxycarbonylamino,
- 1-Desoxyhexoketosyl oder 1-Desoxypentoketosyl,
- Hexosyl oder Pentosyl,

wobei die verknüpften Zucker in der Pyranose- oder der Furanose-Form vorliegen können,
$R^2$ und $R^{2*}$ unabhängig voneinander stehen für
- Wasserstoff,

Methyl, Ethyl, Isopropyl, n-Propyl, n-Butyl, Isobutyl, sec.-Butyl, Pentyl, Hexyl,
- Cyclopentylmethyl, Cyclohexylmethyl,
- 4-Methylcyclohexylmethyl,
- Benzyl,
- 2-Phenylethyl,
- 1-Naphthylmethyl, 2-Naphthylmethyl,
- 2-Methylbenzyl, 3-Methylbenzyl, 4-Methylbenzyl,
- 2,4,6-Trimethylbenzyl,
- 4-tert.-Butylbenzyl,
- 4-Methoxybenzyl,
- 3,4-Dihydroxybenzyl,
- 2,4-Dimethoxybenzyl,
- 3,4-Dimethoxybenzyl,
- 2-Pyridylmethyl, 3-Pyridylmethyl, 4-Pyridylmethyl,
- 3,4-Methylendioxybenzyl oder
- 2-(4-Pyridyl)ethyl,

$R^3$, $R^{3*}$, $R^4$, $R^{4*}$, $R^6$, $R^{6*}$, $R^7$, $R^{10}$ und $R^{10*}$
Wasserstoff bedeuten;
$R^5$ und $R^{5*}$
unabhängig voneinander
- Wasserstoff oder
- Hydroxy bedeuten;

$R^8$ und $R^{8*}$ unabhängig voneinander wie in Anspruch 6 definiert sind;
$R^9$ und $R^{9*}$
unabhängig voneinander wie $R^9$ bzw. $R^{9*}$ in Anspruch 6 definiert sind;
$R^{11}$ und $R^{11*}$ unabhängig voneinander wie Anspruch 6 definiert sind,
sowie deren physiologisch verträgliche Salze.

8. Verbindung der Formel I gemäß den Ansprüchen 1 bis 7, dadurch gekennzeichnet, daß
Y für eine Rest der Formel III steht;
l 0 oder 1 bedeutet;
m 1 bedeutet;
A, A*, D und D* wie in Anspruch 1 definiert sind;
n, n*, o, o*, p und p* unabhängig voneinander 0 oder 1 bedeuten;
E, E*, F, F*, G und G* unabhängig voneinander für eine Aminosäure aus der Reihe Val, Lys, Lys(Z), Phe, Chg, Ser, Asn, Gly, Ile, Tbg, Nva oder Npg bedeuten;
$R^1$ und $R^{1*}$ unabhängig voneinander
- Wasserstoff,
- Carboxyl,
- Methylsulfonyl,
- tert.-Butylsulfonyl,
- tert.-Butoxycarbonyl,
- 2-Hydroxyethylsulfonyl,
- 1,2,3-Trihydroxypropyl,
- 1,2,3-Triacetoxypropyl,
- Benzyloxycarbonyl,
- 4-Methylphenylsulfonyl,
- 4-Chlorbenzylthio,

- Benzylsulfinyl,
- 4-Chlorbenzylsulfonyl,
- Hexadecylsulfonyl,
- 4-Amino-1-piperidyl-sulfonyl,
- N-tert.-butoxycarbonyl-4-amino-1-piperidyl-sulfonyl,
- 4-Amino-1-piperidyl-carbonyl,
- N-tert.-butoxycarbonyl-4-amino-1-piperidyl-carbonyl,
- 2-Amino-3-phenyl-propyl,
- N-tert.-Butoxycarbonyl-2-amino-3-phenyl-propyl,
- 2-Amino-1-hydroxy-4-methyl-pentyl,
- Desoxyfructos-1-yl,
- Mannofuranosyl,
- 4-Aminocyclohexylcarbonyl,
- 2-Pyridylacetyl,
- 4-Pyridylthio-acetyl,
- 2-Chinolylcarbonyl,
- 1-Naphthylacetyl,
- 1-Naphthyloxyacetyl,
- 1-(4-Pyridyl)ethylsulfonyl,
- 12-Aminododecanoyl,
- 4-(N-Oxidopyridyl),
- 4-Pyridyl,
- Tetradecanoyl,
- Phenyl,
- Amino oder
- tert.-Butoxycarbonylamino bedeuten;

$R^2$ und $R^{2*}$ unabhängig voneinander
- Wasserstoff,
- 2-(4-Pyridyl)ethyl,
- Isopropyl,
- Isobutyl,
- n-Pentyl,
- Benzyl,
- 3,4-Methylendioxybenzyl,
- 2,4-Dimethoxybenzyl,
- 4-tert.-Butylbenzyl,
- 2-Phenylethyl oder
- Cyclohexylmethyl bedeuten;

$R^3$, $R^{3*}$, $R^4$, $R^{4*}$, $R^6$, $R^{6*}$, $R^7$, $R^{10}$ und $R^{10*}$
- Wasserstoff

bedeuten;

$R^5$ und $R^{5*}$ unabhängig voneinander
- Wasserstoff oder
- Hydroxy bedeuten;

$R^8$ und $R^{8*}$
- Wasserstoff bedeuten, oder zusammen mit $R^9$ bzw. $R^{9*}$ und den diese tragenden Atomen ein 1,2,3,4-Tetrahydrochinolin-3,4-diyl-System bilden;

$R^9$ und $R^{9*}$ unabhängig voneinander
- Wasserstoff,
- Hydroxy,
- Acetoxy,
- n-Propyl,
- Isopropyl,
- Isobutyl,
- Aminomethyl,
- 4-Aminobutyl,
- Hydroxymethyl,
- tert.-Butoxymethyl,

- Aminocarbonylmethyl,
- 2-Benzyloxycarbonyl-ethyl,
- 4-Benzyloxycarbonylamino-butyl,
- N,N°-Di-(benzyloxycarbonyl)-guanidino-propyl,
- Cyclohexyl,
- Cyclohexylmethyl,
- Benzyl,
- 2-Phenyl-ethyl
- 4-Hydroxy-benzyl,
- 4-Methoxy-benzyl,
- 4-tert.-Butoxy-benzyl,
- 1-Naphthylmethyl,
- 2-Thienylmethyl,
- 1-Imidazolyl-methyl,
- 3-Indolyl-methyl,
- 4-Pyridylmethyl
- 4-(N-Oxidopyridyl)methyl
- 2-Methylthio-ethyl,
- 2-Methylsulfonyl-ethyl
- tert.-Butylsulfonyl-methyl oder
- 2-Carboxyl-ethyl bedeuten;

$R^{11}$ und $R^{11*}$ unabhängig voneinander
- Wasserstoff,
- Hydroxy oder
- Acetoxy bedeuten;

wobei in den vorstehenden Verbindungen eine oder mehrere Amidgruppen (-CONH-) der Haupkette ersetzt sein können durch $-CH_2NH-$ oder $-CH(OH)CH_2-$;
sowie deren physiologisch verträgliche Salze.

9. Verbindung der Formel I gemäß den Ansprüchen 1 bis 8, dadurch gekennzeichnet, daß
$l = 0$;
$m = 1$;
$n + o + p = 1$;
D und D* für einen der Formel VI bzw. VI* stehen;
$R^1$ und $R^{1*}$
- ($C_1$-$C_{12}$)-Alkylsulfonyl, das gegebenenfalls durch bis zu 3 gleiche oder verschiedene Resten aus der Reihe
- Hydroxy,
- Amino oder
- Carboxy substituiert sein kann, bedeuten;

$R^2$ und $R^{2*}$ unabhängig voneinander
- Wasserstoff,
- Carboxyl,
- Methyl, Ethyl, Isopropyl, n-Propyl, n-Butyl, Isobutyl, sec.-Butyl, Pentyl, Hexyl,
- Cyclohexyl,
- Cyclopentylmethyl, Cyclohexylmethyl, Cycloheptylmethyl,
- 4-Methylcyclohexylmethyl,
- 1-Dekahydronaphthylmethyl, 2-Dekahydronaphthylmethyl,
- Phenyl,
- Benzyl,
- 2-Phenylethyl,
- 1-Naphthylmethyl, 2-Naphthylmethyl,
- 2-Methylbenzyl, 3-Methylbenzyl, 4-Methylbenzyl,
- 2,4,6-Trimethylbenzyl,
- 4-tert.-Butylbenzyl,
- 4-tert.-Butoxybenzyl
- 4-Hydroxybenzyl,
- 4-Methoxybenzyl,
- 2,4-Dimethoxybenzyl,

- 3,4-Dihydroxybenzyl,
- 3,4-Dimethoxybenzyl,
- (Benzdioxolan-4-yl)methyl,
- 4-Chlorbenzyl,
- Hydroxymethyl,
- 1-Hydroxyethyl,
- 2-Pyridylmethyl, 3-Pyridylmethyl, 4-Pyridylmethyl, 2-(4-Pyridyl)ethyl,
- 2-Thienylmethyl, 3-Thienylmethyl,
- 2-(2-Thienyl)ethyl, 2-(3-Thienyl)ethyl,
- Indol-2-yl-methyl, Indol-3-yl-methyl,
- (1-Methyl-imidazol-4-yl)methyl,
- Imidazol-4-yl-methyl, Imidazol-1-yl-methyl,
- 2-Thiazolylmethyl,
- 3-Pyrazolylmethyl,
- 4-Pyrimidylmethyl,
- 2-Benzo[b]thienylmethyl, 3-Benzo[b]thienylmethyl,
- 2-Furylmethyl,
- 2-(Methylthio)ethyl,
- 2-(Methylsulfinyl)ethyl oder
- 2-(Methylsulfonyl)ethyl bedeuten;

$R^3$, $R^{3*}$, $R^4$, $R^{4*}$, $R^6$, $R^{6*}$, $R^{11}$ und $R^{11*}$
- Wasserstoff bedeuten;

$R^5$ und $R^{5*}$
- Hydroxy bedeuten;

$R^9$ und $R^{9*}$
wie in Anspruch 8 definiert sind;
sowie deren physiologisch verträgliche Salze.

10. Verfahren zur Herstellung einer Verbindung der Formel I gemäß den Ansprüchen 1 bis 9, dadurch gekennzeichnet, daß man ein Fragment mit endständiger Carboxylgruppe oder dessen reaktives Derivat mit einem entsprechenden Fragment mit freier Aminogruppe kuppelt, gegebenenfalls zum Schutz weiterer funktioneller Gruppen (eine) temporär eingeführte Schutzgruppe(n) abspaltet und die so erhaltene Verbindung gegebenenfalls in ihr physiologisch verträgliches Salz überführt.

11. Verwendung einer Verbindung der Formel I gemäß den Ansprüchen 1 bis 9 als Heilmittel.

12. Verwendung einer Verbindung der Formel I gemäß den Ansprüchen 1 bis 9 zur Hemmung retroviraler Proteasen.

13. Verwendung einer Verbindung der Formel I gemäß den Ansprüchen 1 bis 9 bei der Behandlung des "erworbenen Immunschwäche-Syndroms".

14. Pharmazeutisches Mittel enthaltend eine Verbindung der Formel I gemäß den Ansprüchen 1 bis 9, sowie gegebenenfalls einen oder mehrere Träger.

Patentansprüche für folgende Vertragsstaaten: ES, GR

1. Verfahren zur Herstellung einer Verbindung der Formel I

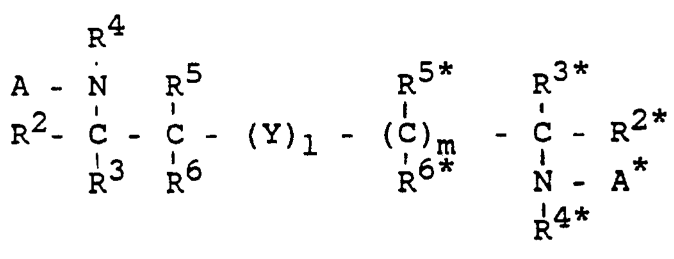

(I)

worin

Y für Sauerstoff, Schwefel, einen Rest der Formel II oder einen Rest der Formel III

$$-\underset{R^{6**}}{\overset{R^{5**}}{C}}- \quad (II) \qquad\qquad -\underset{R^{7}}{N}- \quad (III)$$

steht;
l und m unabhängig voneinander 0 oder 1 sind;
A einen Rest der Formel IV und A* einen Rest der Formel IV* bedeuten

$D - (E)_n - (F)_o - (G)_p -$ (IV)

$D^* - (E^*)_{n^*} - (F^*)_{o^*} - (G^*)_{p^*} -$ (IV*)

wobei
E, E*, F, F*, G und G* unabhängig voneinander für eine natürliche oder unnatürliche Aminosäure, Azaaminosäure oder Iminosäure stehen;
n, n*, o, o*, p und p* unabhängig voneinander 0 oder 1 bedeuten;
D für $R^1$ oder einen Rest der Formel V, VI oder VII und
D* für $R^{1*}$ oder eien Rest der Formeln V*, VI* oder VII* steht

$$R^1 - \overset{R^8}{N} - \underset{R^{10}}{\overset{R^9}{C}} - CO - \quad (V) \qquad R^{1*} - \overset{R^{8*}}{N} - \underset{R^{10*}}{\overset{R^{9*}}{C}} - CO - \quad (V^*)$$

$$R^1 - \overset{R^{11}}{CH} - \overset{R^9}{CH} - CO - \quad (VI) \qquad R^{1*} - \overset{R^{11*}}{CH} - \overset{R^{9*}}{CH} - CO - \quad (VI^*)$$

$$R^1 - O - \overset{R^9}{CH} - CO - \quad (VII) \qquad R^{1*} - O - \overset{R^{9*}}{CH} - CO - \quad (VII^*)$$

und worin $R^1$ und $R^{1*}$ unabhängig voneinander stehen für
$a_1$)
- Wasserstoff,
- Carboxyl,
- $(C_1-C_{18})$-Alkyl, das gegebenenfalls einfach oder zweifach ungesättigt ist und das gegebenenfalls durch bis zu 3 gleiche oder verschiedene Reste aus der Reihe
- Mercapto,
- Hydroxy,
- $(C_1-C_7)$-Alkoxy,
- Carbamoyl,
- $(C_1-C_8)$-Alkanoyloxy,
- Carboxy,
- $(C_1-C_7)$-Alkoxycarbonyl.
- F, Cl, Br, I,
- Amino,
- Amidino, das gegebenenfalls durch einen, zwei oder drei $(C_1-C_8)$-Alkylreste substituiert sein kann,
- Guanidino, das gegebenenfalls durch einen oder zwei Benzyloxycarbonylreste oder durch einen, zwei, drei oder vier $(C_1-C_8)$-Alkylreste substituiert sein kann,
- $(C_1-C_7)$-Alkylamino,
- Di-$(C_1-C_7)$-Alkylamino,
- $(C_1-C_6)$-Alkoxycarbonylamino,
- $(C_7-C_{15})$-Aralkoxycarbonyl,
- $(C_7-C_{15})$-Aralkoxycarbonylamino,
- Phenyl-$(C_1-C_4)$-alkoxy,

- 9-Fluorenylmethoxycarbonylamino,
- ($C_1$-$C_6$)-Alkylsulfonyl,
- ($C_1$-$C_6$)-Alkylsulfinyl,
- ($C_1$-$C_6$)-Alkylthio,
- Hydroxamino,
- Hydroximino,
- Sulfamoyl,
- Sulfo,
- Carboxamido,
- Formyl,
- Hydrazono,
- Imino,
- einen Rest $CONR^{12}R^{13}$ bzw. $CONR^{12*}R^{13*}$,
- durch bis zu sechs Hydroxy oder
- durch bis zu fünf ($C_1$-$C_8$)-Alkanoyloxy substituiert ist;
- mono-, bi oder tricyclisches ($C_3$-$C_{18}$)-Cycloalkyl,
- ($C_3$-$C_{18}$)-Cycloalkyl-($C_1$-$C_6$)-alkyl wobei der Cycloalkylteil jeweils gegebenenfalls durch einen oder zwei gleiche oder verschiedene Reste aus der Reihe
- F, Cl, Br, I,
- Carboxy,
- Carbamoyl,
- Carboxymethoxy,
- Hydroxy,
- ($C_1$-$C_7$)-Alkoxy,
- ($C_1$-$C_7$)-Alkyl,
- ($C_1$-$C_7$)-Alkyloxycarbonyl,
- Amino,
- ($C_1$-$C_6$)-Alkylamino-($C_1$-$C_6$)-alkyl,
- Di-($C_1$-$C_6$)-Alkylamino-($C_1$-$C_6$)-alkyl,
- Amidino,
- Hydroxamino,
- Hydroximino,
- Hydrazono,
- Imino,
- Guanidino,
- ($C_1$-$C_6$)-Alkoxysulfonyl,
- ($C_1$-$C_6$)-Alkoxysulfinyl,
- ($C_1$-$C_6$)-Alkoxycarbonylamino,
- ($C_6$-$C_{12}$)-Aryl-($C_1$-$C_4$)-Alkoxycarbonylamino,
- ($C_1$-$C_7$)-Alkylamino,
- Di-($C_1$-$C_7$)-alkylamino und
- Trifluormethyl

substituiert ist;
- ($C_6$-$C_{14}$)-Aryl,
- ($C_6$-$C_{14}$)-Aryl-($C_1$-$C_6$)-alkyl,
- ($C_6$-$C_{14}$)-Aryloxy-($C_1$-$C_6$)-alkyl oder
- ($C_6$-$C_{14}$)-Aryl-($C_3$-$C_8$)-cycloalkyl, worin der Arylteil jeweils gegebenenfalls durch einen, zwei oder drei gleiche oder verschiedene Reste aus der Reihe
- F, Cl, Br, I,
- Hydroxy,
- Mono-, Di- oder Trihydroxy-($C_1$-$C_4$)-alkyl,
- Trifluormethyl,
- Formyl,
- Carboxamido,
- Mono- oder Di-($C_1$-$C_4$)-alkylaminocarbonyl,
- Nitro,
- ($C_1$-$C_7$)-Alkoxy,
- ($C_1$-$C_7$)-Alkyl,

- $(C_1-C_7)$-Alkoxycarbonyl,
- Amino,
- $(C_1-C_7)$-Alkylamino,
- Di-$(C_1-C_7)$-alkylamino,
- Carboxy,
- Carboxymethoxy,
- Amino-$(C_1-C_7)$-alkyl,
- $(C_1-C_7)$-Alkylamino-$(C_1-C_7)$-alkyl,
- Di-$(C_1-C_7)$-alkylamino-$(C_1-C_7)$-alkyl,
- $(C_1-C_7)$-Alkoxycarbonylmethoxy,
- Carbamoyl,
- Sulfamoyl,
- $(C_1-C_7)$-Alkoxysulfonyl,
- $(C_1-C_8)$-Alkylsulfonyl,
- Sulfo-$(C_1-C_8)$-alkyl,
- Guanidino-$(C_1-C_8)$-alkyl und
- $(C_1-C_6)$-Alkoxycarbonylamino

substituiert ist;

- Het,
- Het-$(C_1-C_6)$-alkyl,
- Het-$(C_3-C_8)$-cycloalkyl,
- Het-$(C_3-C_8)$-cycloalkyl-$(C_1-C_4)$-alkyl,
- Het-$(C_3-C_8)$-cycloalkoxy-$(C_1-C_4)$-alkyl,
- Het-thio-$(C_1-C_6)$-alkyl,
- Het-thio-$(C_3-C_8)$-cycloalkyl,
- Het-thio-$(C_3-C_8)$-cycloalkyl-$(C_1-C_4)$-alkyl,

wobei Het jeweils für den Rest eines 5- bis 7-gliedrigen monocyclischen oder 8- bis 10-gliedrigen bicyclischen Ringsystems steht, das benzanelliert, aromatisch, teilhydriert oder vollständig hydriert sein kann, das als Heteroelemente einen, zwei, drei oder vier verschiedene Reste aus der Gruppe N, O, S, NO, SO, $SO_2$ enthalten kann, das mit 1 bis 6 Hydroxy substituiert sein kann und das gegebenenfalls wie bei $(C_6-C_{14})$-Aryl und $a_1$) definiert und/oder mit Oxo, mono-, di- oder trisubstituiert ist,

oder einen Rest $NR^{12}R^{13}$ bzw. $NR^{12*}R^{13*}$ bedeuten oder,

$a_2$)

- einen Rest der Formel VIII beziehungsweise VIII* bedeuten

$R^{1a}$ - W (VIII)

$R^{1a*}$ - W * (VIII*)

worin $R^{1a}$ und $R^{1a*}$ wie $R^1$ bzw. $R^{1*}$ unter $a_1$) definiert sind und W bzw. W* für -CO-, -CS-, O-CO-, $-SO_2-$, -SO-, -S-, $-NHSO_2-$, -NHCO-, -CH(OH)-, -N(OH)- oder -CO-V- wobei V ein Peptid mit 1 bis 10 Aminosäuren bedeutet, steht;

oder worin $R^1$ und $R^{1*}$ unabhängig voneinander zusammen mit $R^{11}$ bzw. $R^{11*}$ und den diese tragenden Atomen mono- oder bicyclische, gesättigte oder teilweise ungesättigte Ringsysteme mit 5-12 Ringgliedern bilden, die außer Kohlenstoff noch 1 Schwefelatom enthalten können, welches gegebenenfalls zum Sulfoxid oder Sulfon oxidiert sein kann;

$a_3$)

- einen Glycosylrest, bevorzugt einen Glucofuranosyl oder Glucopyranosyl-Rest steht, der sich von natürlich vorkommenden Aldotetrosen, Aldopentosen, Aldohexosen, Ketopentosen, Ketohexosen, Desoxyaldosen, Aminoaldosen, und Oligosacchariden sowie deren Stereoisomeren ableitet;

$R^2$ und $R^{2*}$

unabhängig voneinander definiert sind wie $R^1$ bzw. $R^{1*}$ unter $a_1$) oder $a_2$) oder zusammen mit $R^4$ bzw. $R^{4*}$ und den diese tragenden Atomen mono- oder bicyclische, gesättigte oder teilweise ungesättigte Ringsysteme mit 5 bis 12 Ringgliedern bilden, oder zusammen mit $R^3$ bzw. $R^{3*}$ und den diese tragenden Atomen cyclische, gesättigte oder teilweise ungesättigte Ringsysteme mit 3 bis 12 Ringgliedern bilden;

$R^3$ und $R^{3*}$

unabhängig voneinander

- Wasserstoff oder
- $(C_1-C_3)$-Alkyl bedeuten;

$R^4$ und $R^{4*}$

unabhängig voneinander
- Wasserstoff oder
- $(C_1-C_8)$-Alkyl bedeuten;
$R^5$ $R^{5*}$ und $R^{5**}$
unabhängig voneinander
- Wasserstoff,
- Hydroxy,
- Amino oder
- Carboxy bedeuten, oder
mit $R^6$, $R^{6*}$ bzw. $R^{6**}$ zusammen mit den sie tragenden Kohlenstoffatomen jeweils unabhängig voneinander eine Ketogruppe bilden;
$R^6$, $R^{6*}$ und $R^{6**}$
unabhängig voneinander
- Wasserstoff oder
- $(C_1-C_6)$-Alkyl bedeuten oder
im Falle $l=0$, $R^6$ und $R^{6*}$ gegebebebfalls eine gemeinsame Bindung ausbilden können;
$R^7$
- Wasserstoff,
- Hydroxy oder
- $(C_1-C_6)$-Alkyl bedeutet;
$R^8$ und $R^{8*}$
unabhängig voneinander
- Wasserstoff oder
- $(C_1-C_8)$-Alkyl bedeuten, oder zusammen mit $R^9$ bzw. $R^{9*}$ und den diese tragenden Atomen mono- oder bicyclische, gesättigte oder teilweise ungesättigte Ringsysteme mit 5-12 Ringgliedern bildern;
$R^9$ und $R^{9*}$
unabhängig voneinander definiert sind wie $R^1$ bzw. $R^{1*}$ unter $a_1$), für Hydroxy oder $(C_1-C_4)$-Alkanoyloxy stehen oder zusammen mit $R^{10}$ bzw. $R^{10*}$ und den diese tragenden Atomen cyclische, gesättigte oder teilweise ungesättigte Ringsysteme mit 3 bis 12 Ringgliedern bildern;
oder
zusammen mir $R^{11}$ bzw. $R^{11*}$ und den diese tragenden Atomen ein mono- oder bicyclisches, gesättigtes oder teilweise ungesättigtes Ringsystem mit 5-12 Ringgliedern bilden, das außer Kohlenstoff noch 1 Schwefelatom enthalten kann,
welches gegebenenfalls zum Sulfoxid oder Sulfon oxidiert sein kann; oder 1 Stickstoffatom enthalten kann, wobei das Ringsystem gegebenenfalls durch Amino substituiert sein kann;
$R^{10}$ und $R^{10*}$
unabhängig voneinander
- Wasserstoff oder
- $(C_1-C_6)$-Alkyl bedeuten;
$R^{11}$ und $R^{11*}$
unabhängig voneinander
- Wasserstoff,
- Hydroxy,
- $(C_1-C_4)$-Alkanoyloxy oder
- $(C_1-C_8)$-Alkyl bedeuten;
$R^{12}$, $R^{12*}$, $R^{13}$ und $R^{13*}$
unabhängig voneinander
- Wasserstoff,
- $(C_1-C_8)$-Alkyl, das durch
- Amino,
- $(C_1-C_4)$-Alkylamino,
- Di-$(C_1-C_4)$-alkylamino,
- Mercapto,
- Carboxy,
- Hydroxy oder
- $(C_1-C_4)$-Alkoxy substituiert sein kann,
- $(C_3-C_7)$-Cycloalkyl,
- $(C_1-C_4)$-Alkoxycarbonyl,

- $(C_6-C_{14})$-Aryl, $(C_6-C_{14})$-Aryl-$(C_1-C_4)$-Alkoxycarbonyl, die im Arylteil wie bei $R^1$ bzw. $R^{1*}$ beschrieben, substituiert sein können,
- Het oder
- Het-$(C_1-C_4)$-alkyl, wobei Het wie bei $R^1$ bzw. $R^{1*}$ beschrieben definiert ist, bedeuten

oder wobei $R^{12}$ und $R^{13}$ bzw. $R^{12*}$ und $R^{13*}$ zusammen mit dem sie tragenden Stickstoffatomen monocyclische oder bicyclische, gesättigte, teilweise ungesättigte oder aromatische Ringsysteme bilden, die als weitere Ringglieder neben Kohlenstoff noch 1 oder 2 Stickstoffatome, 1 Schwefelatom oder 1 Sauerstoffatom enthalten und durch $(C_1-C_4)$-Alkyk substituiert sein können,

wobei

in den vorstehenden Verbindungen der Formel I eine oder mehrere Amidgruppen (-CONH-) der Hauptkette ersetzt sein können durch $-CH_2NR^{14}-$, $-CH_2S-$, $-CH_2O-$, $-OCH_2-$, $-CH_2CH_2-$, -CH=CH-(cis und trans), $-COCH_2-$, $-CH(OH)CH_2-$, $-CH_2SO-$, $-CH_2SO_2-$, -COO-, $P(O)(OR^{15})CH_2-$ und $-P(O)(OR^{15})NH-$, oder auch durch eine Amidgruppe mit umgekehrter Polarität (-NHCO-);

worin $R^{14}$ und $R^{15}$

unabhängig voneinander stehen für
- Wasserstoff oder
- $(C_1-C_4)$-Alkyl;

sowie deren physiologisch verträgliche Salze, dadurch gekennzeichnet, daß man ein Fragment mit endständiger Carboxylgruppe oder dessen reaktives Derivat mit einem entsprechenden Fragment mit freier Aminogruppe kuppelt, gegebenenfalls zum Schutz weiterer funktioneller Gruppen (eine) temporär eingeführte Schutzgruppe(n) abspaltet und die so erhaltene Verbindung gegebenenfalls in ihr physiologisch verträgliches Salz überführt.

2. Verfahren zur Herstellung einer Verbindung der Formel I gemäß Anspruch 1, dadurch gekennzeichnet, daß die Reste und Symbole mit und ohne Stern jeweils identisch sind.

3. Verfahren zur Herstellung einer Verbindung der Formel I gemäß den Ansprüchen 1 bis 2, dadurch gekennzeichnet, daß die Verbindung $C_2$-symmetrisch ist.

4. Verfahren zur Herstellung einer Verbindung der Formel I gemäß den Ansprüchen 1 bis 3, dadurch gekennzeichnet, daß Y für einen Rest der Formel II oder einen Rest der Formel III steht;

l, m, A, A*, D, D*, n, n*, o, o*, p und p* wie in Anspruch 1 definiert sind;

E, E*, F, F*, G und G* unabhängig voneinander für eine natürliche oder unnatürliche α-Aminosäure oder α-Iminosäure steht;

$R^1$ und $R^{1*}$

unabhängig voneinander stehen für

$a_1$) - Wasserstoff;
- Carboxyl,
- $(C_1-C_{16})$-Alkyl, das gegebenenfalls einfach ungesättigt ist und das gegebenenfalls durch bis zu 2 gleiche oder verschiedene Reste aus der Reihe
- Hydroxy,
- $(C_1-C_4)$-Alkoxy,
- Carbamoyl,
- $(C_1-C_8)$-Alkanoyloxy,
- Carboxy,
- $(C_1-C_4)$-Alkoxycarbonyl,
- F,
- Amino,
- $(C_1-C_7)$-Alkylamino,
- Di-$(C_1-C_7)$-Alkylamino,
- $(C_1-C_6)$-Alkoxycarbonylamino,
- Benzyloxycarbonyl
- Benzyloxycarbonylamino,
- 9-Fluorenylmethoxycarbonylamino,
- $(C_1-C_4)$-Alkylsulfonyl,
- einen Rest $CONR^{12}R^{13}$ bzw. $CONR^{12*}R^{13*}$,
- durch bis zu sechs Hydroxy oder
- durch bis zu vier $(C_1-C_8)$Alkanoyloxy substituiert ist;
- mono- oder bicyclisches $(C_3-C_{12})$-Cycloalkyl,
- $(C_3-C_{12})$-Cycloalkyl-$(C_1-C_6)$-alkyl wobei der Cycloalkylteil jeweils gegebenenfalls durch einen oder zwei gleiche oder verschiedene Reste aus der Reihe,

- F,
- Carboxy,
- Hydroxy,
- $(C_1-C_7)$-Alkoxy,
- $(C_1-C_4)$-Alkyl,
- $(C_1-C_4)$-Alkyloxycarbonyl,
- Amino,
- $(C_1-C_6)$-Alkoxycarbonylamino,
- Benzyloxycarbonylamino
- $(C_1-C_4)$-Alkylamino und
- Di-$(C_1-C_4)$-alkylamino

substituiert ist;
- $(C_6-C_{10})$-Aryl,
- $(C_6-C_{10})$-Aryloxy-$(C_1-C_6)$-alkyl oder
- $(C_6-C_{10})$-Aryl-$(C_1-C_6)$-alkyl, worin der Arylteil jeweils gegebenenfalls durch einen, zwei oder drei gleiche oder verschiedene Reste aus der Reihe
- F, Cl, Br,
- Hydroxy,
- Hydroxy-$(C_1-C_4)$-alkyl,
- Carboxamido,
- Mono- oder Di-$(C_1-C_4)$-alkylaminocarbonyl,
- $(C_1-C_4)$-Alkoxy,
- $(C_1-C_4)$-Alkyl,
- $(C_1-C_4)$-Alkoxycarbonyl,
- Amino,
- $(C_1-C_4)$-Alkylamino,
- Di-$(C_1-C_4)$-alkylamino,
- Carboxy,
- Carbamoyl,
- $(C_1-C_4)$-Alkoxycarbonylamino

substituiert ist;
- Het,
- Het-$(C_1-C_6)$-alkyl,
- Het-$(C_5-C_6)$-cycloalkyl
- Het-thio-$(C_1-C_4)$-alkyl,
- Het-thio-$(C_5-C_6)$-cycloalkyl,

wobei Het jeweils für den Rest eines 5- bis 6-gliedrigen monocyclischen oder 8- bis 10-gliedrigen bicyclischen Ringsystems steht, das aromatisch, teilhydriert oder vollständig hydriert sein kann, das als Heteroelemente einen, zwei, drei oder vier verschiedene Reste aus der Gruppe N, O, S, NO, SO, $SO_2$ enthalten kann, das mit 1 bis 4 Hydroxy substituiert sein kann und das gegebenenfalls wie bei $(C_6-C_{10})$-Aryl unter $a_1$) definiert und/oder mit Oxo mono-, oder di-substituiert ist,

oder einen Rest $NR^{12}R^{13}$ bzw. $NR^{12*}R^{13*}$ bedeutet oder,

$a_2$) - einen Rest der Formel VIII beziehungsweise VIII* bedeuten

$R^{1a}$ - W (VIII)

$R^{1a*}$ - W* (VIII*)

worin $R^{1a}$ und $R^{1a*}$ wie $R^1$ bzw. $R^{1*}$ unter $a_1$) definiert sind und W bzw. W* für -CO-, -O-CO-, $-SO_2-$, -SO-, -S-, -NHCO- oder -CH(OH)-, steht;

oder worin $R^1$ und $R^{1*}$ unabhängig voneinander zusammen mit $R^{11}$ bzw. $R^{11*}$ und den diese tragenden Atomen monocyclische, gesättigte oder teilweise ungesättigte Ringsysteme mit 5-8 Ringgliedern bilden, die außer Kohlenstoff noch 1 Schwefelatom enthalten können, welches gegebenenfalls zum Sulfoxid oder Sulfon oxidiert sein kann;

$a_3$) - einen Glycosylrest, der wie in Anspruch 1 definiert ist;

$R^2$ und $R^{2*}$

unabhängig voneinander

$b_1$) - Wasserstoff,
- Carboxy,
- $(C_1-C_{10})$-Alkyl, das gegebenenfalls ein- oder zweifach ungesättigt ist und das gegebenenfalls durch bis zu 3 gleiche oder verschiedene Reste aus der Reihe,

- Hydroxy,
- ($C_1$-$C_7$)-Alkoxy,
- ($C_1$-$C_7$)-Alkylthio,
- ($C_1$-$C_7$)-Alkylsulfinyl,
- ($C_1$-$C_7$)-Alkylsulfonyl,
- ($C_1$-$C_7$)-Alkanoyloxy,
- Carboxy,
- ($C_1$-$C_7$)-Alkoxycarbonyl,
- Cl, Br,
- Amino,
- Amidino,
- Guanidino,
- N,N′-Di-(benzyloxycarbonyl)-guanidino,
- Carbamoyl,
- ($C_7$-$C_{15}$)-Aralkoxycarbonyl,
- ($C_1$-$C_5$)-Alkoxycarbonylamino,
- ($C_7$-$C_{15}$)-Aralkoxycarbonylamino oder
- 9-Fluorenylmethoxycarbonylamino substituiert ist;
- ($C_3$-$C_{12}$)-Cycloalkyl,
- ($C_3$-$C_{12}$)-Cycloalkyl-($C_1$-$C_3$)-alkyl,
- ($C_6$-$C_{14}$)-Aryl,
- ($C_6$-$C_{14}$)-Aryl-($C_1$-$C_3$)-alkyl, wobei der Aryl-Teil jeweils gegebenenfalls durch einen, zwei oder drei gleiche oder verschiedene Reste aus der Reihe
- F, Cl, Br, I,
- Hydroxy,
- ($C_1$-$C_7$)-Alkoxy,
- ($C_1$-$C_7$)-Alkyl,
- ($C_1$-$C_7$)-Alkoxycarbonyl;
- Amino und
- Trifluormethyl substituiert ist; oder
- Het-($C_1$-$C_6$)-alkyl, wobei Het für den Rest eines 5-oder 6-gliedrigen monocyclischen oder 9- bis 10-gliedrigen bicyclischen, gegebenenfalls teilweise oder vollständig hydrierten Heteroaromaten, mit mindestens 1 C-Atom, 1-4 N-Atomen und/oder 1-2 S-Atomen und/oder 1-2 O-Atomen als Ringglieder, der gegebenenfalls wie in Anspruch 1 für den Arylteil beschrieben mono- oder disubstituiert ist, bedeuten;

oder

$b_2$) zusammen mit $R^4$ bzw. $R^{4*}$ und den diese tragenden Atomen Pyrrolidin oder Piperidin, die jeweils noch mit Cyclopentyl, Cyclohexyl oder Phenyl annelliert sein können, bilden,

oder zusammen mit $R^3$ bzw. $R^{3*}$ und den diese tragende Atome cyclische, gesättigte oder teilweise ungesättigte Ringsysteme mit 3-8 Ringgliedern bilden;

$R^3$ und $R^{3*}$

unabhängig voneinander
- Wasserstoff,
- Methyl oder
- Ethyl bedeuten;

$R^4$ und $R^{4*}$

unabhängig voneinander
- Wasserstoff,
- ($C_1$-$C_4$)-Alkyl bedeuten;

$R^5$, $R^{5*}$ und $R^{5**}$ unabhängig voneinander wie in Anspruch 1 definiert sind;

$R^6$, $R^{6*}$ und $R^{6**}$

unabhängig voneinander
- Wasserstoff,
- ($C_1$-$C_4$)-Alkyl bedeuten;

$R^7$
- Wasserstoff,
- Hydroxy oder
- ($C_1$-$C_4$)-Alkyl bedeutet;

$R^8$ und $R^{8*}$

unabhängig voneinander
- Wasserstoff,
- $(C_1-C_8)$-Alkyl bedeuten oder
zusammen mit $R^9$ bzw. $R^{9*}$ und den diese tragenden Atomen Pyrrolidin oder Piperidin, die jeweils zusätzlich mit Cyclopentyl, Cyclohexyl oder Phenyl annelliert sein können, bilden;
$R^9$ und $R^{9*}$
unabhängig voneinander definiert sind wie $R^2$ bzw. $R^{2*}$ unter $b_1$), oder
$(C_1-C_8)$-Alkanoyloxy bedeutet oder
zusammen mit $R^{10}$ bzw. $R^{10*}$ und den diese tragenden Atomen cyclische, gesättigte oder teilweise ungesättigten Ringsysteme mit 5 bis 12 Ringgliedern bilden;
oder
zusammen mit $R^{11}$ bzw. $R^{11*}$ und den diese tragenden Atomen ein mono- oder bicyclisches, gesättigtes oder teilweise ungesättigtes Ringsystem mit 5-12 Ringgliedern bilden, das außer Kohlenstoff noch 1 Schwefelatom enthalten kann, welches gegebenenfalls zum Sulfoxid oder Sulfon oxidiert sein kann;
$R^{10}$ und $R^{10*}$
unabhängig voneinander
- Wasserstoff oder
- $(C_1-C_4)$-Alkyl bedeuten;
$R^{11}$ und $R^{11*}$
unabhängig voneinander
- Wasserstoff,
- Hydroxy,
- $(C_1-C_4)$-Alkanoyloxy oder
- $(C_1-C_4)$-Alkyl bedeuten;
$R^{12}$, $R^{12*}$, $R^{13}$ und $R^{13*}$
unabhängig voneinander
- Wasserstoff,
- $(C_1-C_8)$-Alkyl, das durch
- Amino,
- $(C_1-C_4)$-Alkylamino,
- Di-$(C_1-C_4)$-alkylamino,
- Carboxy,
- Hydroxy oder
- $(C_1-C_4)$-Alkoxy substituiert sein kann,
- $(C_1-C_4)$-Alkoxycarbonyl,
- $(C_6-C_{10})$-Aryl, das wie bei $R^1$ bzw. $R^{1*}$ beschrieben substituiert sein kann,
- $(C_6-C_{10})$-Aryl-$(C_1-C_4)$-alkoxycarbonyl,
- Het oder
- Het-$(C_1-C_4)$-alkyl, wobei Het wie bei $R^1$ bzw. $R^{1*}$ beschrieben definiert ist,
wobei
in den vorstehenden Verbindungen der Formel I eine oder mehrere Amidgruppen (-CONH)- der Hauptkette ersetzt sein können durch eine Gruppe bestehend aus $-CH_2NR^{14}-$, $-CH_2O-$, $-OCH_2-$, $-CH_2CH_2-$, $-COCH_2-$, $-CH(OH)CH_2-$, -COO- oder auch durch eine Amidgruppe mit umgekehrter Polarität (-NHCO-);
$R^{14}$ für
- Wasserstoff oder
- $(C_1-C_4)$-Alkyl steht;
sowie deren physiologisch verträgliche Salze.

5. Verfahren zur Herstellung einer Verbindung der Formel I gemäß den Ansprüchen 1 bis 4, dadurch gekennzeichnet, daß Y für einen Rest der Formel II oder einen Rest der Formel III steht;
l, m, A, A*, D, D*, n, n*, o, o* wie in Anspruch 1 definiert sind,
p und p* für 1 stehen;
$R^1$ und $R^{1*}$
unabhängig voneinander stehen für
- Wasserstoff,
- Carboxyl,
- $(C_1-C_{10})$-Alkyl,
- $(C_3-C_8)$-Cycloalkyl
- $(C_3-C_8)$-Cycloalkyl-$(C_1-C_{10})$-alkyl

- Phenyl-($C_1$-$C_8$)-alkyl, das wie in Anspruch 4 im Phenylteil substituiert sein kann,
- gegebenenfalls geschütztes Mono- oder Di-Amino-($C_1$-$C_{12}$)-alkyl oder Amino-($C_6$-$C_{10}$)-aryl-($C_1$-$C_4$)-alkyl oder Amino-($C_3$-$C_{10}$)-cycloalkyl-($C_1$-$C_4$)-alkyl,
- Mono-, Di-, Tri-, Tetra-, Penta- oder Hexahydroxy-($C_1$-$C_{10}$)-alkyl oder -alkanoyl,
- ($C_1$-$C_4$)-Alkoxy-($C_1$-$C_{10}$)-alkyl,
- ($C_1$-$C_4$)-Alkoxycarbonyl-($C_1$-$C_{10}$)-alkyl,
- ($C_1$-$C_{16}$)-Alkylsulfonyl,
- ($C_1$-$C_8$)-Alkylsulfinyl,
- Mono-, Di-, Trihydroxy-($C_1$-$C_8$)-alkylsulfonyl,
- Mono-, Di-, Trihydroxy-($C_1$-$C_8$)-alkylsulfinyl,
- Mono-, Di-, Tri- oder Tetra-($C_1$-$C_8$)-alkanoyloxy-($C_1$-$C_{10}$)-alkyl,
- ($C_1$-$C_{14}$)-Alkanoyl,
- gegebenenfalls geschütztes Amino-($C_1$-$C_{11}$)-alkanoyl,
- Di-($C_1$-$C_7$)-alkylamino-($C_2$-$C_{11}$)-alkanoyl,
- ($C_1$-$C_9$)-Cycloalkylcarbonyl,
- Aminosubstituiertes ($C_3$-$C_9$)-Cycloalkylcarbonyl,
- Aminosubstituiertes ($C_3$-$C_9$)-Cycloalkylsulfonyl,
- ($C_6$-$C_{10}$)-Aryl-($C_2$-$C_{11}$)-alkanoyl,
- ($C_6$-$C_{10}$)-Aryloxy-($C_2$-$C_{11}$)-alkanoyl,
- gegebenenfalls durch Amino, Halogen, ($C_1$-$C_7$)-Alkyl, ($C_1$-$C_7$)-Alkoxy oder ($C_1$-$C_7$)-Alkoxycarbonyl substituiertes Benzoyl, Benzolsulfonyl oder ($C_6$-$C_{10}$)-Aryl-($C_1$-$C_4$)-alkylcarbonyl bzw. -sufonyl,
- ($C_1$-$C_{10}$)-Alkoxycarbonyl,
- substituiertes ($C_1$-$C_{10}$)-Alkoxycarbonyl,
- ($C_6$-$C_{14}$)-Aryl-($C_1$-$C_6$)-alkoxycarbonyl,
- durch gegebenenfalls geschütztes Amino und Hydroxy substituiertes ($C_6$-$C_{10}$)-Aryl-($C_1$-$C_8$)-alkyl, ($C_3$-$C_{10}$)-Cycloalkyl-($C_1$-$C_8$)-alkyl oder ($C_1$-$C_{10}$)-Alkyl,
- 9-Fluorenylmethoxycarbonyl,
- Ketohexosyl,
- Ketopentosyl,
- Desoxyhexoketosyl,
- Desoxypentoketosyl,
- Aldohexosyl,
- Aldopentosyl,
- Desoxyhexoaldosyl,
- Desoxypentoaldosyl,
- 2-Amino-2-desoxyhexosyl,
- 2-Acetamido-2-desoxyhexosyl,
- Lactosyl oder
- Maltosyl wobei die verknüpften Zucker in der Pyranose-oder Furanose vorliegen können,
- Het-($C_1$-$C_6$)-alkyl
- Het-carbonyl oder -sulfonyl,
- Het-($C_1$-$C_6$)-alkylcarbonyl oder -sulfonyl,
- Het-mercapto-($C_1$-$C_6$)-alkylcarbonyl oder -sulfonyl,

wobei Het jeweils für
Furyl, Thienyl, Benzothienyl, Benzdioxolanyl, Pyrrolyl, Imidazolyl, Isoxazolyl, Thiazolyl, Pyrazolyl, Triazolyl, Pyridyl, Pyrimidyl, Pyrazinyl, Pyridazinyl, Triazinyl, Pyrrolidyl, Piperidyl, Piperazinyl, Morpholino, Thiomorpholino, Tetrahydrofuryl, Tetrahydropyryl, Tetrahydrothienyl, Indolyl, Chinolyl oder Isochinolyl,
wobei diese auch durch eine oder zwei gleiche oder verschiedene Reste aus der Gruppe ($C_1$-$C_4$)-Alkyl, ($C_1$-$C_4$)-Alkoxy, ($C_1$-$C_4$)-Alkoxycarbonyl, ($C_1$-$C_4$)-Alkoxycarbonylamino, Hydroxy, Amino, Mono- oder Di-($C_1$-$C_4$)-Alkylamino und Oxido substituiert sein können;

$R^2$ und $R^{2*}$
unabhängig voneinander
- Wasserstoff,
- Carboxyl,
- ($C_1$-$C_8$)-Alkyl, das gegebenenfalls durch bis zu 2 gleiche oder verschiedene Reste aus der Reihe
- Hydroxy,
- ($C_1$-$C_4$)-Alkoxy,
- ($C_1$-$C_4$)-Alkylthio,

- ($C_1$-$C_4$)-Alkylsulfinyl,
- ($C_1$-$C_4$)-Alkylsulfonyl,
- ($C_1$-$C_4$)-Alkanoyloxy,
- Carboxy,
- ($C_1$-$C_4$)-Alkoxycarbonyl,
- Amino,
- Amidino,
- Guanidino,
- N,N′-Di-(benzyloxycarbonyl)-guanidino,
- Carbamoyl,
- ($C_6$-$C_{10}$)-Aryl-($C_1$-$C_3$)-alkoxycarbonyl,
- ($C_1$-$C_5$)-Alkoxycarbonylamino,
- ($C_6$-$C_{10}$)-Aryl-($C_1$-$C_3$)-alkoxycarbonylamino substituiert ist oder
- ($C_3$-$C_{10}$)-Cycloalkyl,
- ($C_3$-$C_{10}$)-Cycloalkyl-($C_1$-$C_3$)-alkyl,
- ($C_1$-$C_4$)-Alkyl-($C_3$-$C_{10}$)-cycloalkyl-($C_1$-$C_3$)-alkyl,
- ($C_6$-$C_{10}$)-Aryl,
- ($C_6$-$C_{10}$)-Aryl-($C_1$-$C_3$)-alkyl, wobei der Arylteil jeweils gegebenenfalls durch einen, zwei oder drei gleiche oder verschiedene Reste aus der Reihe
- F, Cl, Br,
- Hydroxy,
- ($C_1$-$C_4$)-Alkoxy,
- ($C_1$-$C_4$)-Alkyl,
- ($C_1$-$C_4$)-Alkoxycarbonyl und
- Amino substituiert ist, oder
- Het-($C_1$-$C_4$)-alkyl, wobei Het wie bei $R^1$ bzw $R^{1*}$ definiert ist, bedeutet;

$R^3$ und $R^{3*}$
unabhängig voneinander
- Wasserstoff oder
- Methyl bedeuten;

$R^4$ und $R^{4*}$
unabhängig voneinander
- Wasserstoff oder
- Methyl bedeuten;

$R^5$, $R^{5*}$ und $R^{5**}$
unabhängig voneinander
- Wasserstoff,
- Hydroxy,
- Amino oder
- Carboxy bedeuten;

$R^6$, $R^{6*}$ und $R^{6**}$
unabhängig voneinander
- Wasserstoff oder
- Methyl bedeuten;

$R^7$
- Wasserstoff,
- Hydroxy oder
- Methyl bedeutet;

$R^8$ und $R^{8*}$
unabhängig voneinander
- Wasserstoff,
- Methyl, Ethyl oder n-Propyl bedeuten oder zusammen mit $R^9$ bzw $R^{9*}$ und den diese tragenden Atomen ein 1,2,3,4-Tetrahydroisochinolin oder ein 2-Azabicyclooctan-Gerüst bilden;

$R^9$ und $R^{9*}$
unabhängig voneinander definiert sind wie $R^2$ bzw $R^{2*}$ in Anspruch 4, oder
($C_1$-$C_8$)-Alkanoyloxy bedeuten oder
zusammen mit $R^{10}$ bzw. $R^{10*}$ und den diese tragenden Atomen cyclische Ringsysteme mit 5 bis 7 Ringgliedern bilden;

oder zusammen mit $R^{11}$ bzw $R^{11*}$ ein Thiochromansystem bilden, dessen Schwefelatom gegebenenfalls zum Sulfon oxidiert sein kann;
$R^{10}$ und $R^{10*}$
unabhängig voneinander
- Wasserstoff oder
- Methyl bedeuten;
$R^{11}$ und $R^{11*}$ wie in Anspruch 4 definiert sind;
wobei
in den vorstehenden Verbindungen der Formel I eine oder mehrere Amidgruppen (-CONH-) der Hauptkette ersetzt sein können wie in Anspruch 4 definiert;
$R^{14}$ für
- Wasserstoff oder
- Methyl steht;
sowie deren physiologisch verträglichen Salze.
6. Verfahren zur Herstellung einer Verbindung der Formel I gemäß den Ansprüchen 1 bis 5, dadurch gekennzeichnet, daß
$R^1$ und $R^{1*}$
unabhängig voneinander stehen für
$a_1$) - Wasserstoff,
- Carboxyl,
- $(C_1$-$C_{16})$-Alkylsulfonyl,
- $(C_1$-$C_8)$-Alkylsulfinyl,
- $(C_1$-$C_8)$-Mono-, Di- oder Tri-Hydroxyalkylsulfonyl,
- Hydroxy-$(C_1$-$C_{10})$-alkanoyl,
- Mono-, Di-, Tri- oder Tetra-Hydroxy-$(C_1$-$C_4)$-alkyl,
- $(C_1$-$C_8)$-Alkanoyloxy-$(C_1$-$C_{10})$-alkyl,
- 1,2-Diacetoxyethyl,
- 1,2,3-Triacetoxypropyl,
- $(C_1$-$C_{14})$-Alkanoyl,
- Amino-$(C_1$-$C_{12})$-alkanoyl,
- N-$(C_1$-$C_4)$-Alkoxycarbonylamino-$(C_1$-$C_8)$-alkyl,
- Di-$(C_1$-$C_7)$-alkylamino-$(C_2$-$C_{11})$-alkanoyl,
- $(C_3$-$C_9)$-Cycloalkylcarbonyl,
- Amino-$(C_3$-$C_8)$-Cycloalkylcarbonyl,
- Amino-$(C_3$-$C_8)$-Cycloalkylsulfonyl,
- Phenyl
- $(C_6$-$C_{10})$-Aryl-$(C_2$-$C_{11})$-alkanoyl,
- $(C_6$-$C_{10})$-Aryloxy-$(C_2$-$C_{11})$-alkanoyl,
- gegebenenfalls durch Halogen, Amino, $(C_1$-$C_7)$-Alkyl, $(C_1$-$C_7)$-Alkoxy oder $(C_1$-$C_7)$-Alkoxycarbonyl substituiertes Benzoyl oder -Benzolsulfonyl
- gegebenenfalls durch Halogen, Amino, $(C_1$-$C_7)$-Alkyl, $(C_1$-$C_7)$-Alkoxy oder $(C_1$-$C_7)$-Alkoxycarbonyl substituiertes Benzylsulfonyl, Benzylsulfinyl oder Benzylthio,
- Amino,
- $(C_1$-$C_4)$-Alkoxycarbonylamino,
- $(C_1$-$C_{12})$-Alkanoyl, das durch Hydroxy, Amino und gegebenenfalls durch Phenyl oder Cyclohexyl substituiert ist,
- gegebenenfalls geschütztes aminosubstituiertes $(C_6$-$C_{10})$-Aryl- oder $(C_3$-$C_{10})$-Cycloalkyl-$(C_1$-$C_4)$-alkyl oder $(C_1$-$C_8)$-Alkyl,
- $(C_1$-$C_{10})$-Alkoxycarbonyl,
- substituiertes $(C_1$-$C_{10})$-Alkoxycarbonyl,
- $(C_6$-$C_{14})$-Aryl-$(C_1$-$C_6)$-alkoxycarbonyl,
- 9-Fluorenylmethoxycarbonyl,
- 1-Desoxyhexoketosyl oder 1-Desoxypentoketosyl,
- Hexosyl oder Pentosyl,
- 6-Desoxyhexosyl,
- Aminozuckerreste,
- Lactosyl
- Maltosyl

wobei die verknüpften Zucker in der Pyranose- oder der Furanose-Form vorliegen können,
- Het,
- Het-carbonyl oder Het-sulfonyl,
- Het-($C_1$-$C_6$)-alkyl,
- Het-($C_1$-$C_6$)-alkanoyl,
- Het-($C_1$-$C_6$)-alkylsulfonyl oder
- Het-mercapto-($C_1$-$C_3$)-alkylcarbonyl, wobei Het jeweils steht für
- Pyrrolyl,
- Imidazolyl,
- Pyridyl,
- Pyrimidyl,
- Pyrrolidyl,
- Piperidyl,
- Morpholino,
- Chinolyl oder
- Isochinolyl,

wobei dieser auch durch einen oder zwei gleiche oder verschiedene Reste aus der Gruppe ($C_1$-$C_4$)-Alkyl, ($C_1$-$C_4$)-Alkoxycarbonyl, ($C_1$-$C_4$)-Alkoxycarbonylamino, Hydroxy, Amino, Mono- oder Di-($C_1$-$C_4$)-Alkylamino substituiert sein kann;

$R^2$ und $R^{2*}$ unabhängig voneinander stehen für
- Wasserstoff,
- Carboxyl,
- Methyl, Ethyl, Isopropyl, n-Propyl, n-Butyl, Isobutyl, sec.-Butyl, n-Pentyl, n-Hexyl,
- Cyclohexyl,
- Cyclopentylmethyl, Cyclohexylmethyl, Cycloheptylmethyl,
- 4-Methylcyclohexylmethyl,
- 1-Dekahydronaphthylmethyl, 2-Dekahydronaphthylmethyl,
- Phenyl,
- Benzyl,
- 2-Phenylethyl,
- 1-Naphthylmethyl, 2-Naphthylmethyl,
- 2-Methylbenzyl, 3-Methylbenzyl, 4-Methylbenzyl,
- 2,4,6-Trimethylbenzyl,
- 4-tert.-Butylbenzyl,
- 4-tert.-Butoxybenzyl
- 4-Hydroxybenzyl,
- 4-Methoxybenzyl,
- 2,4-Dimethoxybenzyl,
- 3,4-Dihydroxybenzyl,
- 3,4-Dimethoxybenzyl,
- (Benzdioxolan-5-yl)methyl,
- 4-Chlorbenzyl,
- Hydroxymethyl,
- 1-Hydroxyethyl,
- 4-Pyridyl, 4-(N-Oxidopyridyl),
- 2 Pyridylmethyl, 3-Pyridylmethyl, 4-Pyridylmethyl, -2-(4-Pyridyl)ethyl,
- 2-Thienylmethyl, 3-Thienylmethyl,
- 2-(2-Thienyl)ethyl, 2-(3-Thienyl)ethyl,
- Indol-2-yl-methyl, Indol-3-yl-methyl,
- (1-Methyl-imidazol-4-yl)methyl,
- Imidazol-4-yl-methyl, Imidazol-1-yl-methyl,
- 2-Thiazolylmethyl,
- 3-Pyrazolylmethyl,
- 4-Pyrimidylmethyl,
- 2-Benzo[b]thienylmethyl, 3-Benzo[b]thienylmethyl,
- 2-Furylmethyl,
- 2-(Methylthio)ethyl,
- 2-(Methylsulfinyl)ethyl,

- 2-(Methylsulfonyl)ethyl,
$R^3$, $R^{3*}$, $R^4$, $R^{4*}$, $R^6$, $R^{6*}$, $R^{10}$ und $R^{10*}$
Wasserstoff bedeuten;
$R^5$ und $R^{5*}$
unabhängig voneinander stehen für
- Wasserstoff
- Hydroxy oder
- Amino;
$R^7$ - Wasserstoff,
- Hydroxy oder
- Methyl bedeutet;
$R^8$ und $R^{8*}$
unabhängig voneinander bedeuten
- Wasserstoff oder
zusammen mit $R^9$ bzw. $R^{9*}$ und den diese tragenden Atomen ein 1,2,3,4-Tetrahydroisochinolin oder 2-Azabicyclooctan-Gerüst bilden;
$R^9$ und $R^{9*}$
unabhängig voneinander
wie $R^2$ bzw. $R^{2*}$ definiert sind oder
- Hydroxy,
- Acetoxy,
- tert.-Butoxymethyl,
- 3-Guanidinopropyl,
- Carbamoylmethyl, Carbamoylethyl,
- Carboxymethyl, Carboxyethyl,
- Mercaptomethyl,
- (1-Mercapto-1-methyl)ethyl,
- Aminomethyl, 2-Aminoethyl, 3-Aminopropyl, 4-Aminobutyl,
- N,N-Dimethylamino,
- N,N′-Di-(benzyloxycarbonyl)-guanidino-propyl,
- 2-Benzyloxycarbonylethyl, Benzyloxycarbonylmethyl
- tert.- Butylsulfonylmethyl
oder
- 4-Benzylcarbonylaminobutyl bedeuten;
$R^{11}$ und $R^{11*}$ unabhängig voneinander
- Wasserstoff,
- Hydroxy oder
- Acetoxy bedeuten;
wobei
in den vorstehenden Verbindungen dieser Erfindung eine oder mehrere Amidgruppen (-CONH-) der Hauptkette ersetzt sein können durch $-CH_2NR_{14}-$ oder $-CH(OH)CH_2-$;
$R^{14}$ für
- Wasserstoff oder
- Methyl steht;
sowie deren physiologisch verträgliche Salze.

7. Verfahren zur Herstellung einer Verbindung der Formel I gemäß den Ansprüchen 1 bis 6, dadurch gekennzeichnet, daß
$R^1$ und $R^{1*}$
unabhängig voneinander stehen für
$a_1$) - Wasserstoff,
- Carboxyl,
- $(C_1-C_{16})$-Alkylsulfonyl,
- $(C_1-C_8)$-Mono- oder Dihydroxyalkylsulfonyl,
- Mono-, Di- oder Trihydroxy-$(C_1-C_3)$-alkyl,
- $(C_1-C_8)$-Alkoxycarbonyl,
- $(C_1-C_{14})$-Alkanoyl,
- Amino-$(C_1-C_{12})$-alkanoyl
- $(C_6-C_{10})$-Aryl-$(C_1-C_4)$-alkoxycarbonyl,

- $(C_6$-$C_{10}$-Aryl-$(C_1$-$C_4)$-alkylcarbonyl,
- 9-Fluorenylmethoxycarbonyl,
- $(C_1$-$C_4)$-Alkanoyloxy-$(C_1$-$C_6)$-alkyl,
- 1,2-Diacetoxyethyl,
- 1,2,3-Triacetoxypropyl,
- Phenyl
- gegebenenfalls durch Halogen, Amino, $(C_1$-$C_4)$-Alkyl, oder Methoxy substituiertes Benzolsulfonyl,
- gegebenenfalls durch Halogen, Amino, $(C_1$-$C_4)$-Alkyl, oder Methoxy substituiertes Benzylsulfonyl, -sulfinyl oder -thio,
- Het- oder Het-sulfonyl,
- Het-$(C_1$-$C_4)$-alkanoyl,
- Het-mercapto-$(C_1$-$C_3)$-alkylcarbonyl,

wobei Het jeweils steht für

- Pyrrolyl,
- Imidazolyl,
- Pyridyl,
- Pyrimidyl,
- Pyrrolidyl,
- Chinolyl,
- Isochinolyl,
- Piperidyl oder
- Morpholino,

wobei dieser Rest auch durch durch einen oder zwei gleiche oder verschiedene Reste aus der Gruppe Methyl, Amino und $(C_1$-$C_4)$-Alkoxycarbonylamino substituiert sein kann,

- Amino-$(C_3$-$C_6)$-Cycloalkylcarbonyl,
- $(C_1$-$C_8)$-Alkanoyl, das durch Hydroxy und Amino und gegebenenfalls durch Phenyl oder Cyclohexyl substituiert ist,
- gegebenenfalls geschütztes aminosubstituiertes Phenyl-oder Cyclohexyl-$(C_1$-$C_6)$-alkyl,
- Amino,
- $(C_1$-$C_4)$-Alkoxycarbonylamino,
- Benzyloxycarbonylamino,
- 1-Desoxyhexoketosyl oder 1-Desoxypentoketosyl,
- Hexosyl oder Pentosyl,

wobei die verknüpften Zucker in der Pyranose- oder der Furanose-Form vorliegen können,

$R^2$ und $R^{2*}$ unabhängig voneinander stehen für

- Wasserstoff,

Methyl, Ethyl, Isopropyl, n-Propyl, n-Butyl, Isobutyl, sec.-Butyl, Pentyl, Hexyl,

- Cyclopentylmethyl, Cyclohexylmethyl,
- 4-Methylcyclohexylmethyl,
- Benzyl,
- 2-Phenylethyl,
- 1-Naphthylmethyl, 2-Naphthylmethyl,
- 2-Methylbenzyl, 3-Methylbenzyl, 4-Methylbenzyl,
- 2,4,6-Trimethylbenzyl,
- 4-tert.-Butylbenzyl,
- 4-Methoxybenzyl,
- 3,4-Dihydroxybenzyl,
- 2,4-Dimethoxybenzyl,
- 3,4-Dimethoxybenzyl,
- 2-Pyridylmethyl, 3-Pyridylmethyl, 4-Pyridylmethyl,
- 3,4-Methylendioxybenzyl oder
- 2-(4-Pyridyl)ethyl,

$R^3$, $R^{3*}$, $R^4$, $R^{4*}$, $R^6$, $R^{6*}$, $R^7$, $R^{10}$ und $R^{10*}$ Wasserstoff bedeuten;

$R^5$ und $R^{5*}$ unabhängig voneinander

- Wasserstoff oder
- Hydroxy bedeuten;

$R^8$ und $R^{8*}$ unabhängig voneinander wie in Anspruch 6 definiert sind;
$R^9$ und $R^{9*}$
unabhängig voneinander wie $R^9$ bzw. $R^{9*}$ in Anspruch 6 definiert sind;
$R^{11}$ und $R^{11*}$ unabhängig voneinander wie Anspruch 6 definiert sind, sowie deren physiologisch verträgliche Salze.

8. Verfahren zur Herstellung einer Verbindung der Formel I gemäß den Ansprüchen 1 bis 7, dadurch gekennzeichnet, daß
Y für einen Rest der Formel III steht;
l 0 oder 1 bedeutet;
m 1 bedeutet;
A, A*, D und D* wie in Anspruch 1 definiert sind;
n, n*, o, o*, p und p* unabhängig voneinander 0 oder 1 bedeuten;
E, E*, F, F*, G und G* unabhängig voneinander für eine Aminosäure aus der Reihe Val, Lys, Lys(Z), Phe, Chg, Ser, Asn, Gly, Ile, Tbg, Nva oder Npg bedeuten;
$R^1$ und $R^{1*}$ unabhängig voneinander
- Wasserstoff,
- Carboxyl,
- Methylsulfonyl,
- tert.-Butylsulfonyl,
- tert.-Butoxycarbonyl,
- 2-Hydroxyethylsulfonyl,
- 1,2,3-Trihydroxypropyl,
- 1,2,3-Triacetoxypropyl,
- Benzyloxycarbonyl,
- 4-Methylphenylsulfonyl,
- 4-Chlorbenzylthio,
- Benzylsulfinyl,
- 4-Chlorbenzylsulfonyl,
- Hexadecylsulfonyl,
- 4-Amino-1-piperidyl-sulfonyl.
- N-tert.-butoxycarbonyl-4-amino-1-piperidyl-sulfonyl,
- 4-Amino-1-piperidyl-carbonyl,
- N-tert.-butoxycarbonyl-4-amino-1-piperidyl-carbonyl,
- 2-Amino-3-phenyl-propyl.
- N-tert.-Butoxycarbonyl-2-amino-3-phenyl-propyl,
- 2-Amino-1-hydroxy-4-methyl-pentyl.
- Desoxyfructos-1-yl,
- Mannofuranosyl,
- 4-Aminocyclohexylcarbonyl.
- 2-Pyridylacetyl,
- 4-Pyridylthio-acetyl,
- 2-Chinolylcarbonyl,
- 1-Naphthylacetyl,
- 1-Naphthyloxyacetyl.
- 1-(4-Pyridyl)ethylsulfonyl.
- 12-Aminododecanoyl.
- 4-(N-Oxidopyridyl).
- 4-Pyridyl,
- Tetradecanoyl,
- Phenyl,
- Amino oder
- tert.-Butoxycarbonylamino bedeuten;
$R^2$ und $R^{2*}$ unabhängig voneinander
- Wasserstoff,
- 2-(4-Pyridyl)ethyl,
- Isopropyl,
- Isobutyl,
- n-Pentyl,

- Benzyl,
- 3,4-Methylendioxybenzyl,
- 2,4-Dimethoxybenzyl,
- 4-tert.-Butylbenzyl,
- 2-Phenylethyl oder
- Cyclohexylmethyl bedeuten;

$R^3$, $R^{3*}$, $R^4$, $R^{4*}$, $R^6$, $R^{6*}$, $R^7$, $R^{10}$ und $R^{10*}$
- Wasserstoff

bedeuten;

$R^5$ und $R^{5*}$ unabhängig voneinander
- Wasserstoff oder
- Hydroxy bedeuten;

$R^8$ und $R^{8*}$
- Wasserstoff bedeuten, oder zusammen mit $R^9$ bzw. $R^{9*}$ und den diese tragenden Atomen ein 1,2,3,4-Tetrahydrochinolin-3,4-diyl-System bilden;

$R^9$ und $R^{9*}$ unabhängig voneinander
- Wasserstoff,
- Hydroxy,
- Acetoxy,
- n-Propyl,
- Isopropyl,
- Isobutyl,
- Aminomethyl,
- 4-Aminobutyl,
- Hydroxymethyl,
- tert.-Butoxymethyl,
- Aminocarbonylmethyl,
- 2-Benzyloxycarbonyl-ethyl,
- 4-Benzyloxycarbonylamino-butyl,
- N,N°-Di-(benzyloxycarbonyl)-guanidino-propyl,
- Cyclohexyl,
- Cyclohexylmethyl,
- Benzyl,
- 2-Phenyl-ethyl
- 4-Hydroxy-benzyl,
- 4-Methoxy-benzyl,
- 4-tert.-Butoxy-benzyl,
- 1-Naphthylmethyl,
- 2-Thienylmethyl,
- 1-Imidazolyl-methyl,
- 3-Indolyl-methyl,
- 4-Pyridylmethyl
- 4-(N-Oxidopyridyl)methyl
- 2-Methylthio-ethyl,
- 2-Methylsulfonyl-ethyl
- tert.-Butylsulfonyl-methyl oder
- 2-Carboxyl-ethyl bedeuten;

$R^{11}$ und $R^{11*}$ unabhängig voneinander
- Wasserstoff,
- Hydroxy oder
- Acetoxy bedeuten;

wobei in den vorstehenden Verbindungen eine oder mehrere Amidgruppen (-CONH-) der Hauptkette ersetzt sein können durch $-CH_2NH-$ oder $-CH(OH)CH_2-$;
sowie deren physiologisch verträgliche Salze.

9. Verfahren zur Herstellung einer Verbindung der Formel I gemäß den Ansprüchen 1 bis 8, dadurch gekennzeichnet, daß
$l = 0$;
$m = 1$;

n + o + p = 1;
D und D* für einen Rest der Formel VI bzw. VI* stehen
$R^1$ und $R^{1*}$
- ($C_1$-$C_{12}$)-Alkylsulfonyl, das gegebenenfalls durch bis zu 3 gleiche oder verschiedene Resten aus der Reihe
- Hydroxy,
- Amino oder
- Carboxy substituiert sein kann;
bedeuten;
$R^2$ und $R^{2*}$ unabhängig voneinander
- Wasserstoff,
- Carboxyl,
- Methyl, Ethyl, Isopropyl, n-Propyl, n-Butyl, Isobutyl, sec.-Butyl, Pentyl, Hexyl,
- Cyclohexyl,
- Cyclopentylmethyl, Cyclohexylmethyl, Cycloheptylmethyl,
- 4-Methylcyclohexylmethyl,
- 1-Dekahydronaphthylmethyl, 2-Dekahydronaphthylmethyl,
- Phenyl,
- Benzyl,
- 2-Phenylethyl,
- 1-Naphthylmethyl, 2-Naphthylmethyl,
- 2-Methylbenzyl, 3-Methylbenzyl, 4-Methylbenzyl,
- 2,4,6-Trimethylbenzyl,
- 4-tert.-Butylbenzyl,
- 4-tert.-Butoxybenzyl
- 4-Hydroxybenzyl,
- 4-Methoxybenzyl,
- 2,4-Dimethoxybenzyl,
- 3,4-Dihydroxybenzyl,
- 3,4-Dimethoxybenzyl,
- (Benzdioxolan-4-yl)methyl,
- 4-Chlorbenzyl,
- Hydroxymethyl,
- 1-Hydroxyethyl,
- 2-Pyridylmethyl, 3-Pyridylmethyl, 4-Pyridylmethyl, - 2-(4-Pyridyl)ethyl,
- 2-Thienylmethyl, 3-Thienylmethyl,
- 2- (2-Thienyl)ethyl, 2-(3-Thienyl)ethyl,
- Indol-2-yl-methyl, Indol-3-yl-methyl,
- (1-Methyl-imidazol-4-yl)methyl,
- Imidazol-4-yl-methyl, Imidazol-1-yl-methyl,
- 2-Thiazolylmethyl,
- 3-Pyrazolylmethyl,
- 4-Pyrimidylmethyl,
- 2-Benzo[b]thienylmethyl, 3-Benzo[b]thienylmethyl,
- 2-Furylmethyl,
- 2-(Methylthio)ethyl,
- 2-(Methylsulfinyl)ethyl oder
- 2-(Methylsulfonyl)ethyl bedeuten;
$R^3$, $R^{3*}$, $R^4$, $R^{4*}$, $R^6$, $R^{6*}$, $R^{11}$ und $R^{11*}$
- Wasserstoff bedeuten;
$R^5$ und $R^{5*}$
- Hydroxy bedeuten;
$R^9$ und $R^{9*}$
wie in Anspruch 8 definiert sind;
sowie deren physiologisch verträgliche Salze.

10. Verfahren zur Herstellung einer pharmazeutischen Mittels, enthaltend eine Verbindung der Formel I gemäß den Ansprüchen 1 bis 8, dadurch gekennzeichnet, daß man diese und gegebenenfalls einen oder mehrere Träger in eine geeignete Darreichungsform bringt.

11. Verwendung einer Verbindung der Formel I gemäß den Ansprüchen 1 bis 9 als Heilmittel.

12. Verwendung einer Verbindung der Formel I gemäß den Ansprüchen 1 bis 9 zur Hemmung retroviraler Proteasen.

13. Verwendung einer Verbindung der Formel I gemäß den Ansprüchen 1 bis 9 bei der Behandlung des "erworbenen Immunschwäche-Syndroms".